# EUROPEAN PATENT APPLICATION

(11) **EP 2 194 110 A1**
(43) Date of publication of application: **09.06.2010**
(21) Application number: 09156586.1
(22) Date of filing: 30.03.2009
(51) Int. Cl.: C09K 11/06, H05B 33/14, C07C 15/28, H01L 51/00

(54) **Electroluminescent device using electroluminescent compounds**

(30) Priority: 26.11.2008 KR 20080118428
(71) Applicant: Gracel Display Inc., Seoul 133-833 (KR)
(72) Inventor: Cho, Young Jun, Seoul 136-060 (KR); Kwon, Hyuck Joo, Seoul 130-100 (KR); Kim, Bong Ok, Seoul 135-090 (KR); Kim, Sung Min, Seoul 157-886 (KR); Yoon, Seung Soo, Seoul 135-884 (KR)
(74) Representative: Kent, Venetia Katherine

(57) **Abstract**

Provided is an electroluminescent device including an organic layer interposed between an anode and a cathode on a substrate, wherein the organic layer includes an electroluminescent layer containing one or more dopant compound(s) represented by Chemical Formula 1 and one or more host compound(s) represented by Chemical Formula 2: 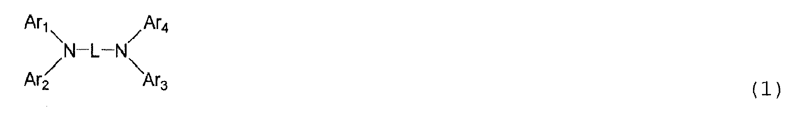 with the proviso that at least one of Ar₁ through Ar₄ is 1- or 2-naphthyl with or without substituent; and

Ar₁₀-(An)ₐ-Ar₂₀ (2)

Including an electroluminescent compound with an arylamine group substituted by at least one of 1- or 2-naphthyl, the disclosed electroluminescent device exhibits superior luminous efficiency, excellent color purity and very good operation life.

## Description

### CROSS-REFERENCE TO RELATED APPLICATION

This application claims priority to Korean Patent Application No. 10-2008-0118428, filed on November 26, 2008, and all the benefits accruing therefrom under 35 U.S.C. §119.

### BACKGROUND

### 1. Field

The present invention relates to an electroluminescent device in which an organic layer is interposed between an anode and a cathode on a substrate, wherein the organic layer comprises an electroluminescent layer comprising one or more dopant compound(s) represented by Chemical Formula 1 and one or more host compound(s) represented by Chemical Formula 2: 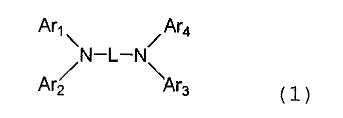 wherein at least one of Ar₁ through Ar₄ is 1- or 2-naphthyl with or without substituent;

Ar₁₀-(An)ₐ-Ar₂₀ (2)

### 2. Description of the Related Art

Among display devices, electroluminescence (EL) devices are advantageous in that they provide wide view angle, superior contrast and fast response rate as self-emissive display devices. In 1987, Eastman Kodak first developed an organic EL device using low-molecular-weight aromatic diamine and aluminum complex as electroluminescent materials [Appl. Phys. Lett. 51, 913, 1987].

In an organic EL device, when a charge is applied to an organic layer formed between an electron injection electrode (cathode) and a hole injection electrode (anode), an electron and a hole are paired and emit light as the electron-hole pair is extinguished. The organic EL device is advantageous in that it can be formed on a flexible transparent substrate such as plastic, is operable with relatively low voltage (10 V or lower) as compared to plasma display panels or inorganic EL displays, consumes less power and provides excellent color. Since an organic EL device can exhibit green, blue and red colors, it is drawing a lot of attention as a full-color display device of the next generation. Aprocess of manufacturing an organic EL device is as follows:
(1) First, an anode material is coated on a transparent substrate. Indium tin oxide (ITO) is frequently used as the anode material.
(2) Then, a hole injection layer (HIL) is formed thereupon. The hole injection layer is typically formed by coating copper phthalocyanine (CuPc) to a thickness of 10 to 30 nm.
(3) Then, a hole transport layer (HTL) is formed. The hole transport layer is formed by depositing 4,4'-bis[N-(1-naphthyl)-N-phenylamino]biphenyl (NPB) to a thickness of about 30 to 60 nm.
(4) An organic electroluminescent layer (organic emitting layer) is formed thereupon. If required, a dopant is added thereto. In case of green electroluminescence, an organic emitting layer is formed frequently by depositing tris(8-hydroxyquinolato)aluminum (Alq₃) to a thickness of about 30 to 60 nm. For the dopant, N-methylquinacridone (MQD) is commonly used.
(5) An electron transport layer (ETL) and an electron injection layer (EIL) are formed sequentially, or an electron injection/transport layer is formed thereupon. In case of green electroluminescence, the ETL and/or EIL may be unnecessary because Alq₃ has good electron transport ability.
(6) Then, a cathode is formed, and, finally, a protective layer is formed thereupon.

With such a structure, blue, green and red EL devices are obtained respectively depending on how the electroluminescent layer is formed. The existing green electroluminescent compounds used to create green EL devices do not have good life property or luminous efficiency.

In an organic EL device, the most important factor affecting such quality as luminous efficiency, life property, etc. is the electroluminescent material. Several requirements of the electroluminescent material include high fluorescence quantum yield in solid state, high electron and hole mobility, resistance to decomposition during vacuum deposition, ability to form uniform and thin film, and good stability.

Organic electroluminescent materials may be roughly classified into high-molecular-weight materials and low-molecular-weight materials. The low-molecular-weight materials may be classified into metal complexes and metal-free pure organic electroluminescent materials, depending on molecular structure. Chelate complexes such as tris(8-quinolato)aluminum, coumarin derivatives, tetraphenylbutadiene derivatives, bisstyrylarylene derivatives, oxadiazole derivatives, or the like are known. It is reported that electroluminescence from the blue to the red region can be obtained using these materials, and the realization of full-color display devices is being expected.

### SUMMARY

The present inventors have invented an electroluminescent device comprising an organic layer interposed between an anode and a cathode on a substrate, the organic layer comprising a combination of specific compounds, in order to realize an electroluminescent device having high color purity, high luminous efficiency and long operation life.

An object of the present invention is to provide an electroluminescent device comprising an organic layer interposed between an anode and a cathode on a substrate, wherein the organic layer comprises an electroluminescent layer comprising one or more host compound(s) and one or more dopant compound(s). Another object of the present invention is to provide an electroluminescent device having excellent luminous efficiency, superior color purity, low driving voltage and good operation life. Another obj ect of the present invention is to provide an organic solar cell comprising two or more of the electroluminescent compounds.

### BRIEF DESCRIPTION OF THE DRAWINGS

The above and other aspects, features and advantages of the present invention will become apparent from the following description of preferred embodiments given in conjunction with the accompanying drawing, in which:

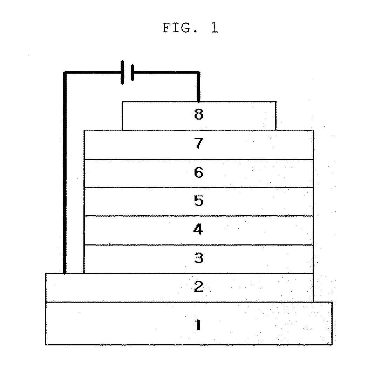

FIG. 1 is a cross-sectional view of an OLED device.

### DETAILED DESCRIPTION

Exemplary embodiments now will be described more fully hereinafter with reference to the accompanying drawings, in which exemplary embodiments are shown. This disclosure may, however, be embodied in many different forms and should not be construed as limited to the exemplary embodiments set forth therein. Rather, these exemplary embodiments are provided so that this disclosure will be thorough and complete, and will fully convey the scope of this disclosure to those skilled in the art. In the description, details of well-known features and techniques may be omitted to avoid unnecessarily obscuring the presented embodiments.

The terminology used herein is for the purpose of describing particular embodiments only and is not intended to be limiting of this disclosure. As used herein, the singular forms "a", "an" and "the" are intended to include the plural forms as well, unless the context clearly indicates otherwise. Furthermore, the use of the terms a, an, etc. does not denote a limitation of quantity, but rather denotes the presence of at least one of the referenced item. The use of the terms "first", "second", and the like does not imply any particular order, but they are included to identify individual elements. Moreover, the use of the terms first, second, etc. does not denote any order or importance, but rather the terms first, second, etc. are used to distinguish one element from another. It will be further understood that the terms "comprises" and/or "comprising", or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Unless otherwise defined, all terms (including technical and scientific terms) used herein have the same meaning as commonly understood by one of ordinary skill in the art. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

The shape, size and regions, and the like, of the drawing may be exaggerated for clarity.

The present invention relates to an electroluminescent device. More particularly, the electroluminescent device according to the present invention comprises an organic layer interposed between an anode and a cathode on a substrate, wherein the organic layer comprises an electroluminescent layer comprising one or more dopant compound (s) represented by Chemical Formula 1 and one or more host compound(s) represented by Chemical Formula 2: 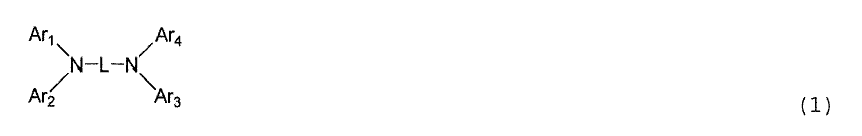 wherein
Ar₁ through Ar₄ independently represent (C6-C60)aryl with or without substituent or (C5-C50) heteroaryl with or without substituent, with the proviso that at least one of Ar₁ through Ar₄ is 1- or 2-naphthyl with or without substituent;
the substituent substituted at the aryl or heteroaryl of Ar₁ through Ar₄ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
L represents (C6-C60)arylene with or without substituent, (C3-C60)heteroarylene with or without substituent, stilbenylene with
or without substituent, 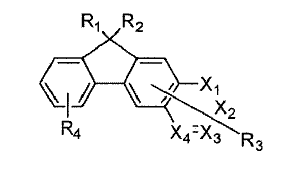 or 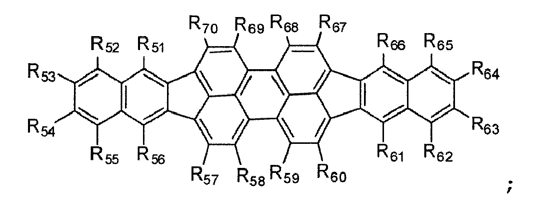 the substituent substituted at the arylene, heteroarylene or stilbenylene of L is one or more selected from deuterium, halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
X₁ through X₄ independently represent carbon or nitrogen;
R₁ through R₄ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, amino, mono- or di-(C1-C60)alkylamino, mono- or di-(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₁ through R₄ may be linked to an adjacent substituent to form a saturated or unsaturated mono- or polycyclic ring or heteroaromatic ring;
R₅₁ through R₇₀ independently represent hydrogen, deuterium, halogen, (C1-C60)alkyl, (C6-C60)aryl, (C9-C60)heteroaryl, (C1-C60)alkylamino or (C6-C60)arylamino, or each of R₅₁ through R₇₀ may be linked to an adjacent substituent via (C3-C60)alkylene or (C3-C60)alkenylene with or without a fused ring to form an alicyclic ring or a mono- or polycyclic ring;
the aryl, heteroaryl or arylamino, or the mono- or polycyclic aromatic ring may be further substituted by one or more substituent (s) selected from deuterium, halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, carboxyl, nitro and hydroxyl;

Ar₁₀-(An)ₐ-Ar₂₀ (2)

wherein
An represents anthracenylene with or without one or more substituent(s) or -X-L₁₀₀-Y-;
X and Y independently represent anthracenylene;
L₁₀₀ represents (C6-C60)arylene or (C5-C60)heteroarylene with or without one or more substituent(s);
Ar₁₀ and Ar₂₀ independently represent (C6-C60)aryl with or without substituent or (C5-C60)heteroaryl with or without substituent;
the substituent substituted at An, Ar₁₀, Ar₂₀ or L₁₀₀ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a represents an integer from 1 to 4.

In the present invention, "alkyl", "alkoxy" and other substituents including "alkyl" moietymay be either linear or branched.

In the present invention, "aryl" means an organic radical derived from an aromatic hydrocarbon by the removal of one hydrogen, and may include a 4- to 7-membered, particularly 5- or 6-membered, single ring or fused ring. Specific examples include phenyl, naphthyl, biphenyl, anthryl, indenyl, fluorenyl, phenanthryl, triphenylenyl, pyrenyl, perylenyl, chrysenyl, naphthacenyl, fluoranthenyl, etc., but not limited thereto.

In the present invention, "arylene" includes those formed as identical or different arylenes are fused, and "heteroarylene" includes those formed as identical or different heteroarylenes are fused.

In the present invention, "heteroaryl" means aryl group containing 1 to 4 heteroatom(s) selected from N, O and S as aromatic ring backbone atom(s), other remaining aromatic ring backbone atoms being carbon. It may be 5- or 6-membered monocyclic heteroaryl or polycyclic heteroaryl resulting from condensation with a benzene ring, and may be partially saturated. The heteroaryl includes a divalent aryl group wherein the heteroatom(s) in the ring may be oxidized or quaternized to form, for example, N-oxide or quaternary salt. Specific examples include monocyclic heteroaryl such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., polycyclic heteroaryl such as benzofuranyl, benzothiophenyl, isobenzofuranyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl, benzodioxolyl, etc., N-oxide thereof (e.g., pyridyl N-oxide, quinolyl N-oxide, etc.), quaternary salt thereof, etc., but not limited thereto.

The naphthyl includes 1-naphthyl and 2-naphthyl, the anthryl includes 1-anthryl, 2-anthryl and 9-anthryl, and the fluorenyl includes all of 1-fluorenyl, 2-fluorenyl, 3-fluorenyl, 4-fluorenyl and 9-fluorenyl.

And, in the present invention, the substituents including "(C1-C60)alkyl" moiety may have 1 to 60 carbon atoms, 1 to 20 carbon atoms, or 1 to 10 carbon atoms. The substituents including "(C6-C60)aryl" moiety may have 6 to 60 carbon atoms, 6 to 20 carbon atoms, or 6 to 12 carbon atoms. The substituents including "(C3-C60)heteroaryl" moiety may have 3 to 60 carbon atoms, 4 to 20 carbon atoms, or 4 to 12 carbon atoms. The substituents including "(C3-C60)cycloalkyl" moiety may have 3 to 60 carbon atoms, 3 to 20 carbon atoms, or 3 to 7 carbon atoms. The substituents including "(C2-C60)alkenyl or alkynyl" moiety may have 2 to 60 carbon atoms, 2 to 20 carbon atoms, or 2 to 10 carbon atoms.

The electroluminescent device according to the present invention exhibits an effective energy transfer mechanism between a host and a dopant and, therefore, may provide good, high-efficiency EL characteristics through improved electron density distribution. Further, it can overcome the problems associated with the existing materials, i.e., decrease of initial efficiency, short operation life, etc., and can provide high-performance EL characteristics with high efficiency and long operation life for individual colors.

In the dopant compound represented by Chemical Formula 1, Ar₁ through Ar₄ are independently selected from the following structures, but not limited thereto: 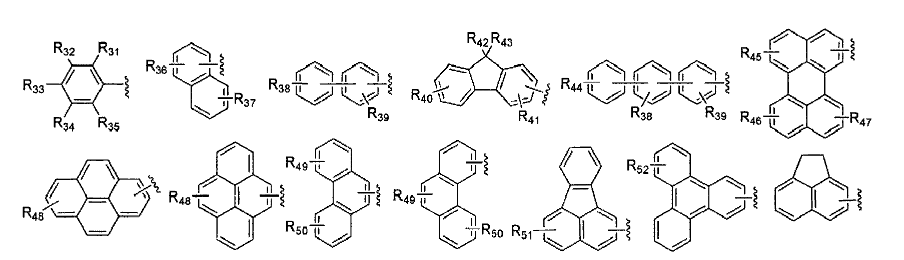 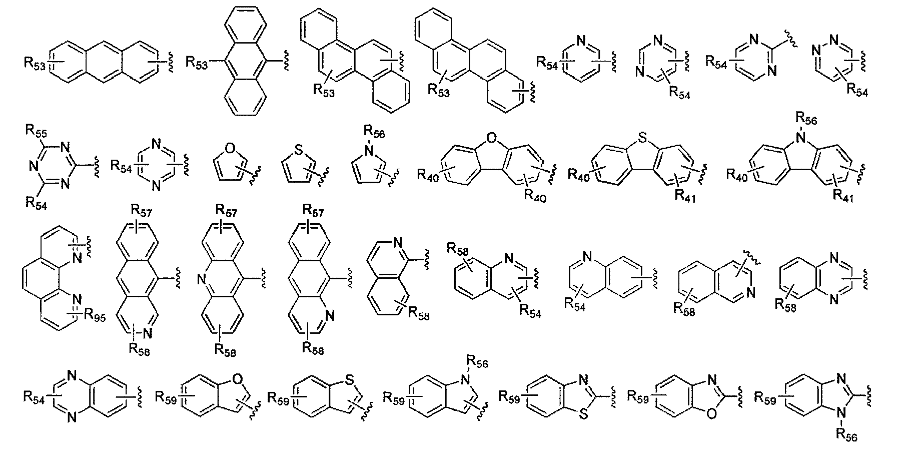 wherein
R₃₁ through R₅₉ independently represent hydrogen, deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, and R₄₂ and R₄₃ may be linked via (C3-C60)alkylene or (C3-C60) alkenylene with or without a fused ring to form an alicyclic ring or a mono- or polycyclic ring.

In the dopant compound represented by Chemical Formula 1, at least one of 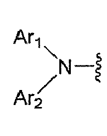 and 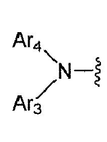 is selected from the following structures:

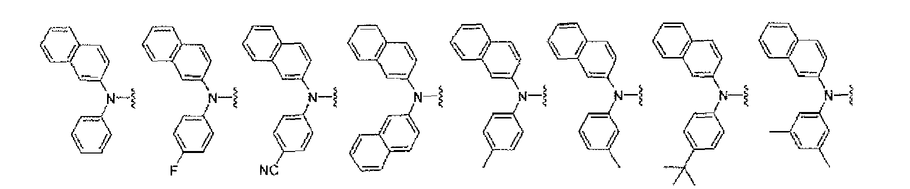

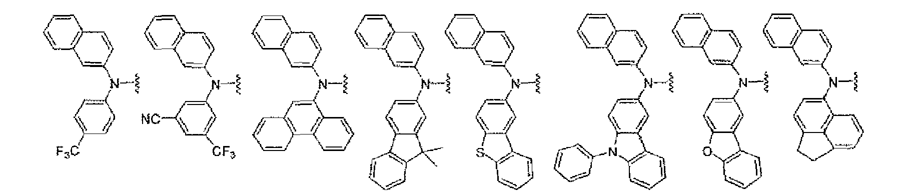

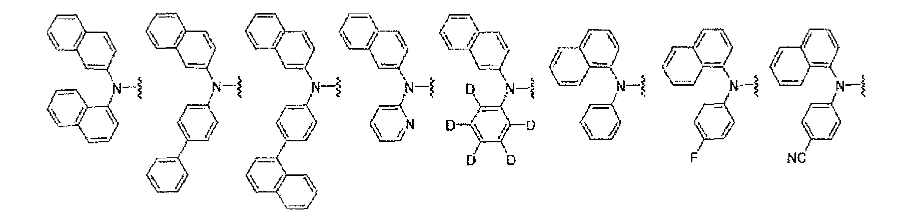

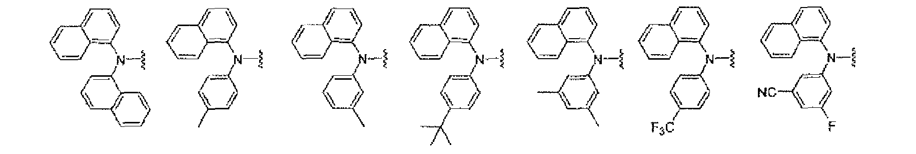

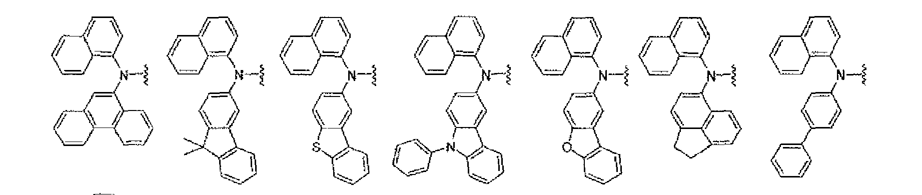

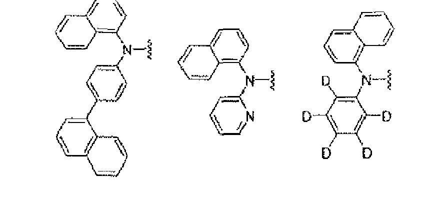

And, in the dopant compound represented by Chemical Formula 1, L is selected from the following structures, but not limited thereto: 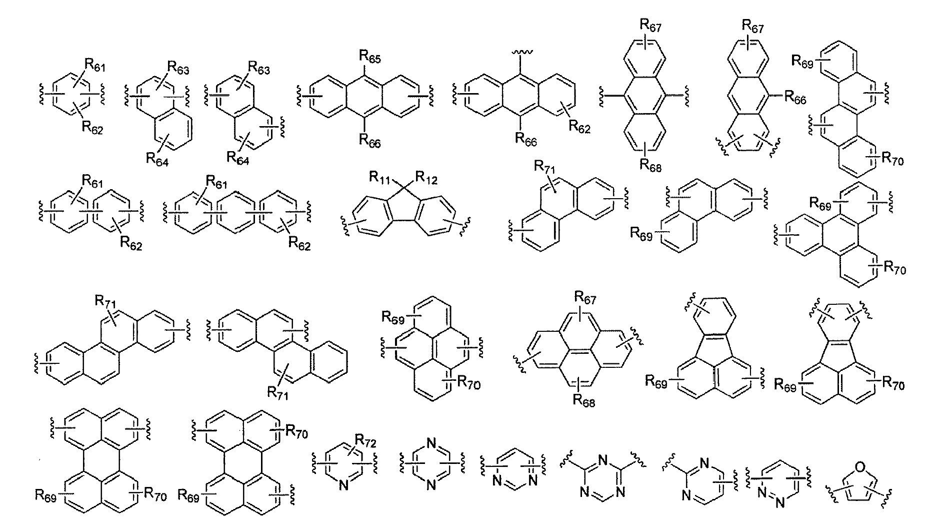 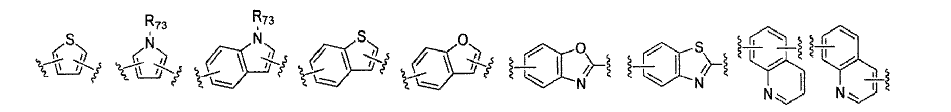 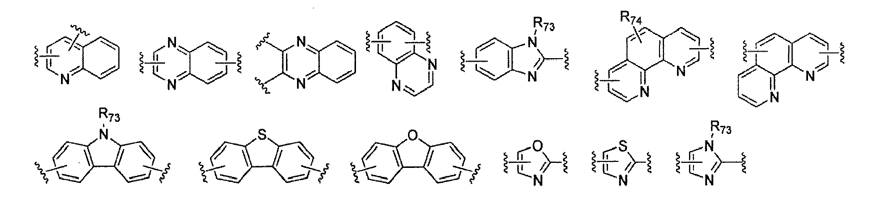 wherein
R₆₁ through R₇₄ independently represent hydrogen, deuterium, halogen, halo(C1-C60)alkyl, (C1-C60)alkyl, (C6-C60)aryl, (C3-C60)heteroaryl with or without (C6-C60)aryl substituent, 5- or 6-membered heterocycloalkyl containing one or more heteroatom(s) selected from N, O and S, 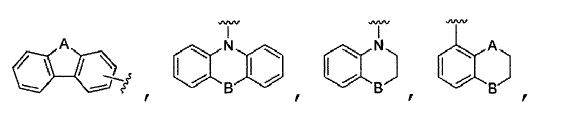 (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, and the alkyl, cycloalkyl, heterocycloalkyl, aryl, heteroaryl, arylsilyl, alkylsilyl, alkylamino or arylamino may be further substituted by one or more substituent(s) selected from deuterium, halogen, (C1-C60)alkyl, halo(C1-C60)alkyl, (C6-C60)aryl, (C3-C60) heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
A represents -C(R₁₂)(R₁₃)-, -N(R₁₄)-, -S-, -O- or -Si(R₁₅)(R₁₆)-;
B represents a chemical bond, -(CR₁₂R₁₃)ₘ-, -N(R₁₄)-, -S-, -O-, -Si-(R₁₅)(R₁₆)- or -(R₂₅)C=C(R₂₆)-;
R₁₂ through R₁₆, R₂₅ and R₂₆ independently represent hydrogen, deuterium, halogen, (C1-C60)alkyl, (C6-C60)aryl, (C3-C60)heteroaryl containing one ormore heteroatom (s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, amino, mono- or di-(C1-C60)alkylamino, mono- or di-(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₂ and R₁₃, and R₁₅ and R₁₆ may be linked via (C3-C60)alkylene or (C3-C60) alkenylene with or without a fused ring to form an alicyclic ring or a mono- or polycyclic ring; and
m represents an integer from 1 to 4.

Specifically, the dopant compound represented by Chemical Formula 1 may be exemplified by the following compounds, but not limited thereto:

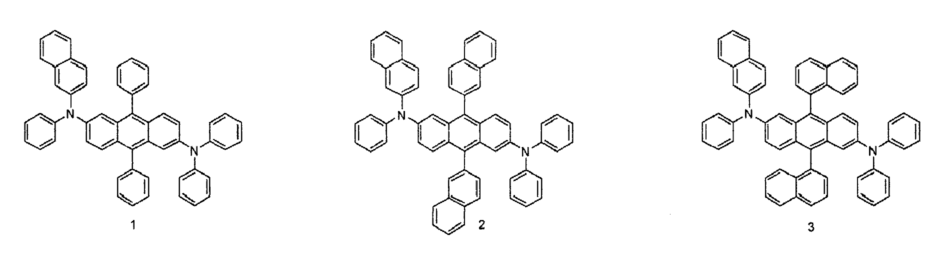

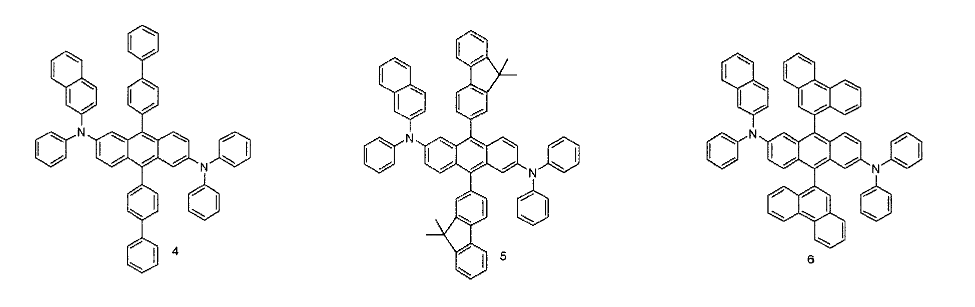

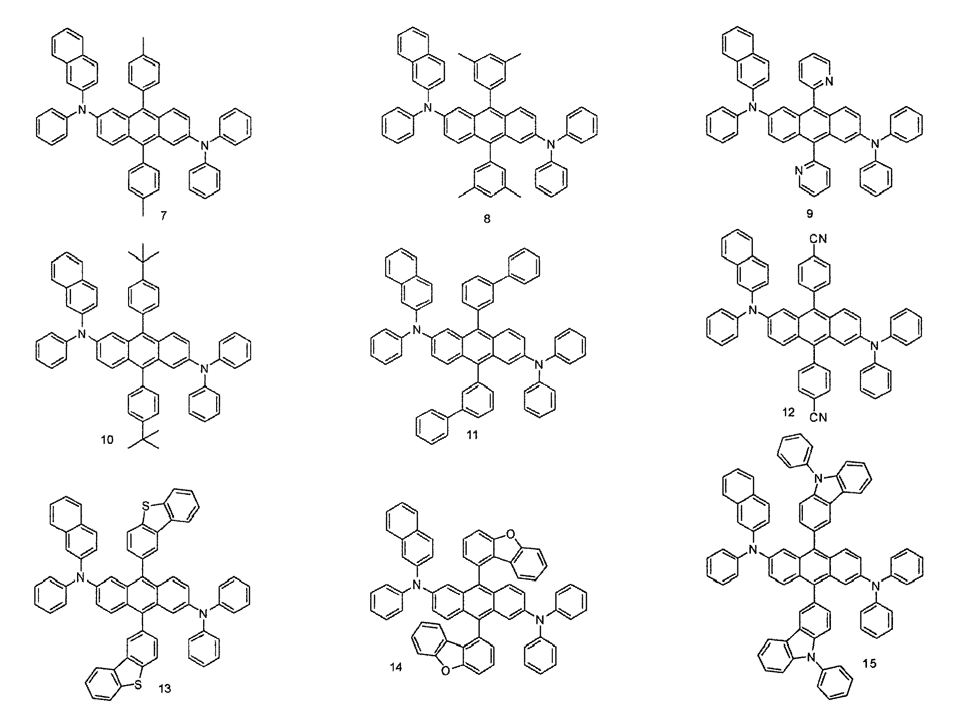

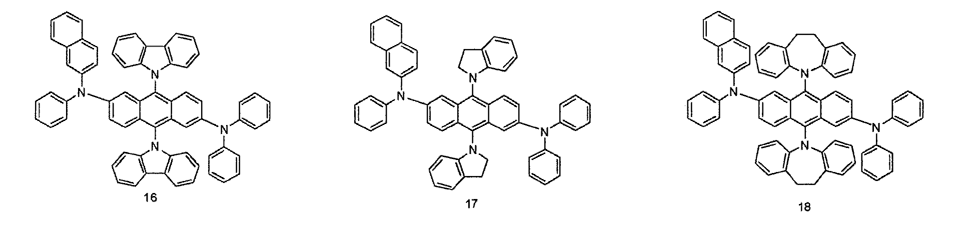

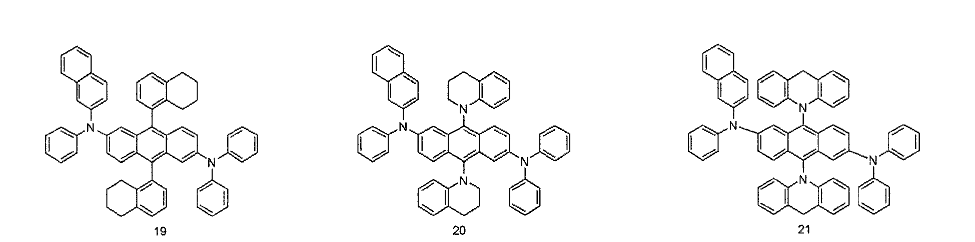

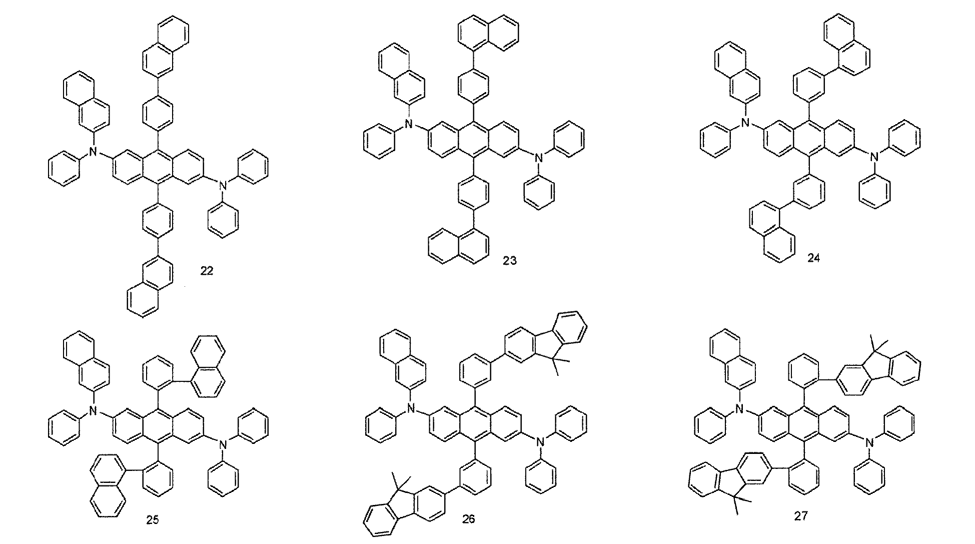

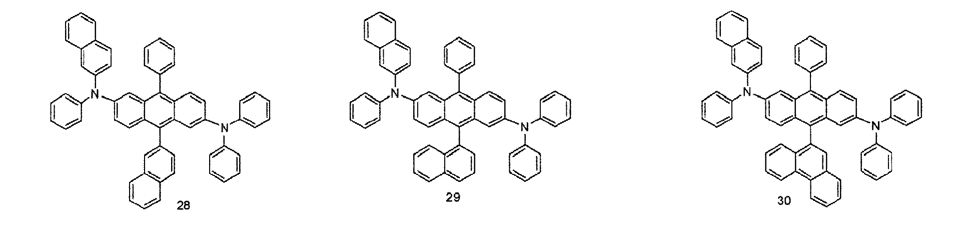

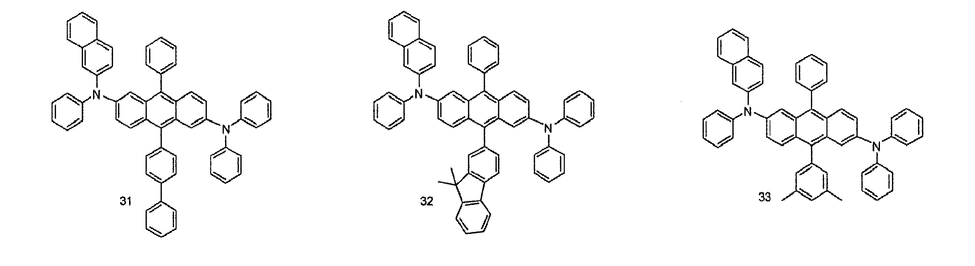

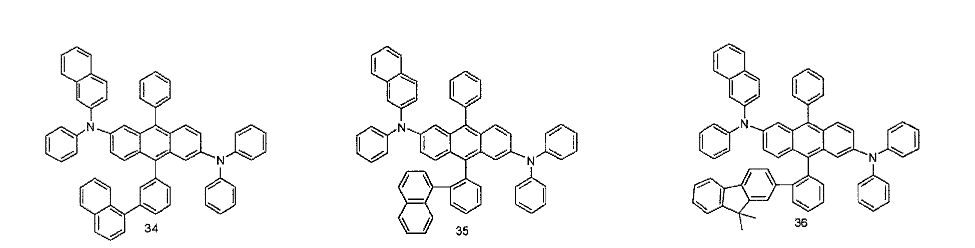

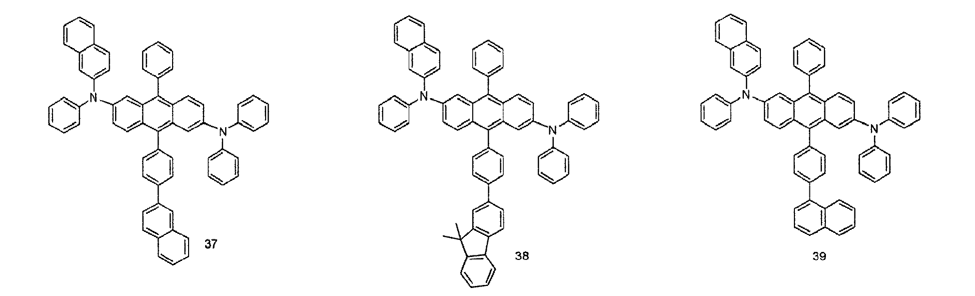

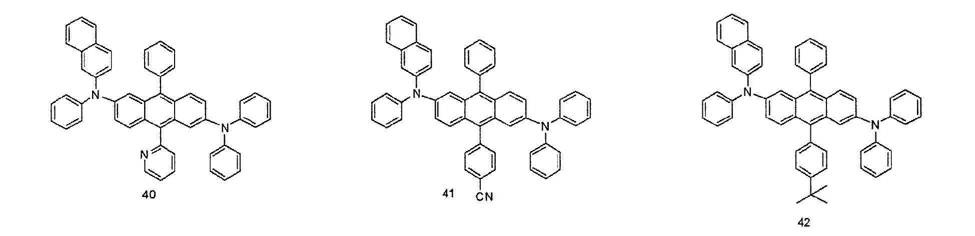

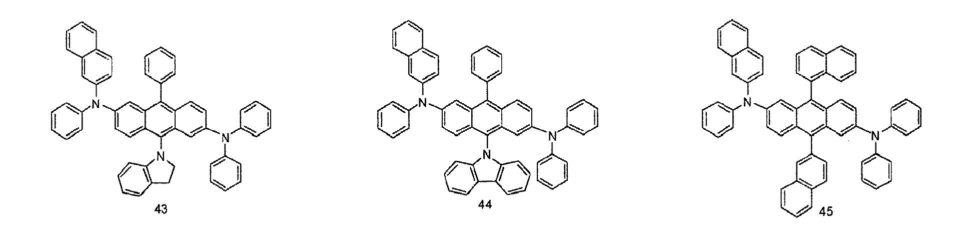

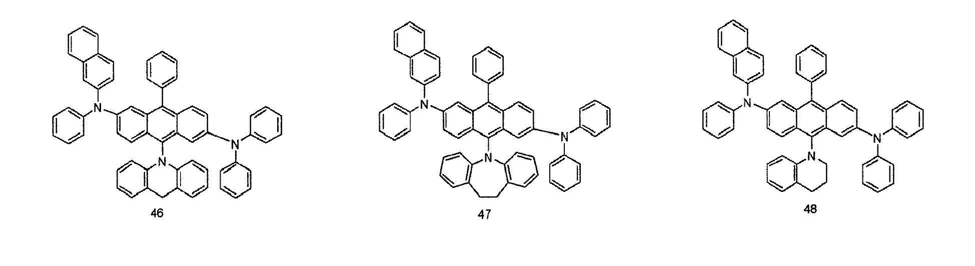

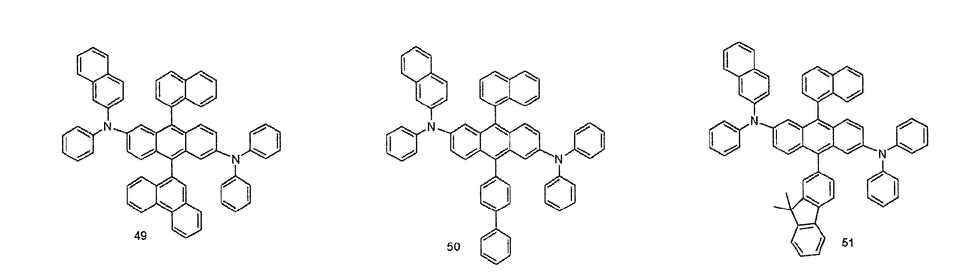

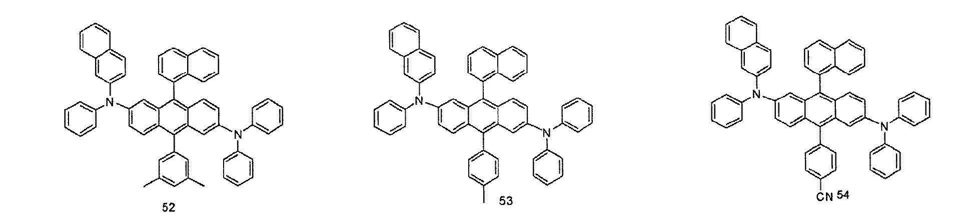

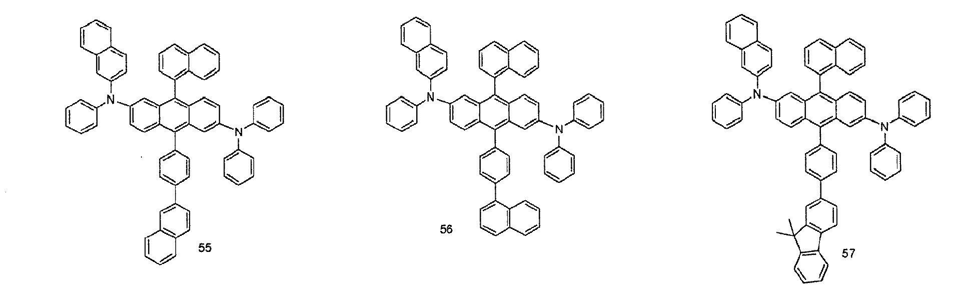

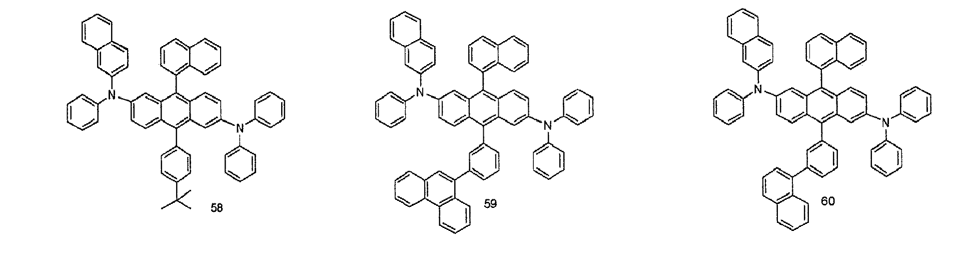

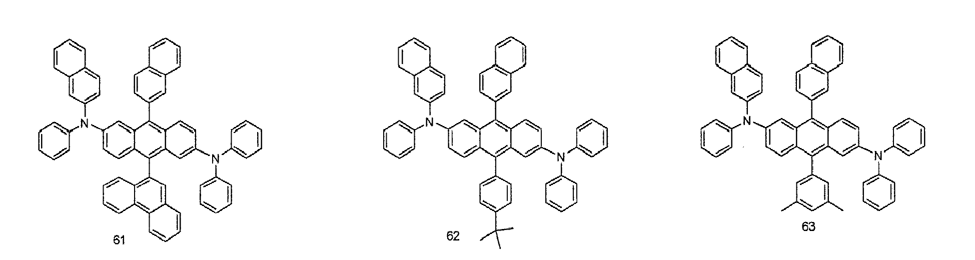

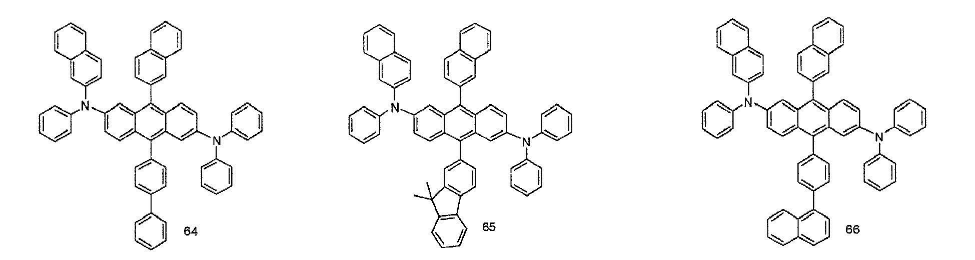

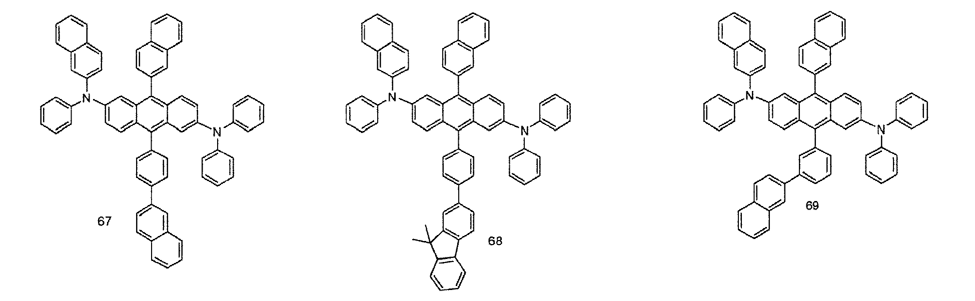

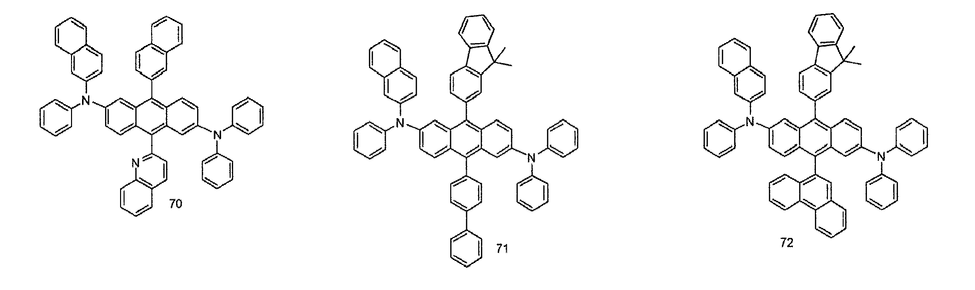

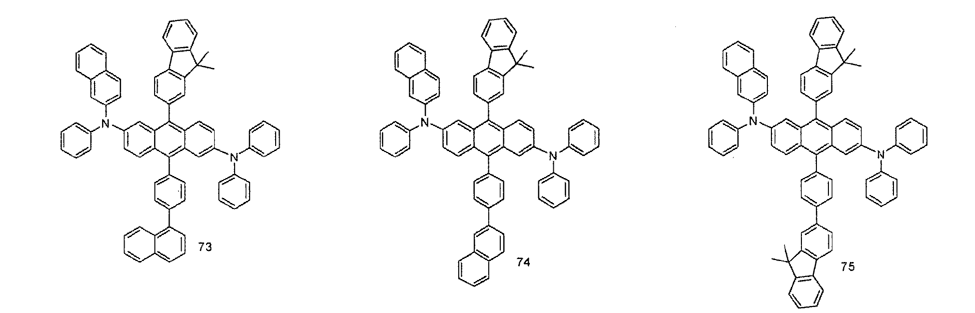

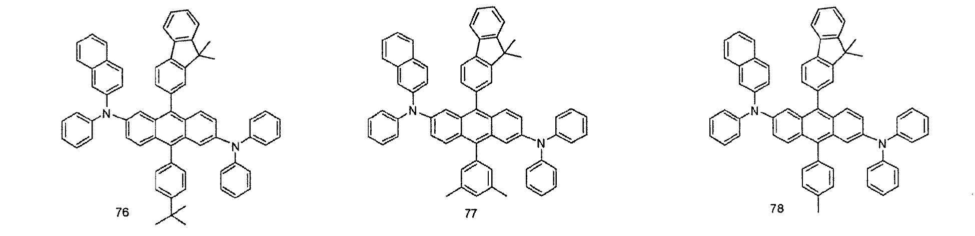

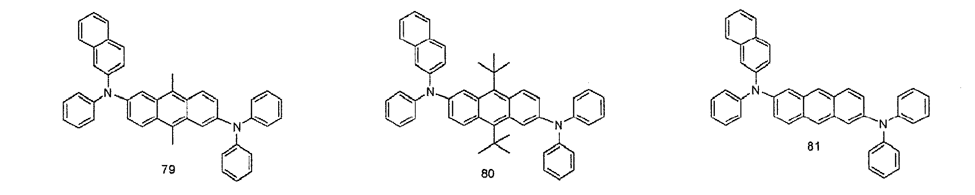

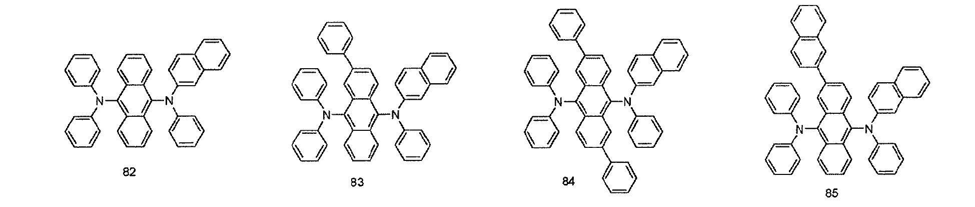

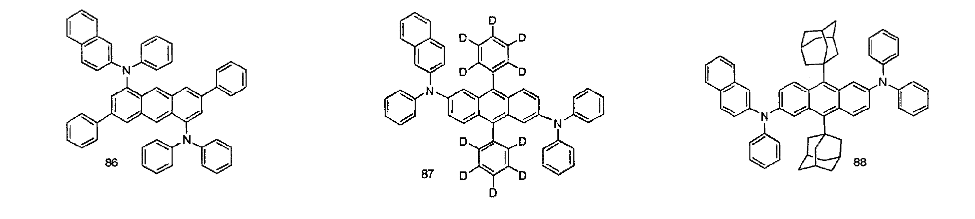

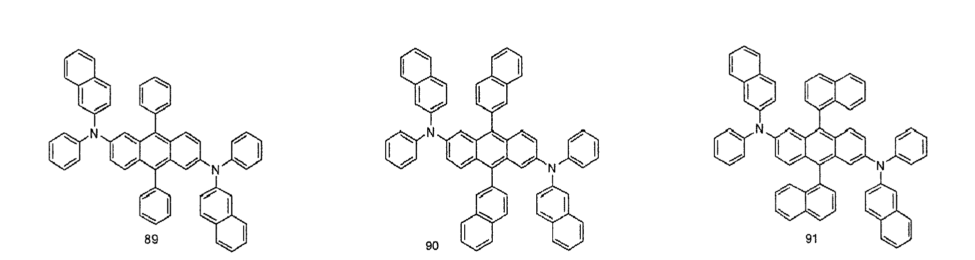

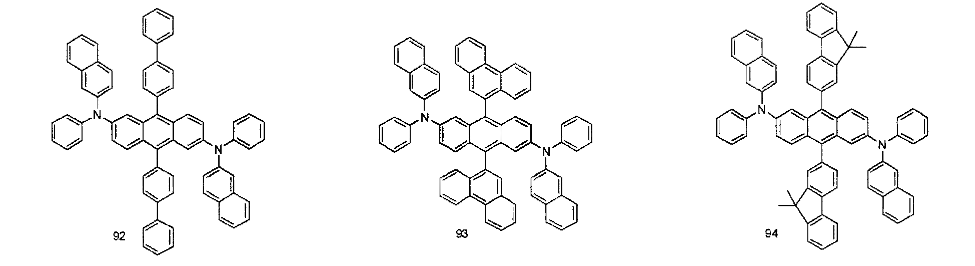

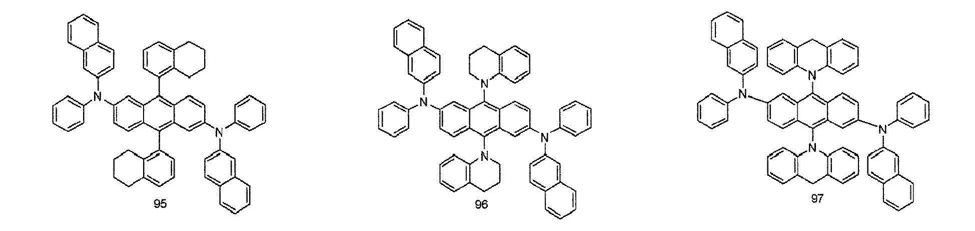

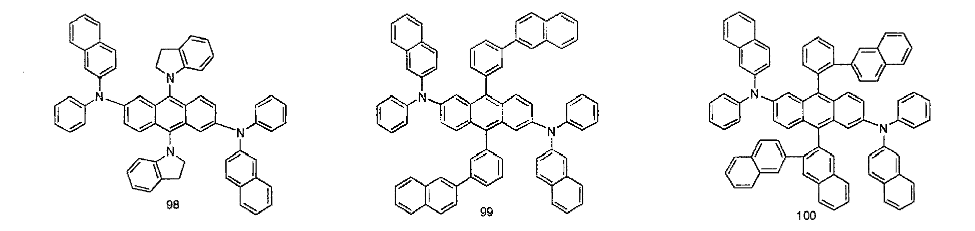

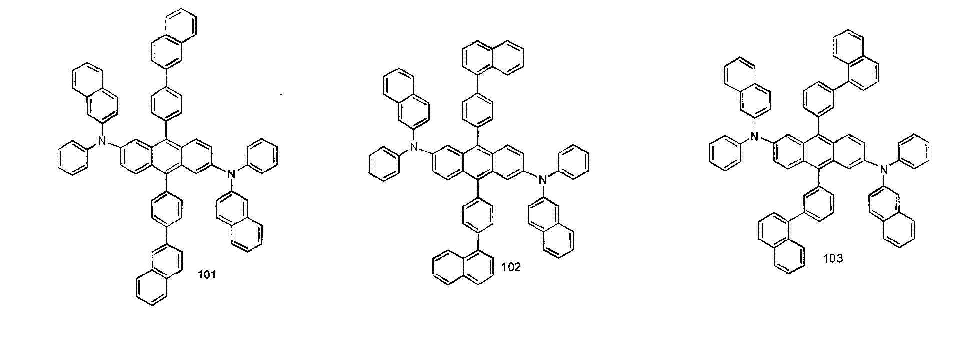

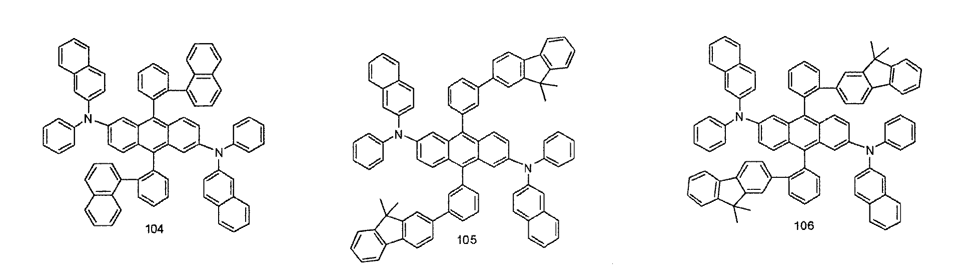

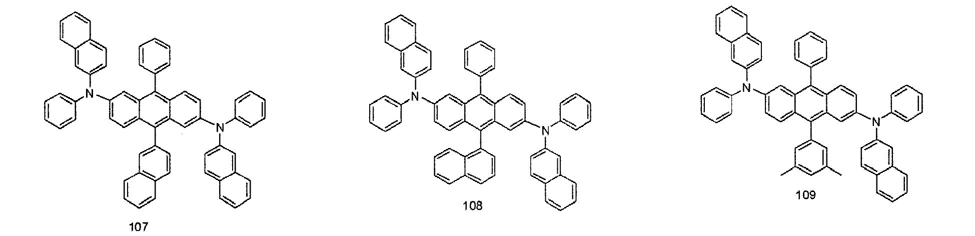

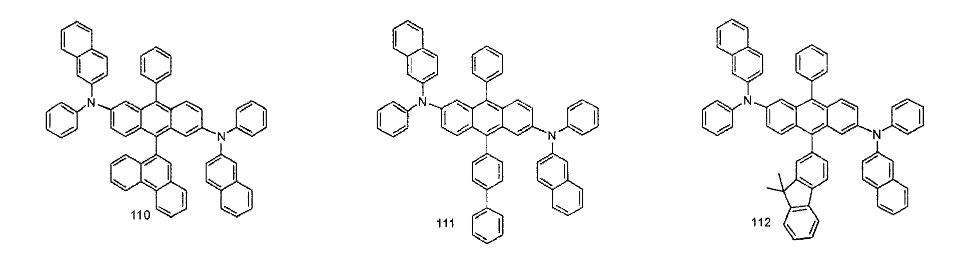

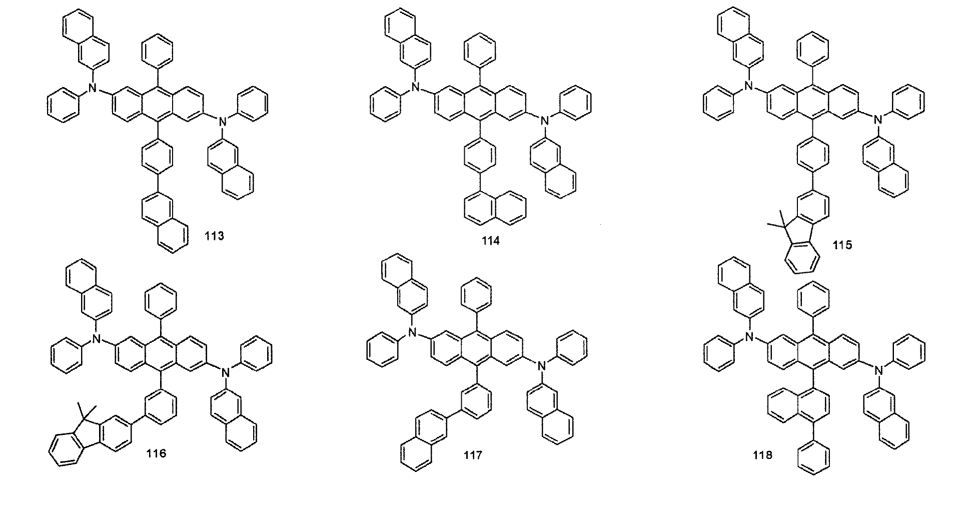

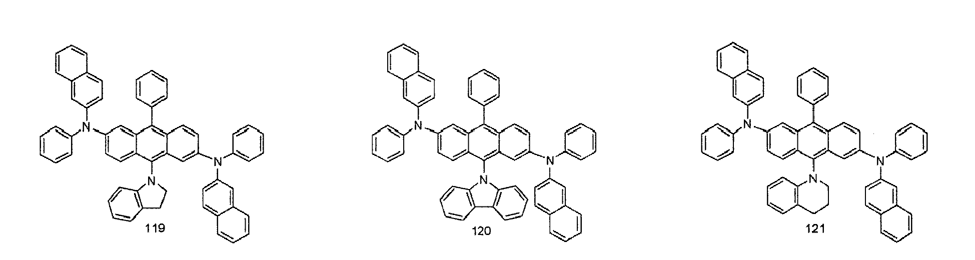

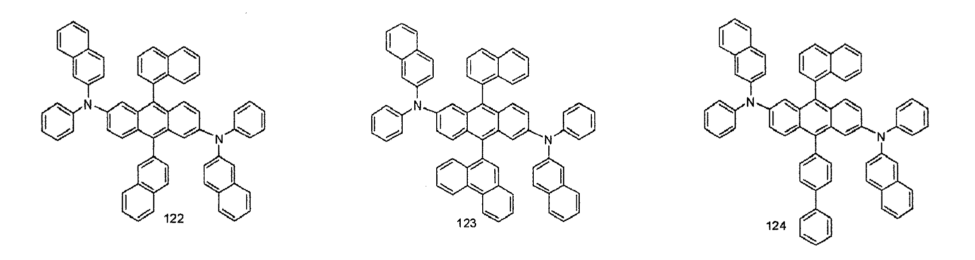

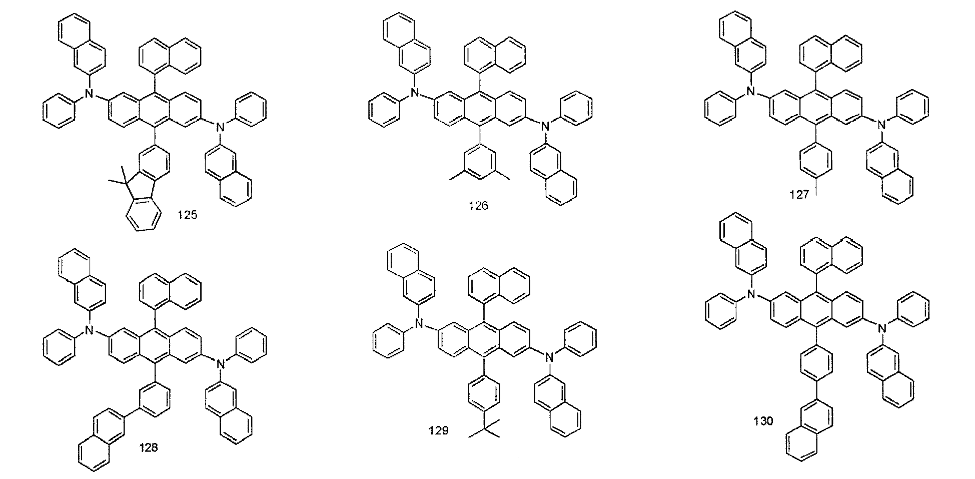

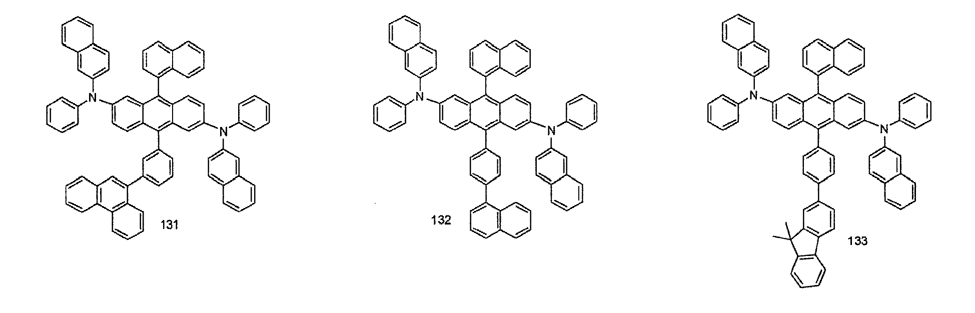

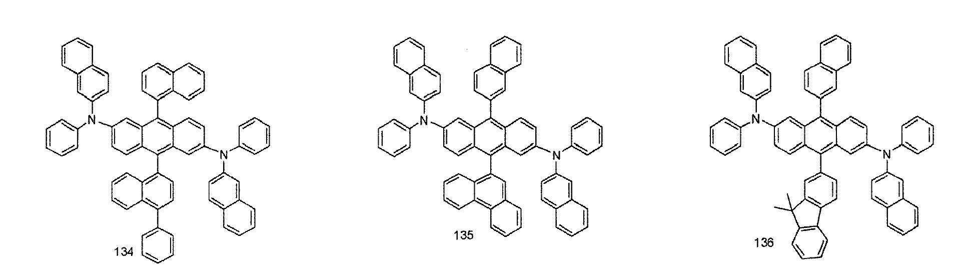

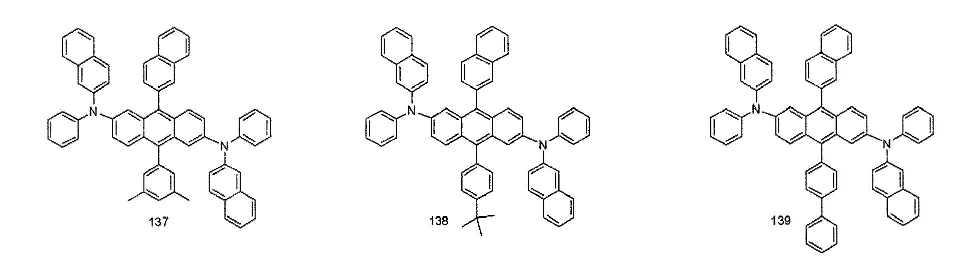

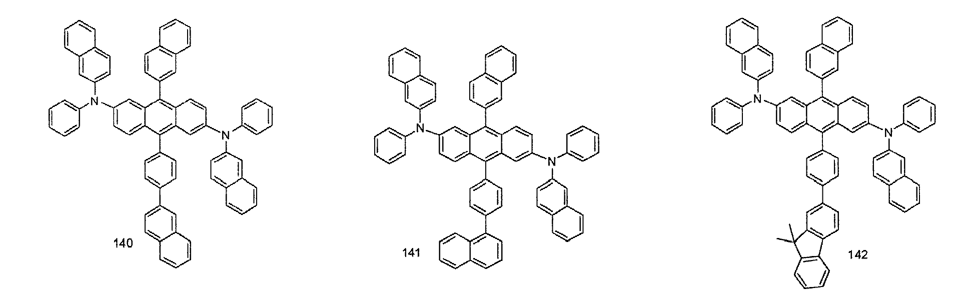

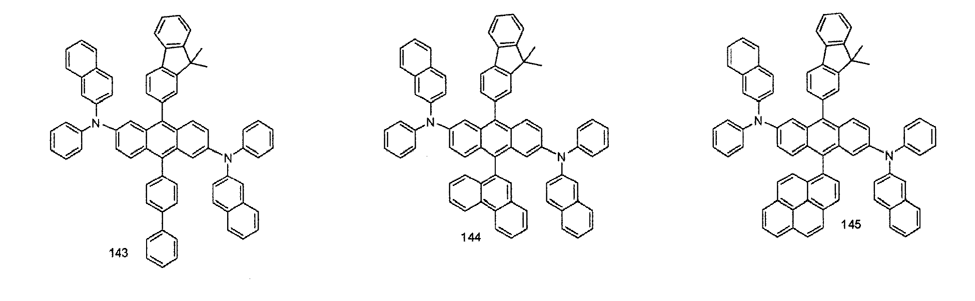

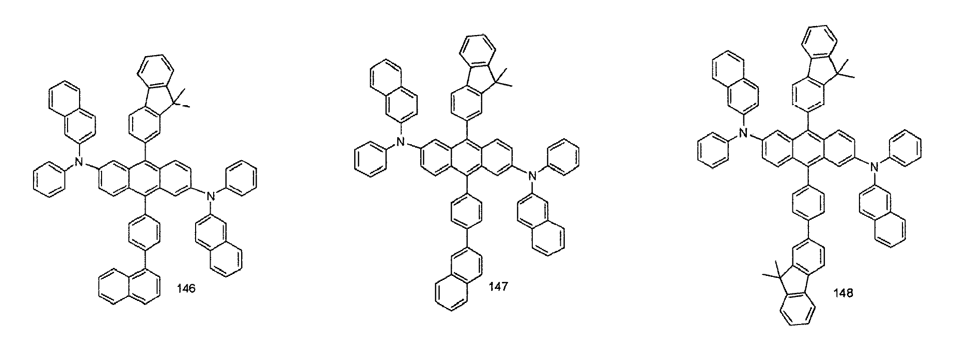

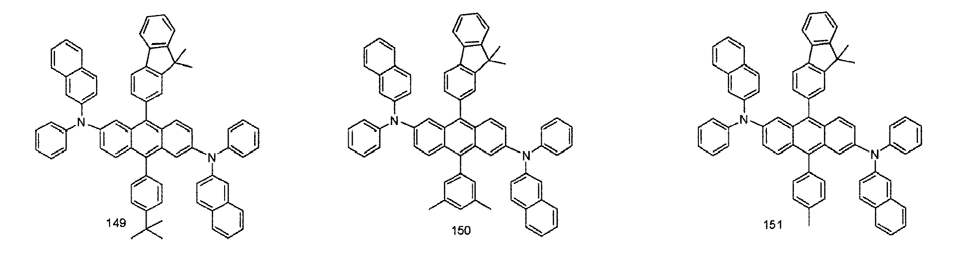

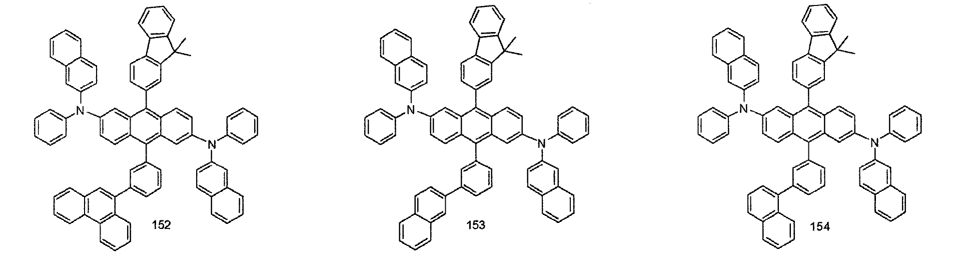

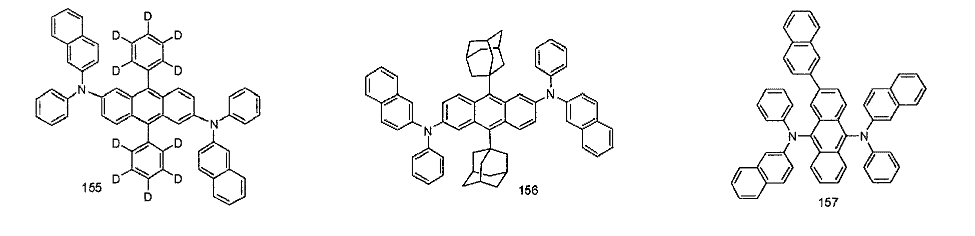

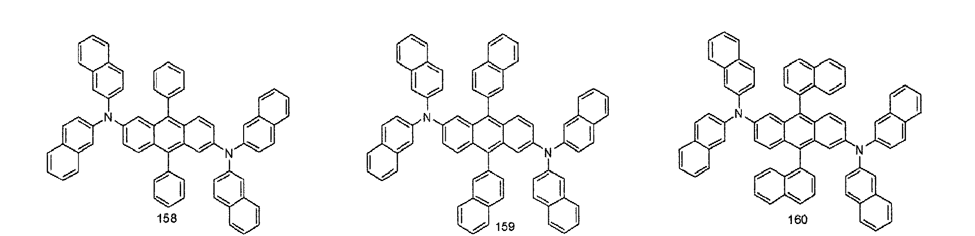

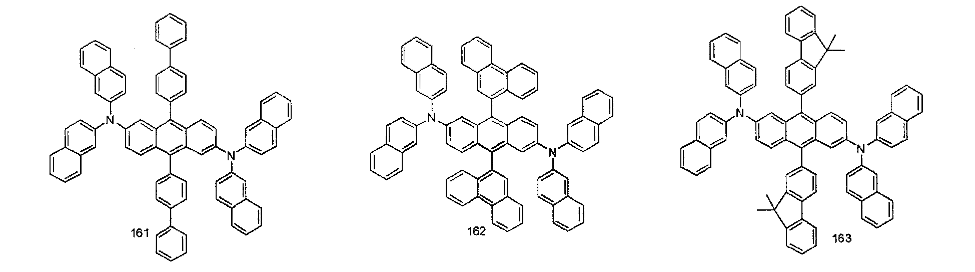

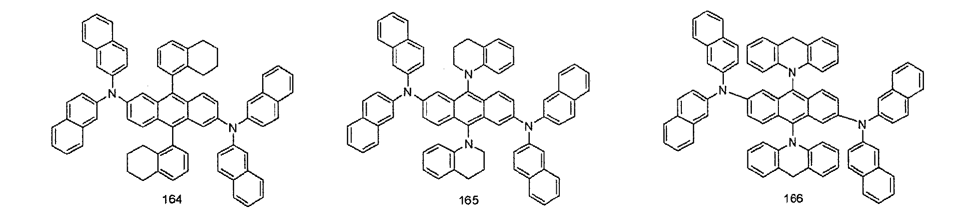

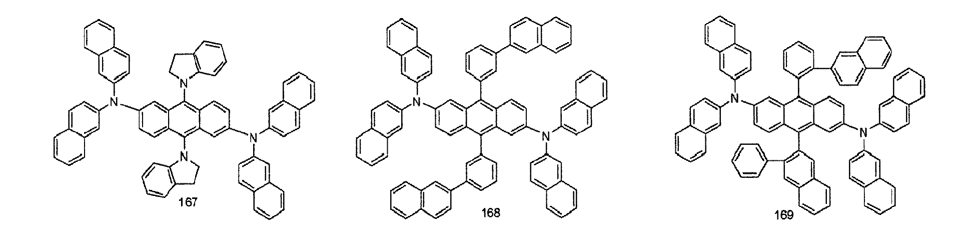

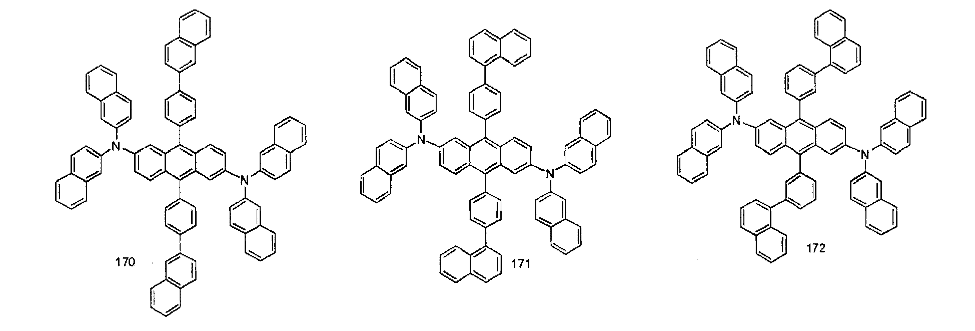

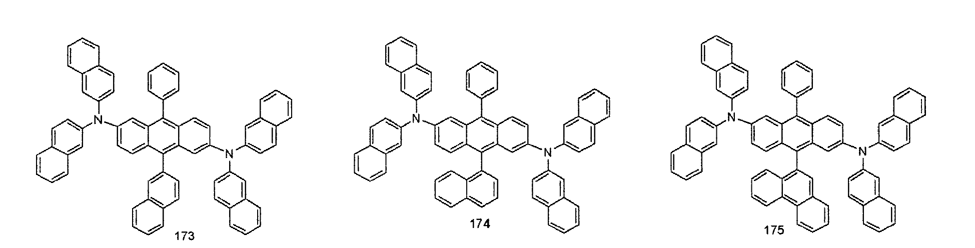

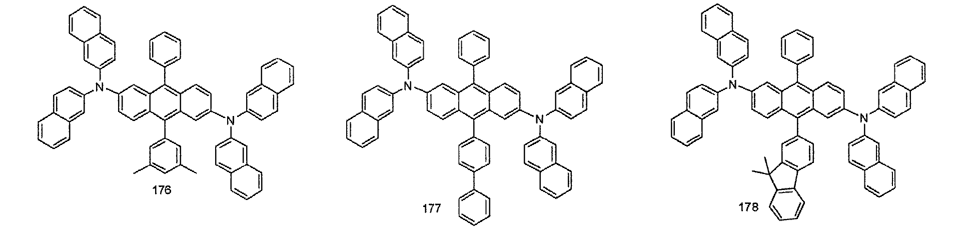

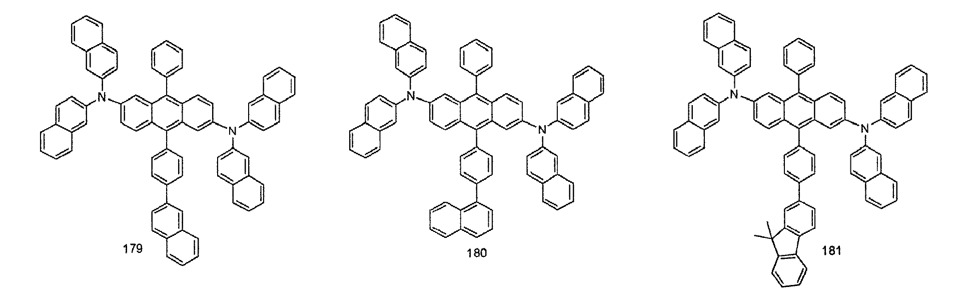

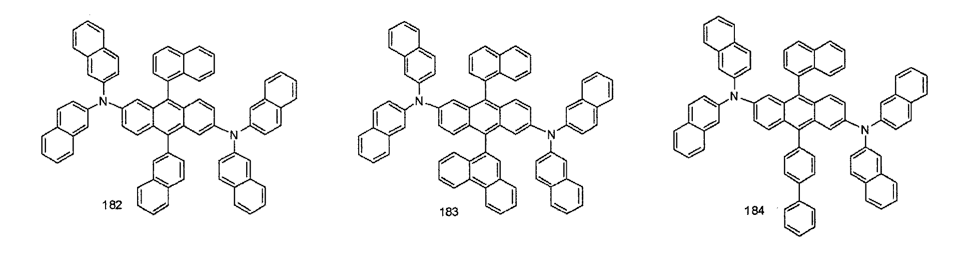

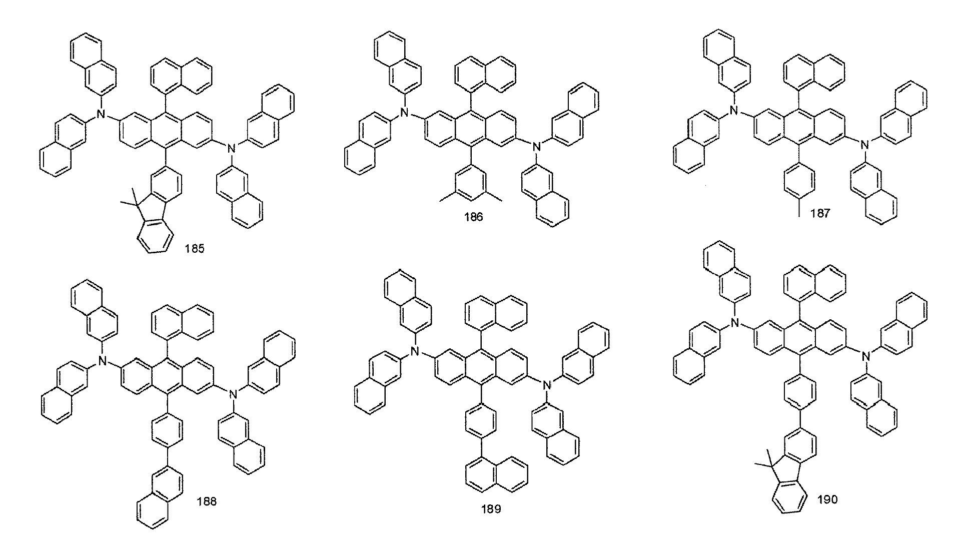

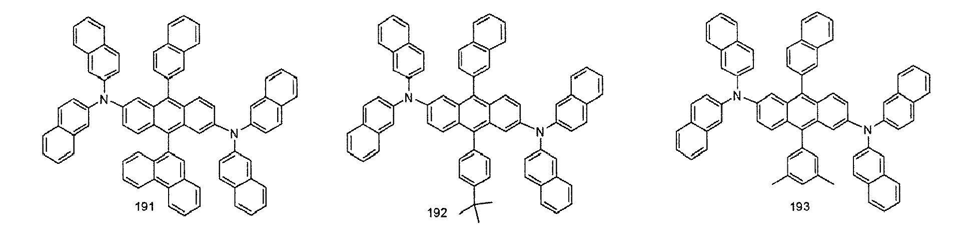

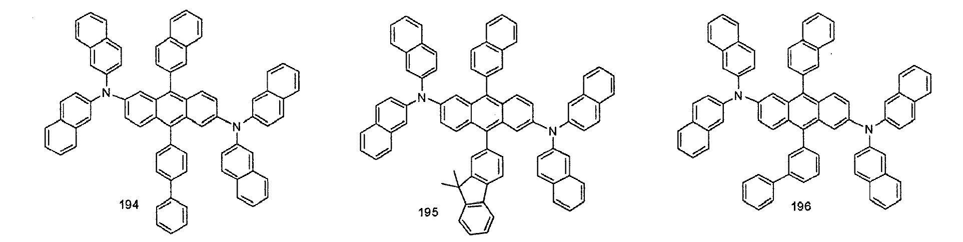

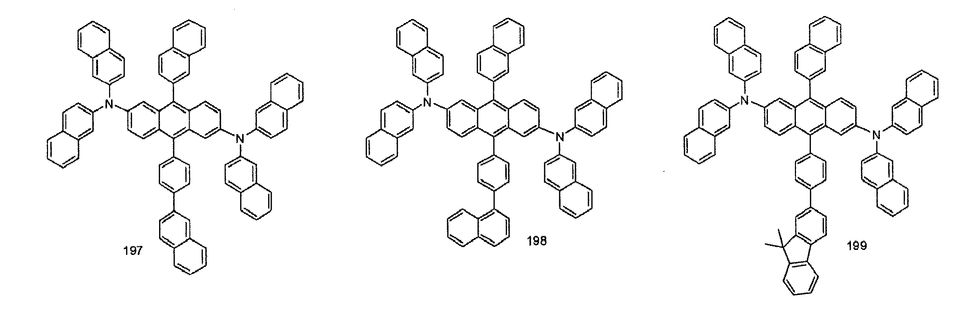

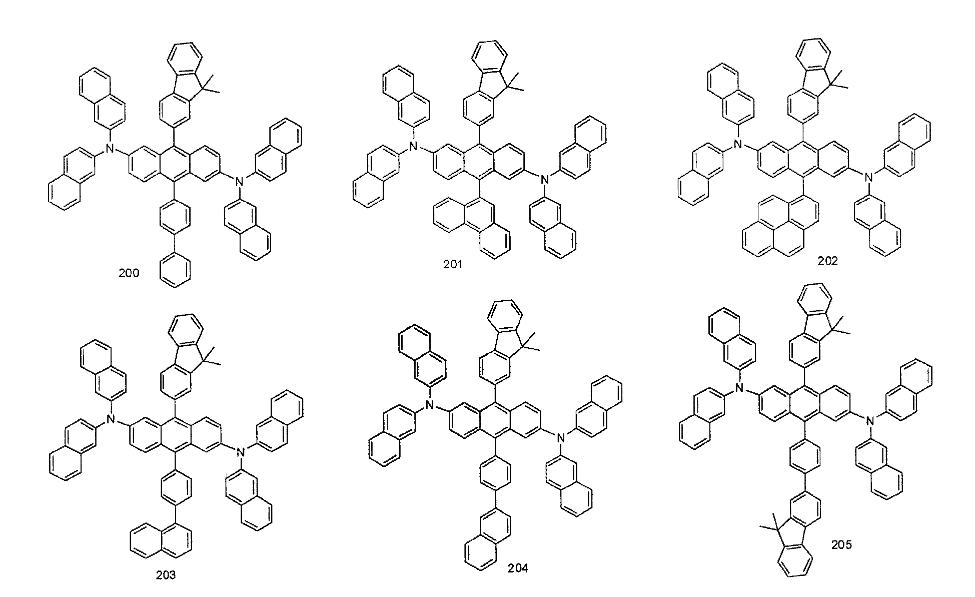

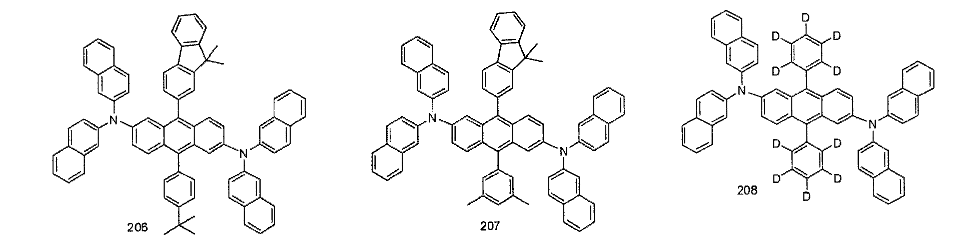

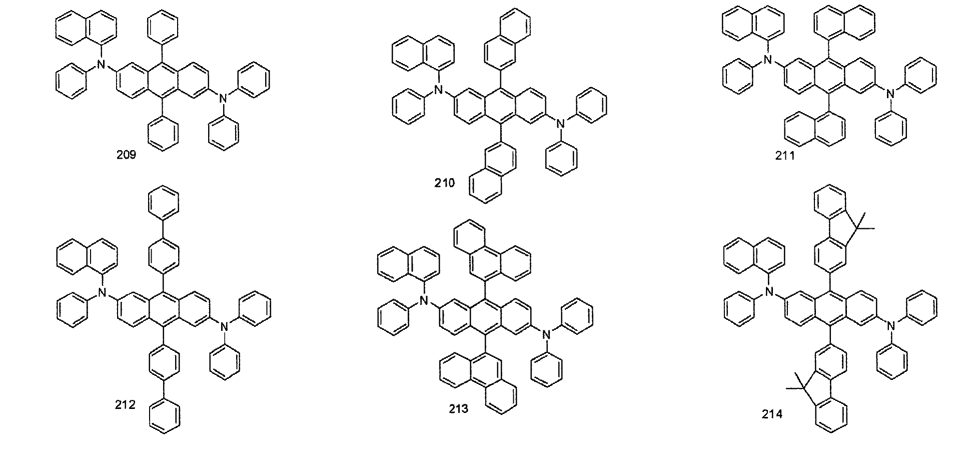

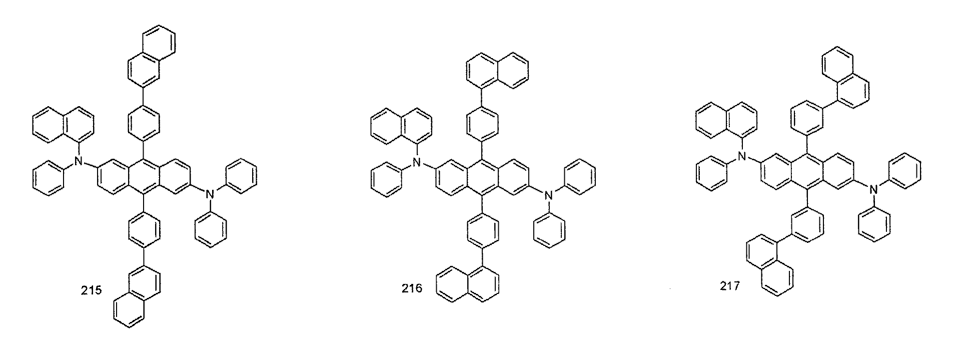

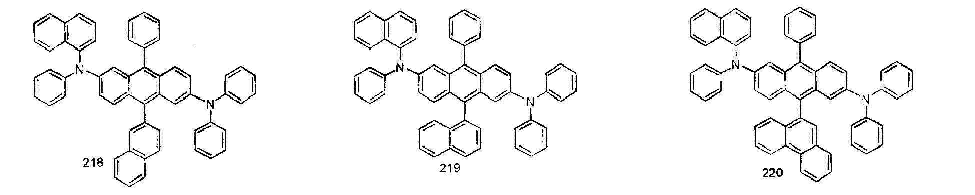

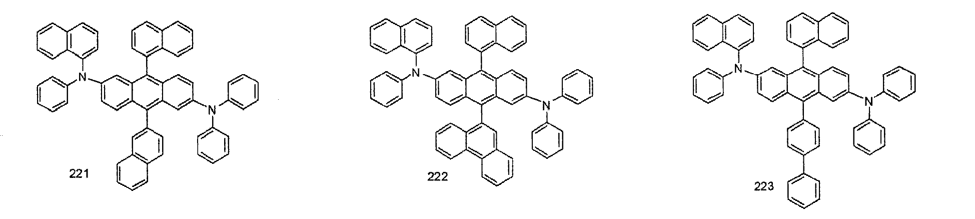

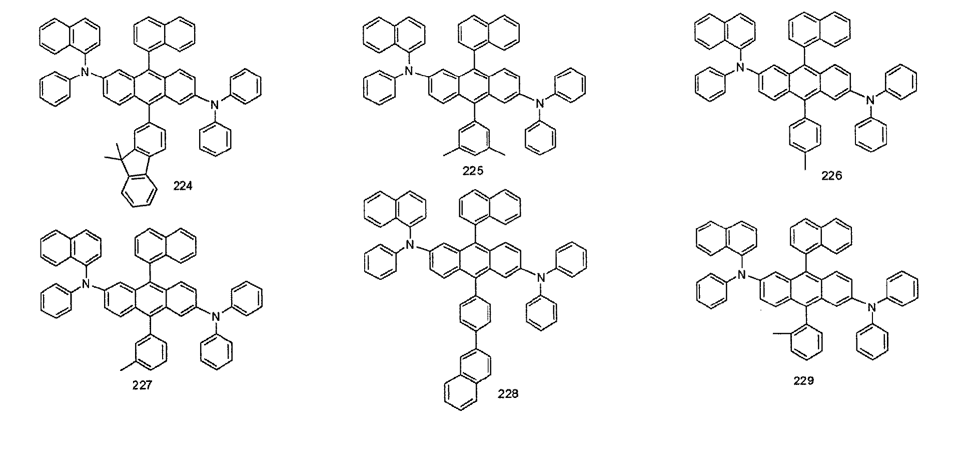

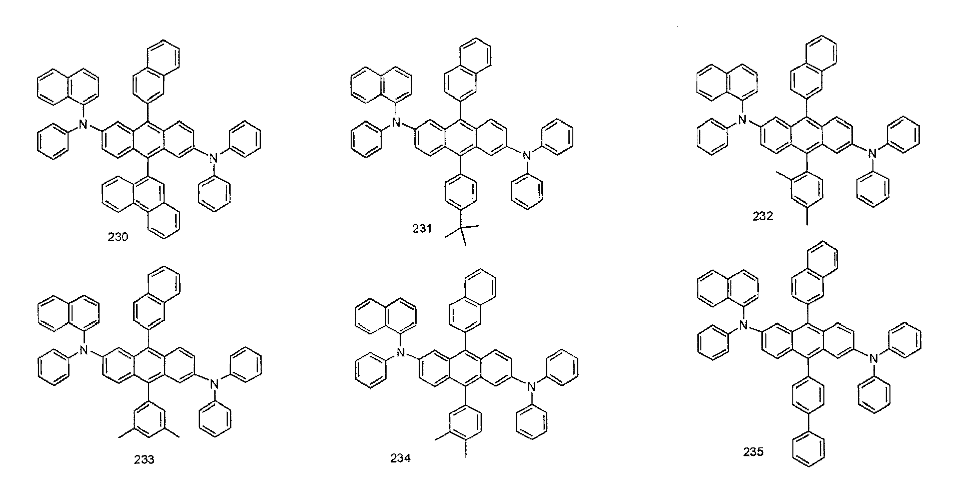

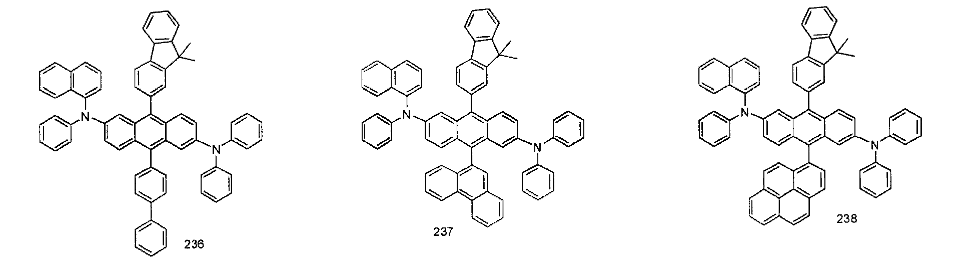

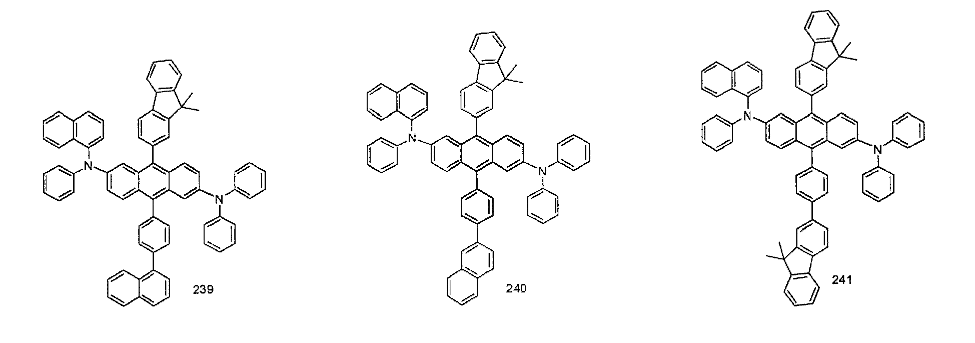

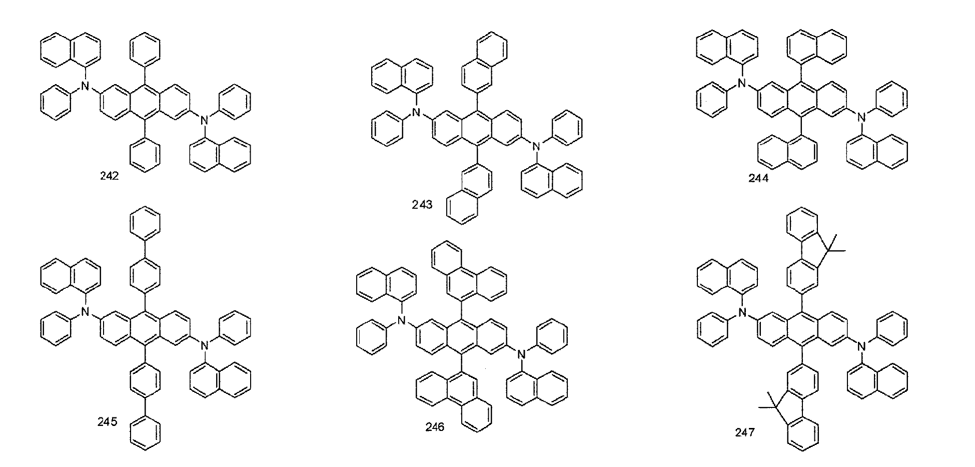

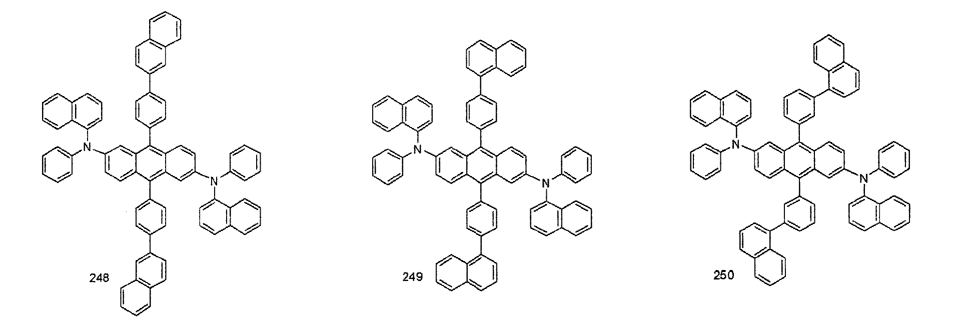

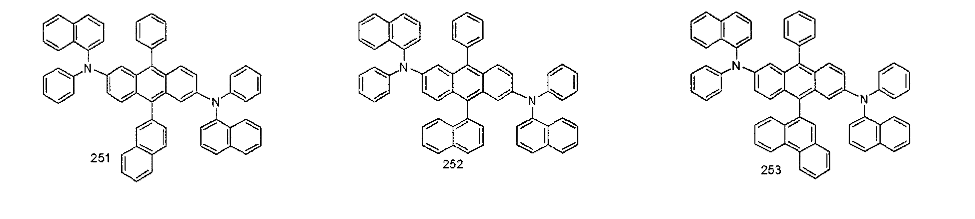

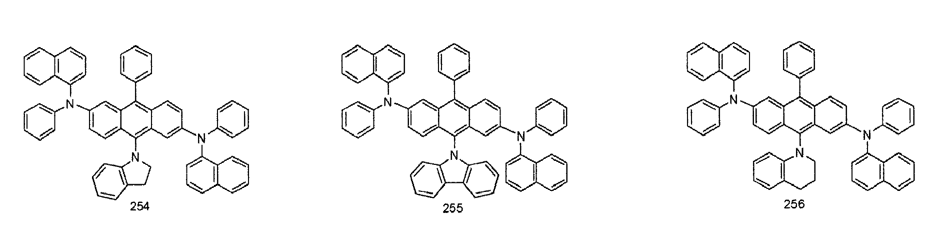

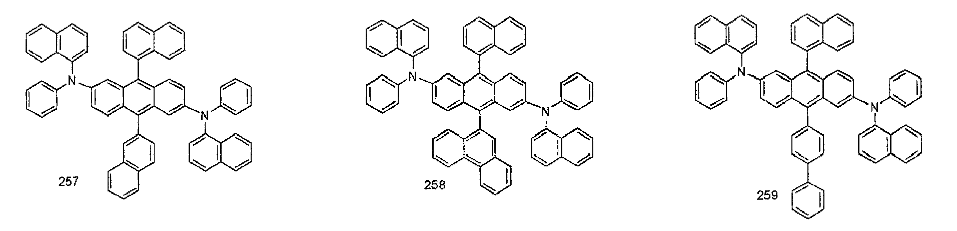

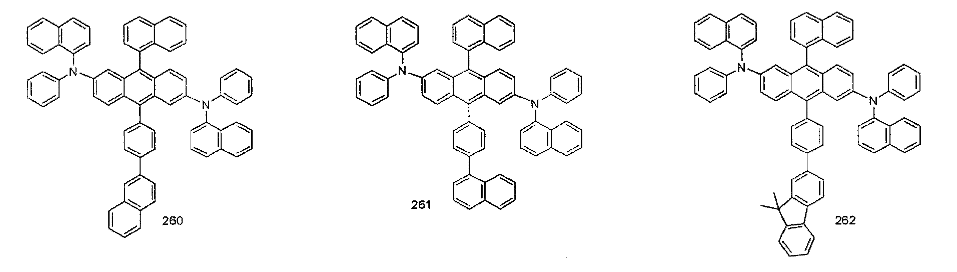

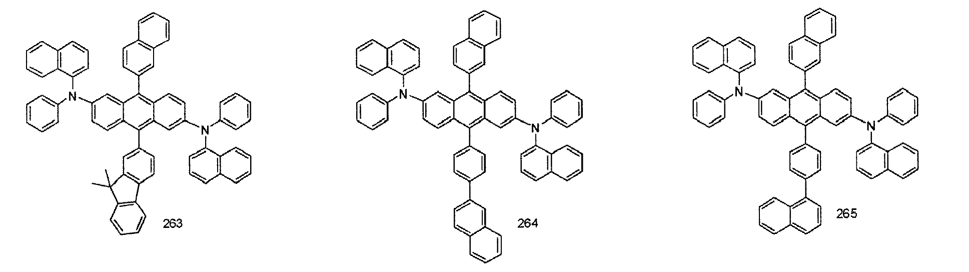

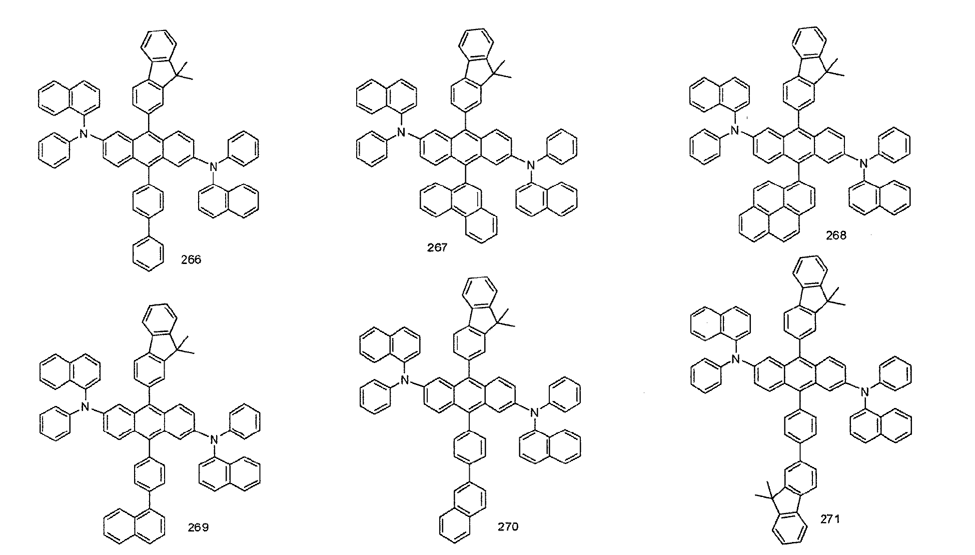

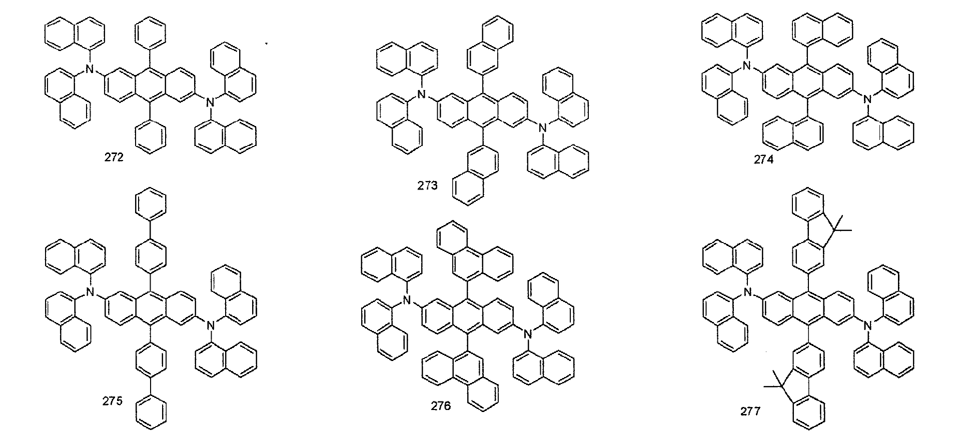

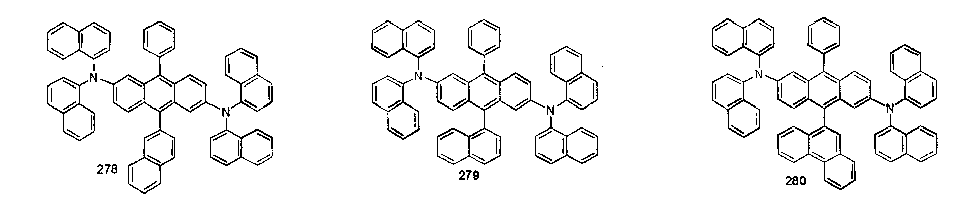

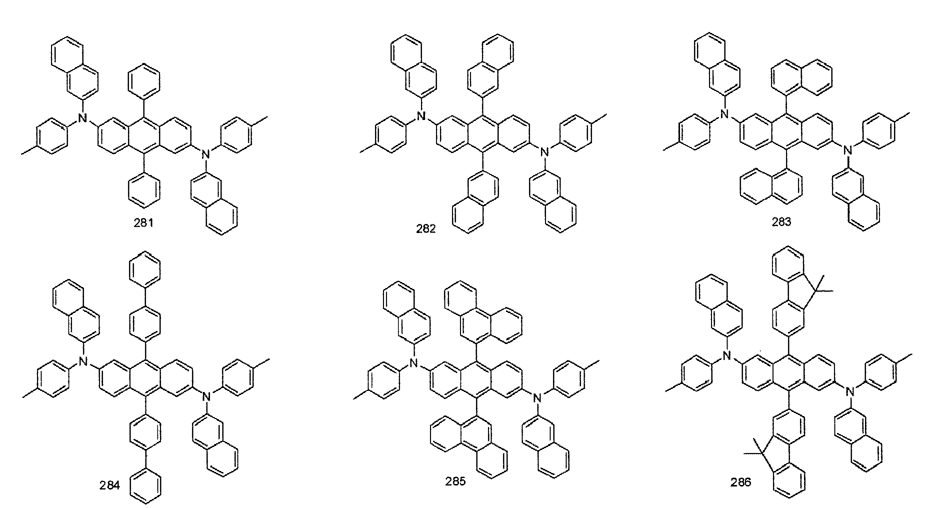

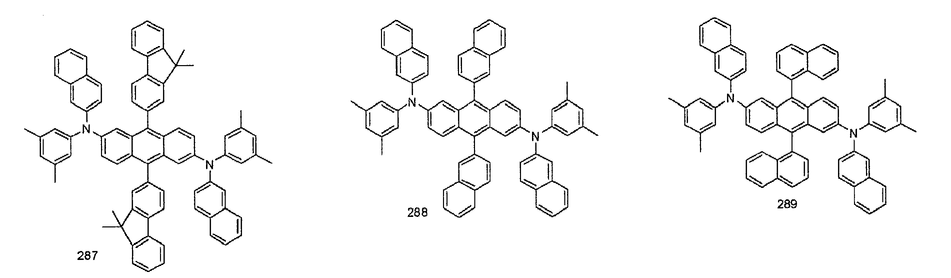

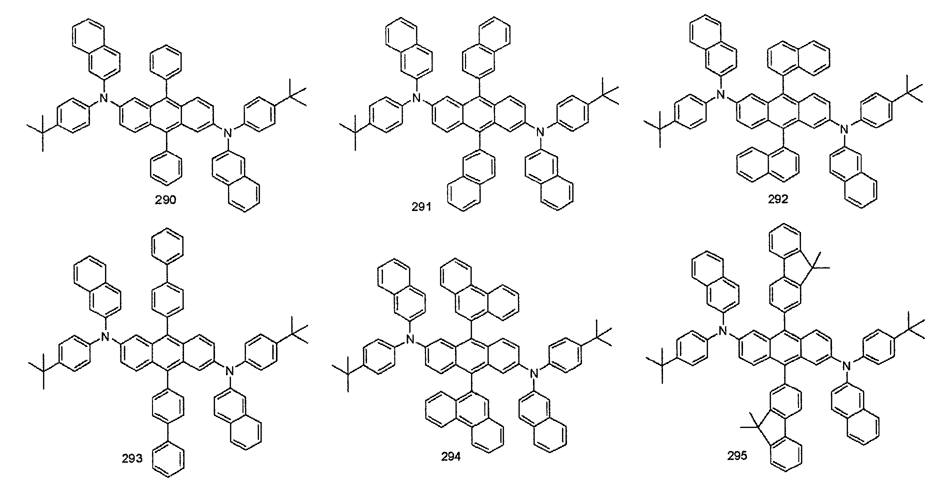

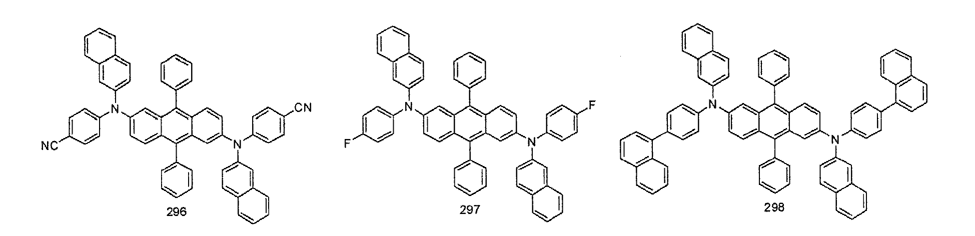

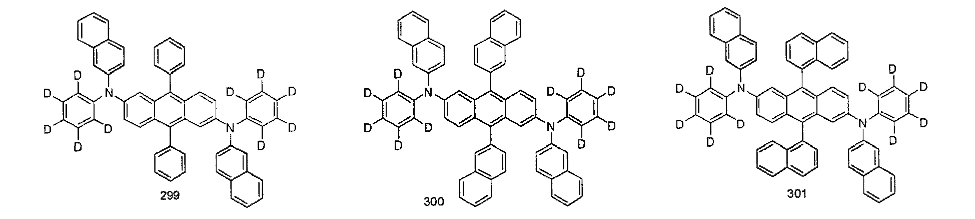

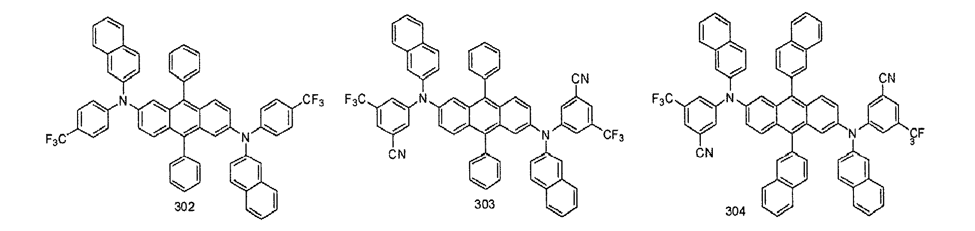

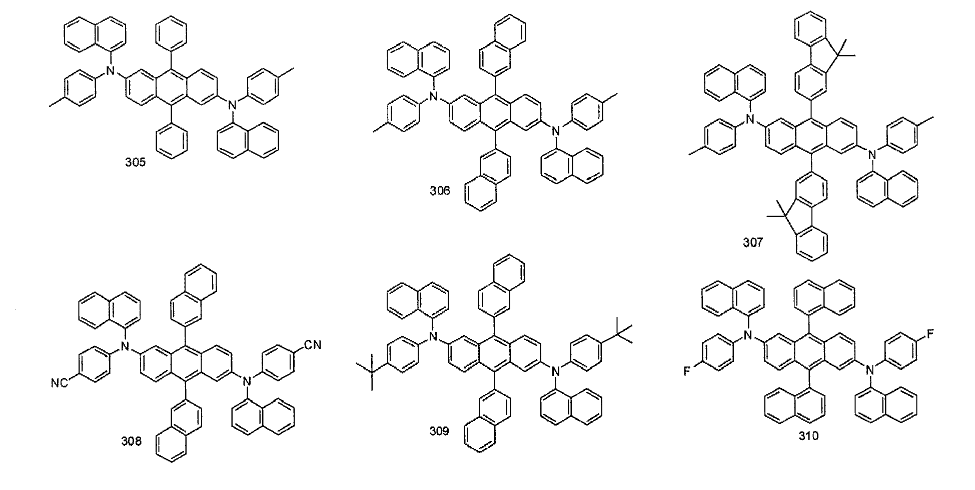

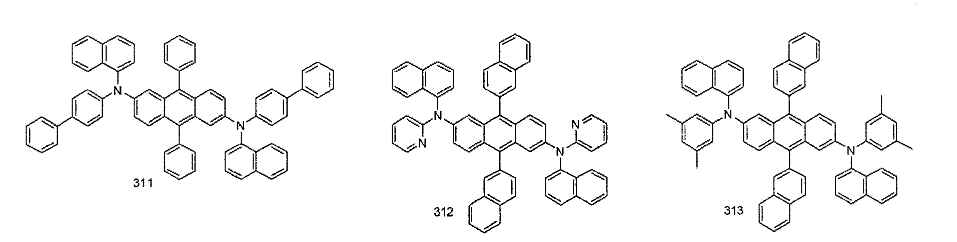

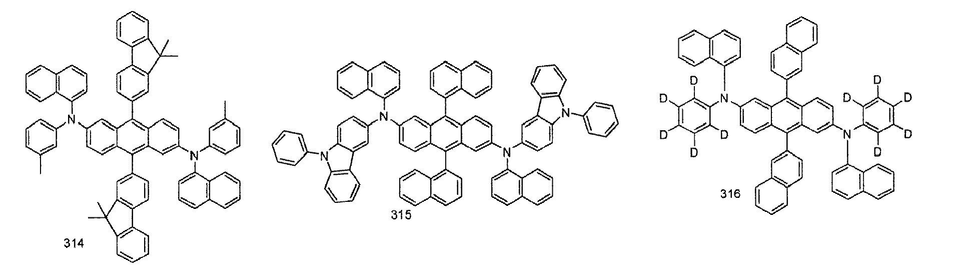

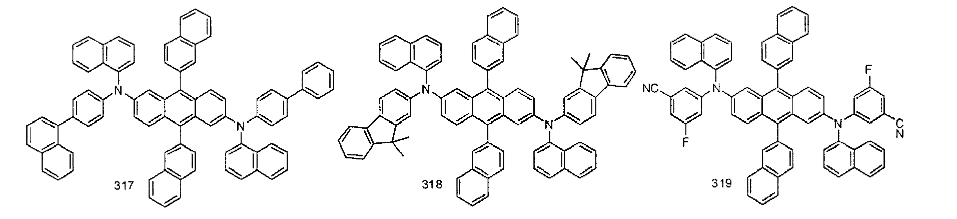

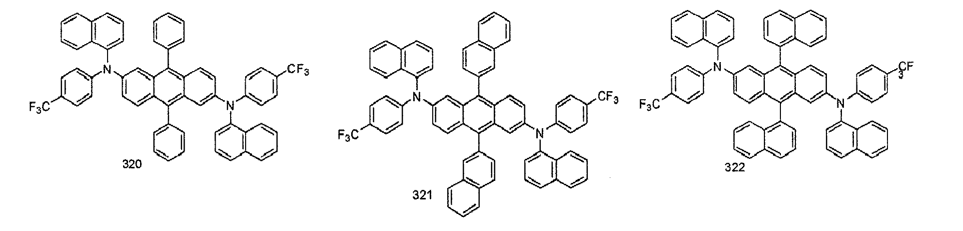

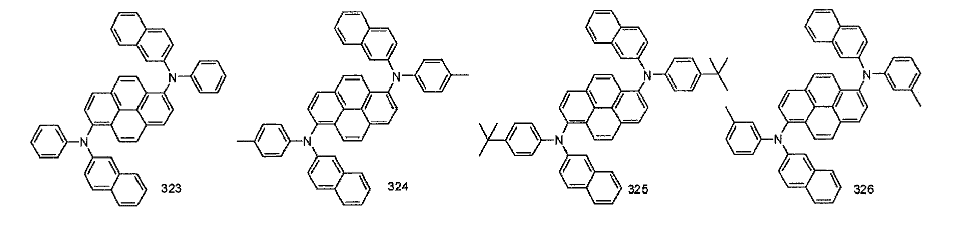

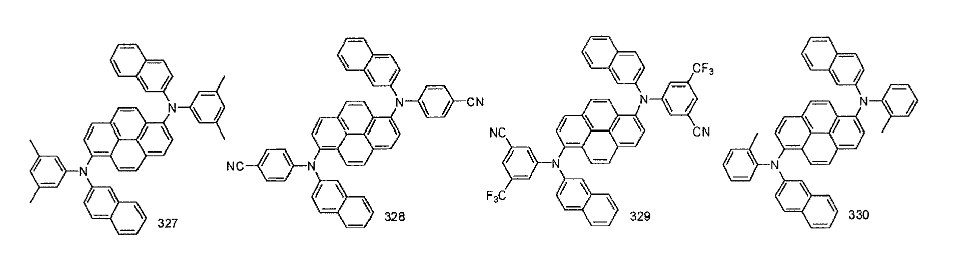

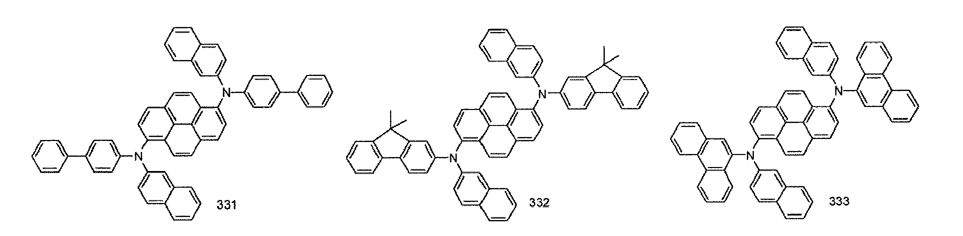

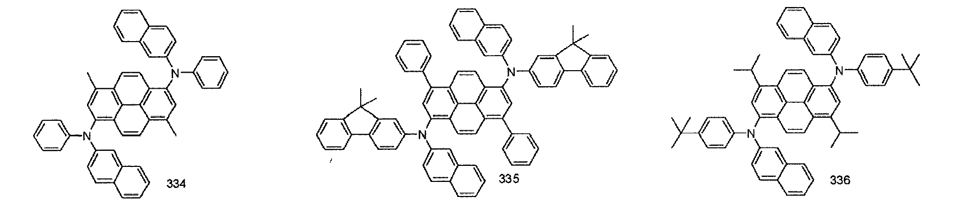

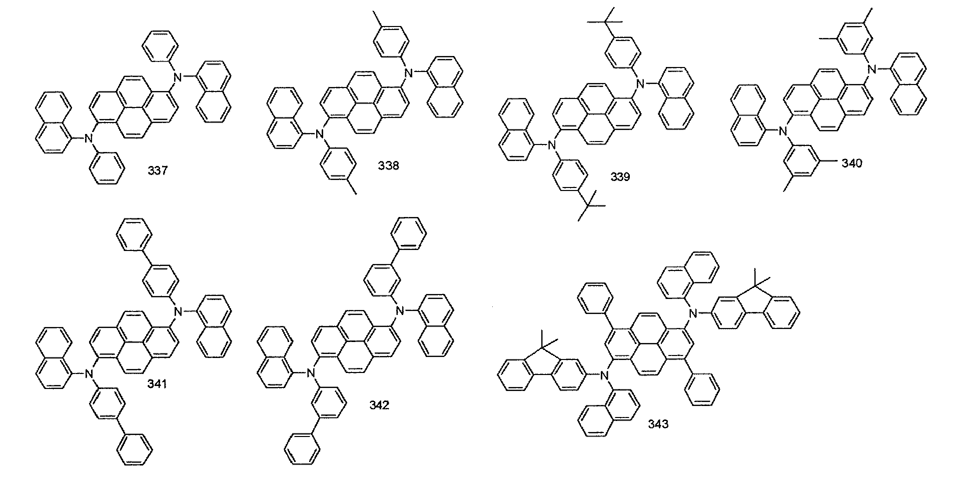

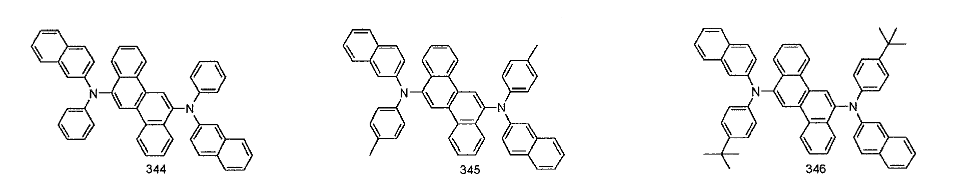

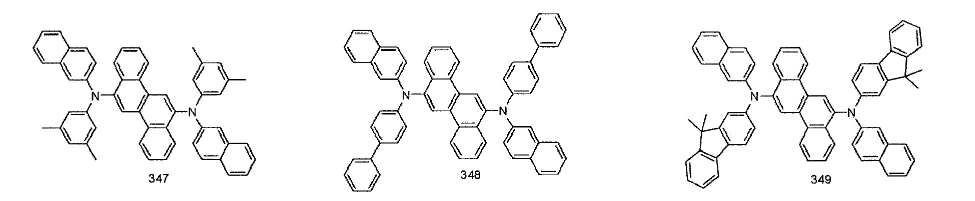

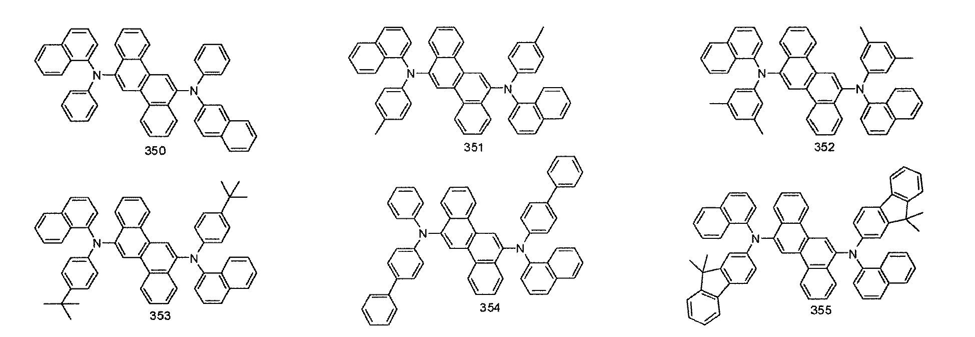

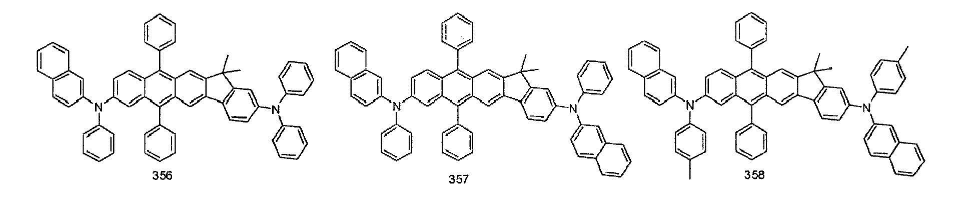

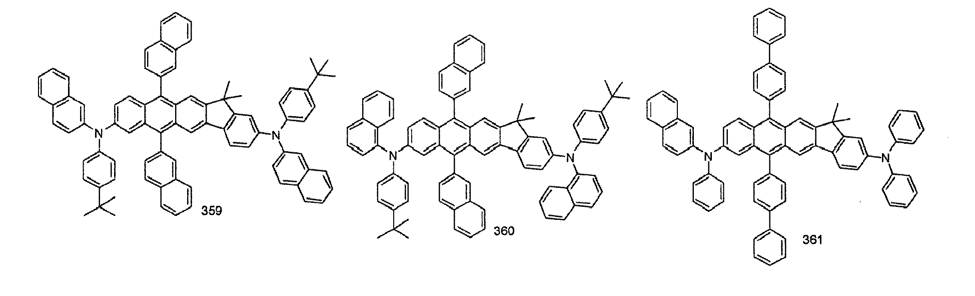

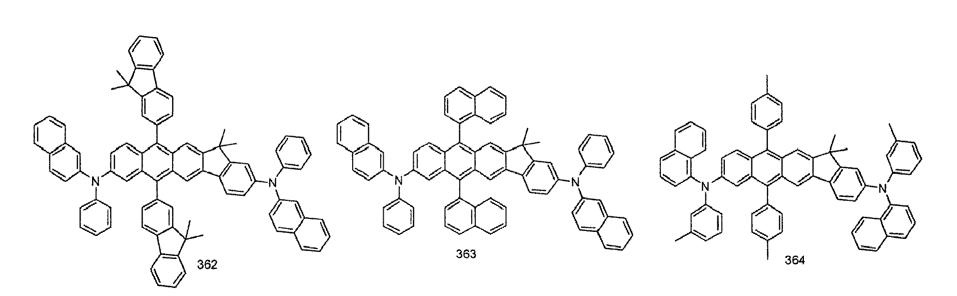

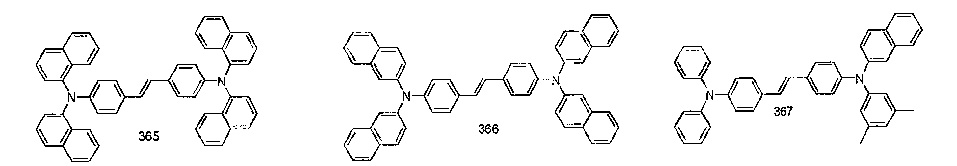

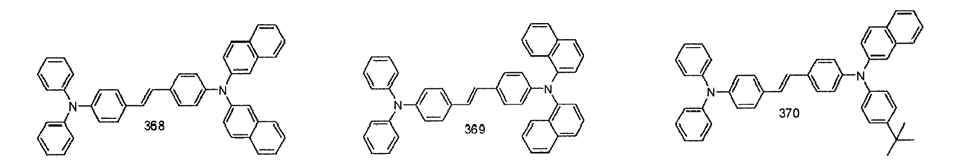

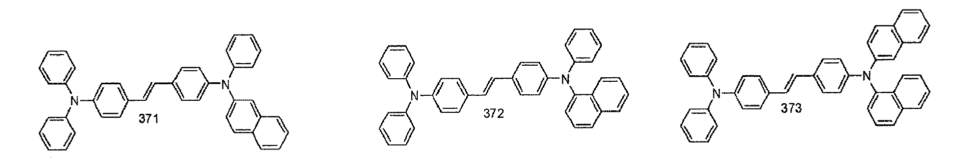

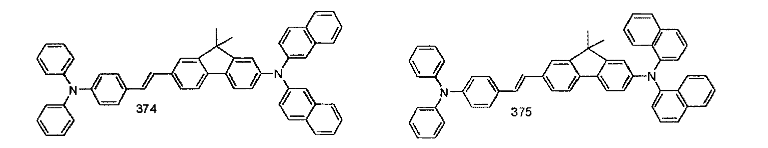

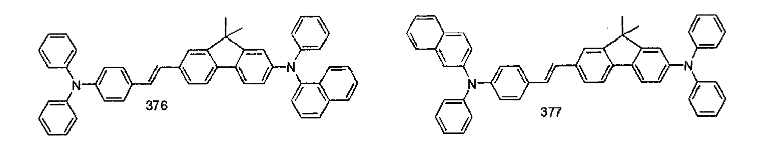

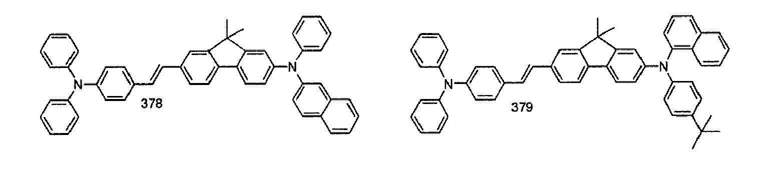

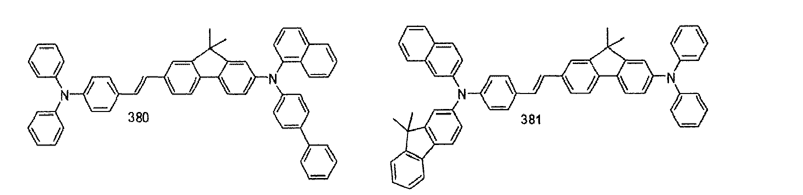

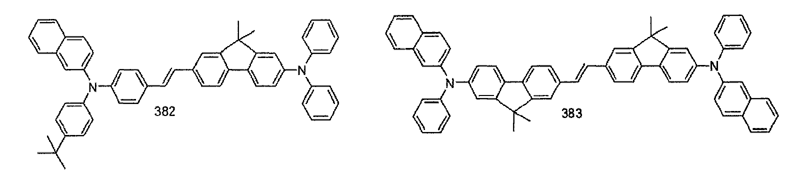

The host compound represented by Chemical Formula 2 may be exemplified by the compounds represented by Chemical Formulas 3 to 5: 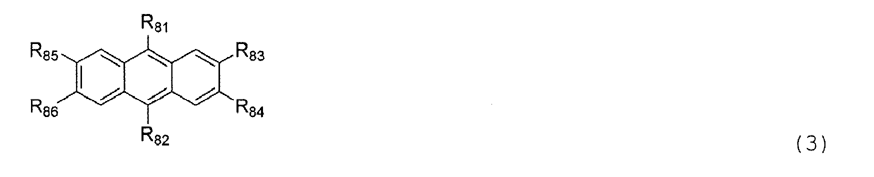 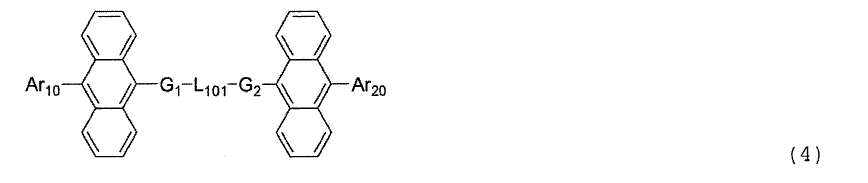 wherein
R₈₁ and R₈₂ independently represent (C6-C60)aryl, (C4-C60)heteroaryl, 5- or 6-membered heterocycloalkyl containing one or more heteroatom(s) selected from N, O and S or (C3-C60) cycloalkyl, and the aryl or heteroaryl of R₈₁ and R₈₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₈₃ through R₈₆ independently represent hydrogen, (C1-C60)alkyl, (C1-C60)alkoxy, halogen, (C4-C60)heteroaryl, (C5-C60)cycloalkyl or (C6-C60)aryl, and the heteroaryl, cycloalkyl or aryl of R₈₃ through R₈₆ may be further substituted by one or more substituent (s) selected from a group consisting of (C1-C60)alkyl with or without halogen substituent, (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond or (C6-C60)arylene with or without one or more substituent(s) selected from (C1-C60)alkyl, (C1-C60)alkoxy, (C6-C60)aryl, (C4-C60)heteroaryl and halogen;
Ar₁₀ and Ar₂₀ independently represent (C4-C60) heteroaryl or aryl selected from the following structures: 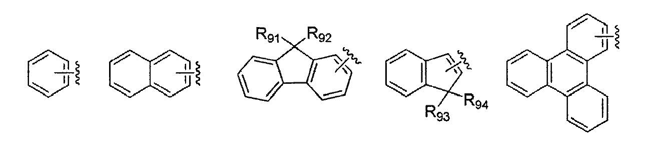 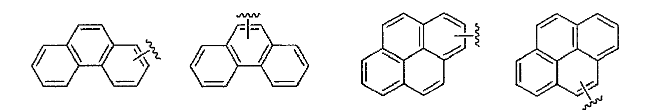 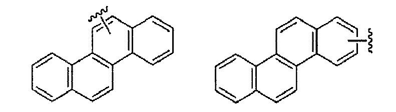 the aryl or heteroaryl of Ar₁₀ and Ar₂₀ may be substituted by one or more substituent(s) selected from (C1-C60)alkyl, (C1-C60)alkoxy, (C6-C60)aryl and (C4-C60)heteroaryl;
L₁₀₁ represents (C6-C60)arylene, (C4-C60)heteroarylene or a compound of the following formula: 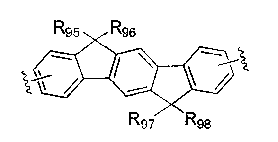 the arylene or heteroarylene of L₁₀₁ may be substituted by one or more substituent(s) selected from (C1-C60)alkyl, (C1-C60)alkoxy, (C6-C60)aryl, (C4-C60)heteroaryl and halogen;
R₉₁ through R₉₄ independently represent hydrogen, (C1-C60)alkyl or (C6-C60) aryl, or each of themmay be linked to an adj acent substituent via (C3-C60)alkylene or (C3-C60)alkenylene with or without a fused ring to form an alicyclic ring or a mono- or polycyclic ring;
R₉₅ through R₉₈ independently represent hydrogen, (C1-C60) alkyl, (C1-C60)alkoxy, (C6-C60) aryl, (C4-C60) heteroaryl or halogen, or each of themmay be linked to an adjacent substituent via (C3-C60) alkylene or (C3-C60) alkenylene with or without a fused ring to form an alicyclic ring or a mono- or polycyclic ring; 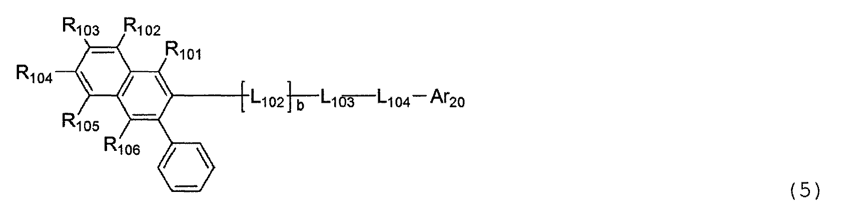 wherein
L₁₀₂ represents anthracenylene;
L₁₀₃ and L₁₀₄ independently represent a chemical bond, (C1-C60)alkyleneoxy, (C1-C60)alkylenethio, (C6-C60)aryleneoxy, (C6-C60)arylenethio, (C6-C60)arylene or (C3-C60)heteroarylene containing one or more heteroatom(s) selected from N, O and S;
Ar₂₀ represents (C6-C60)aryl or (C5-C60)heteroaryl with or without one or more substituent (s) selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
R₁₀₁ through R₁₀₆ independently represent hydrogen, deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl; and
b represents an integer from 1 to 4.

Specifically, the host compound represented by any of Chemical Formulas 3 to 5 may be exemplified by the following compounds, but not limited thereto:

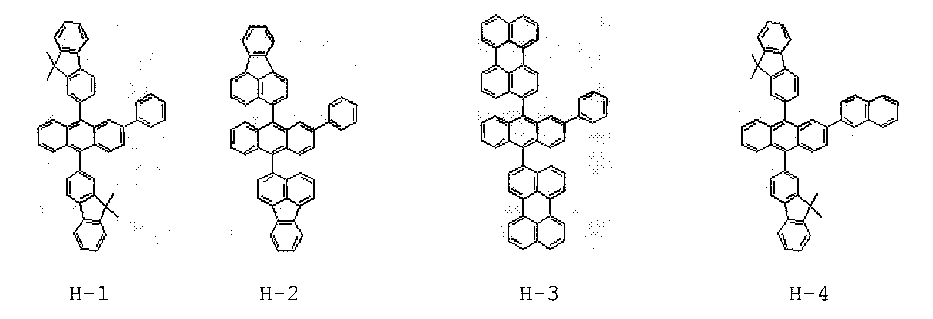

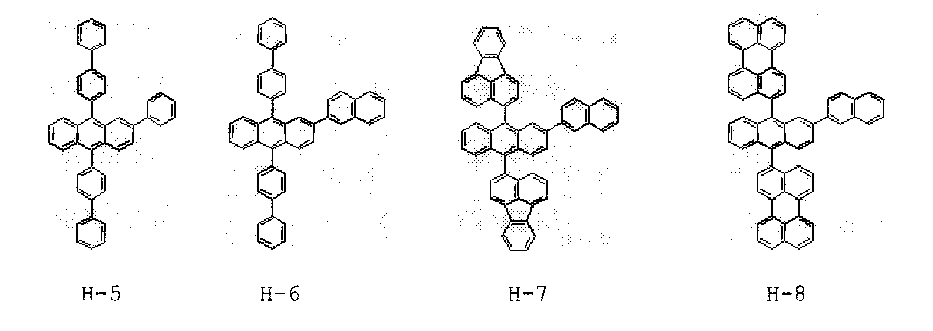

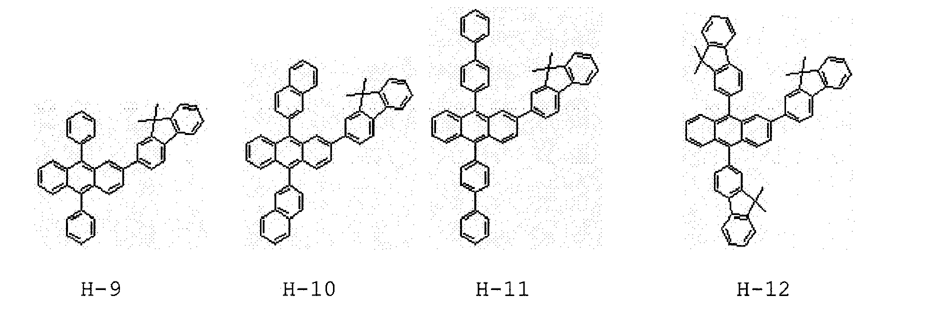

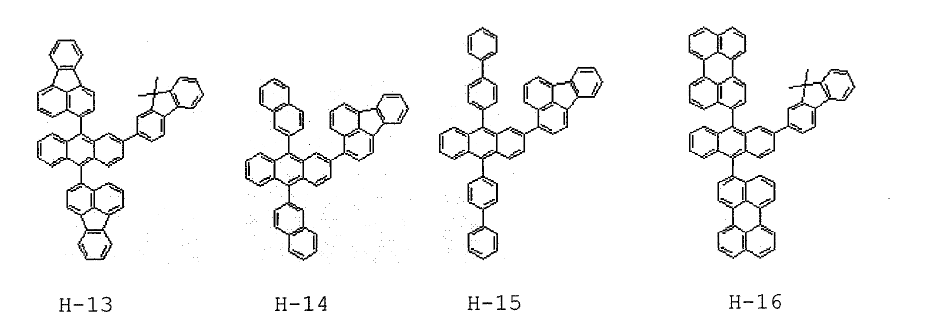

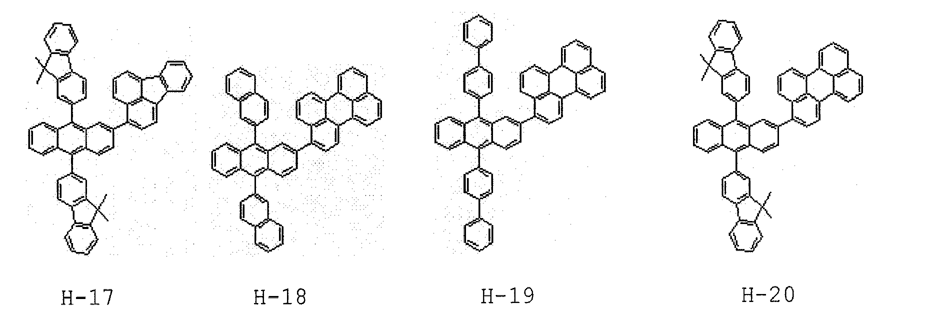

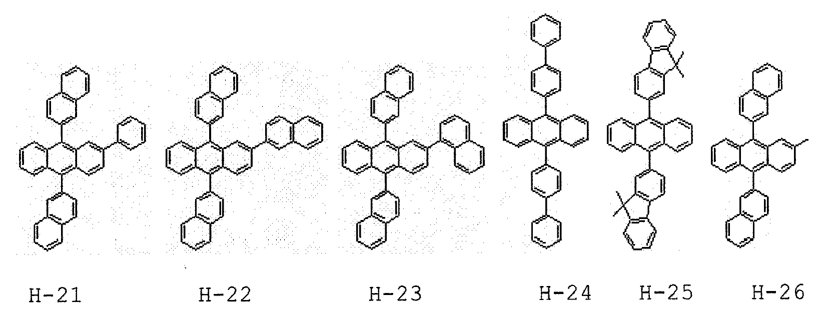

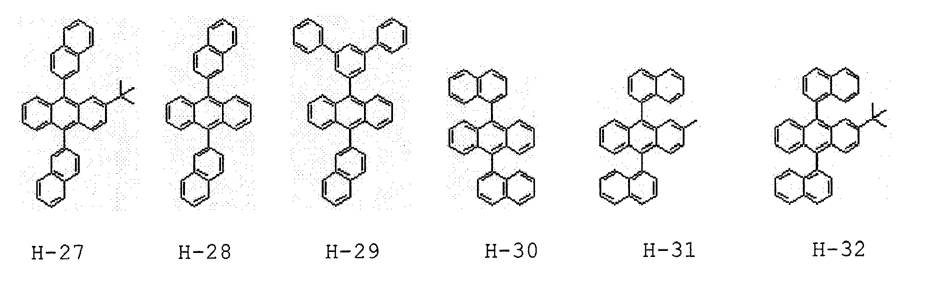

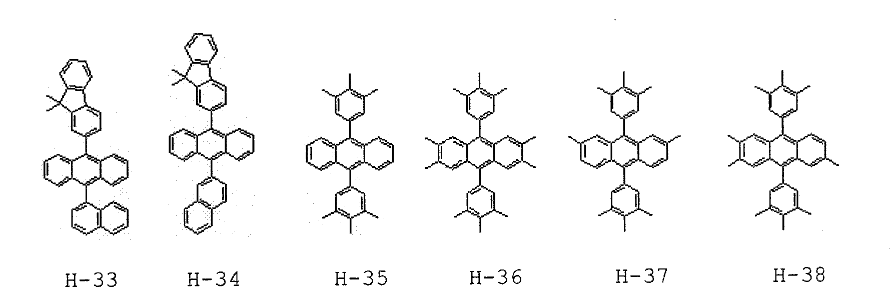

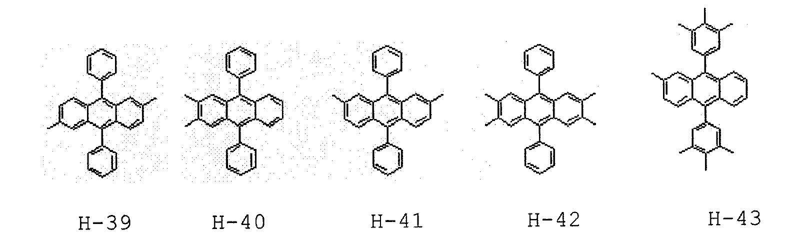

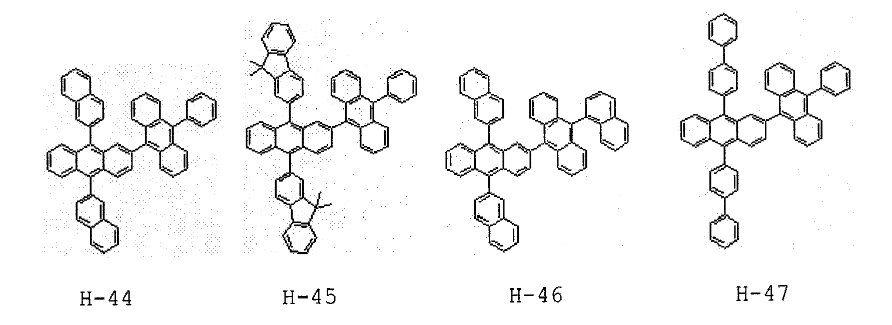

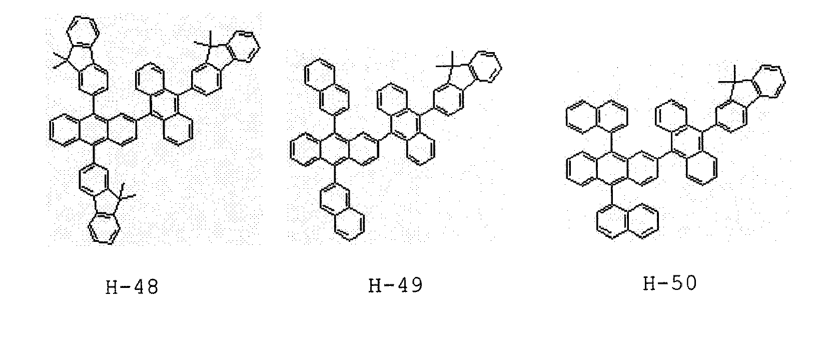

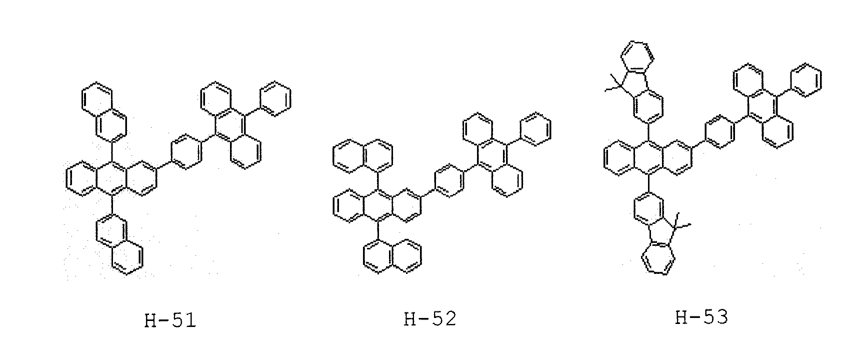

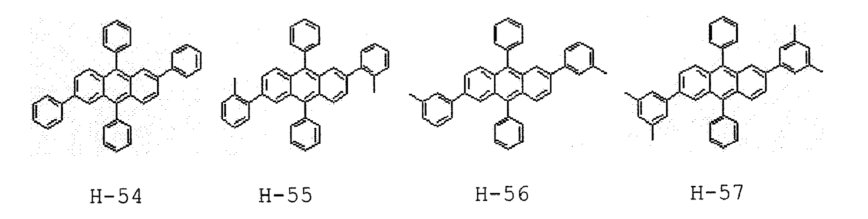

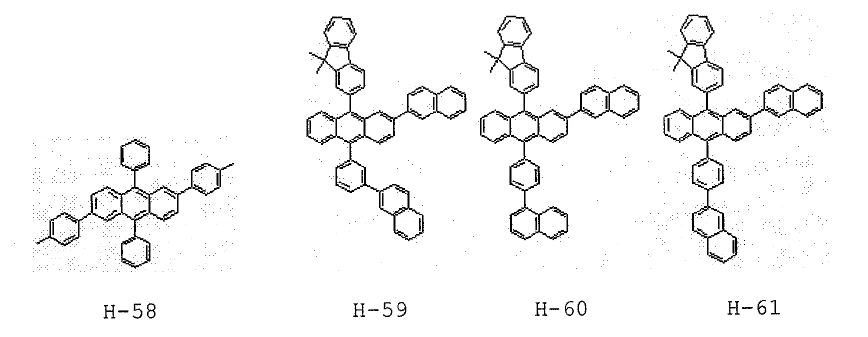

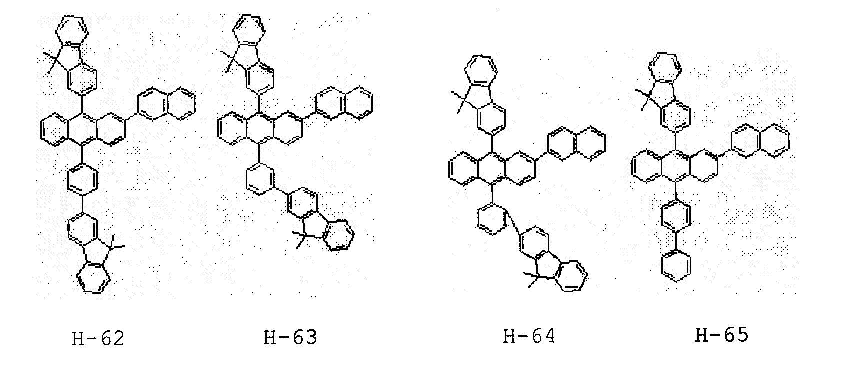

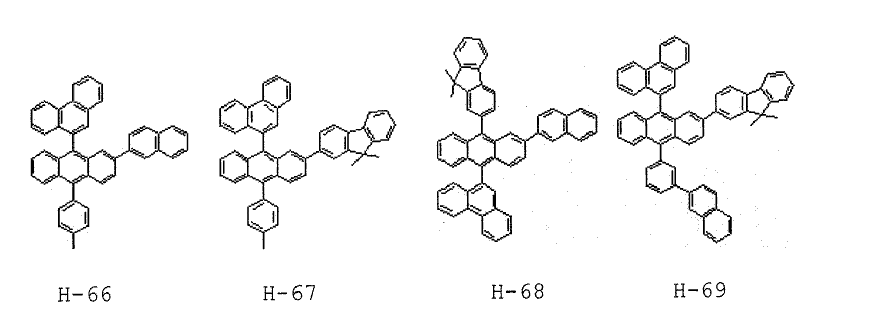

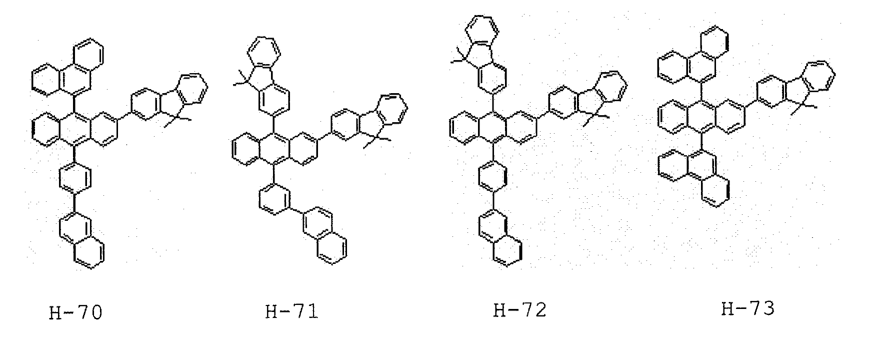

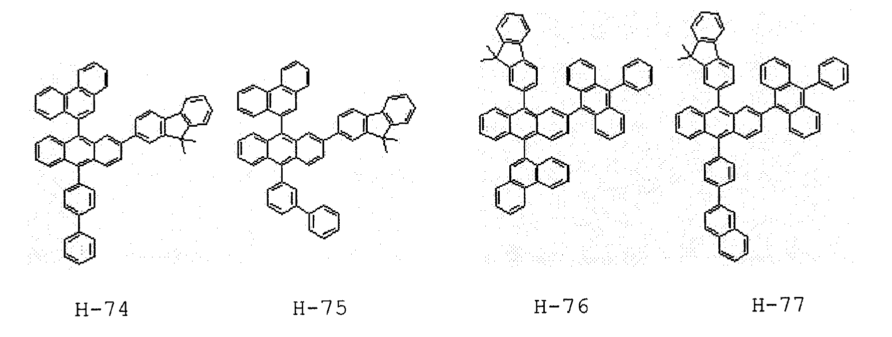

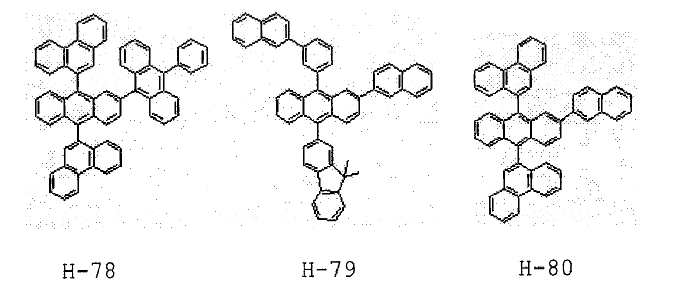

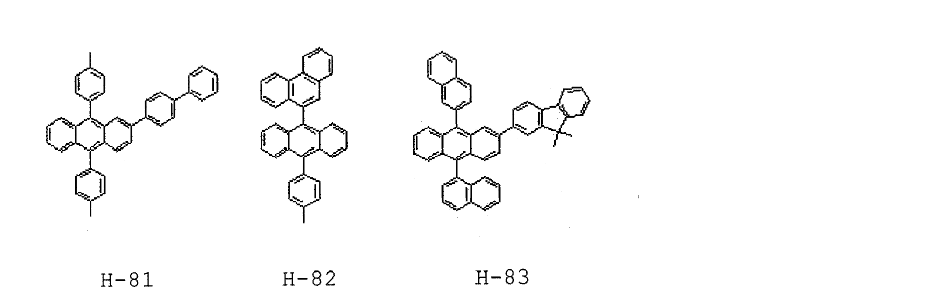

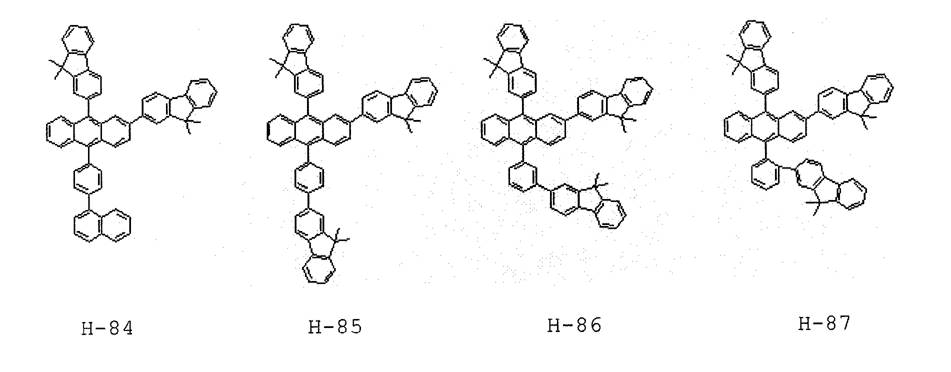

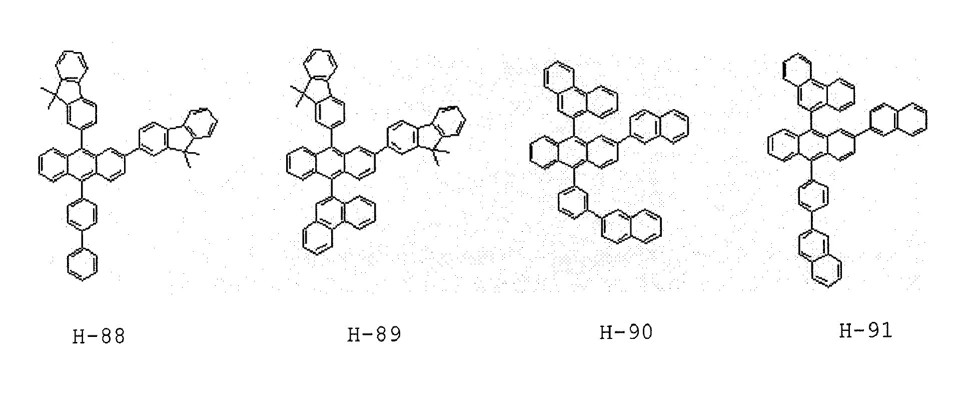

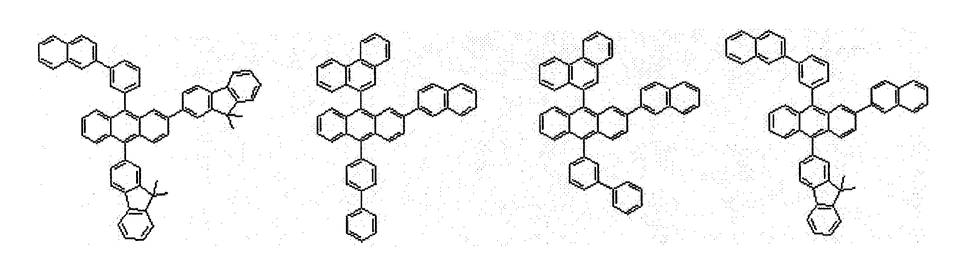

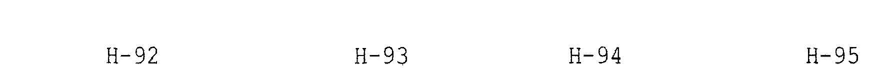

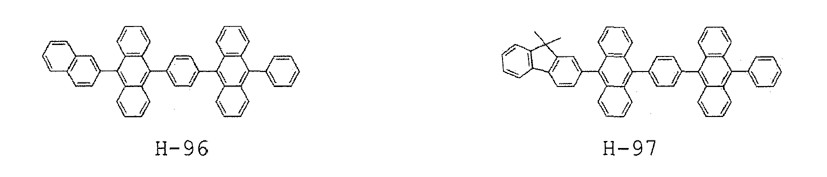

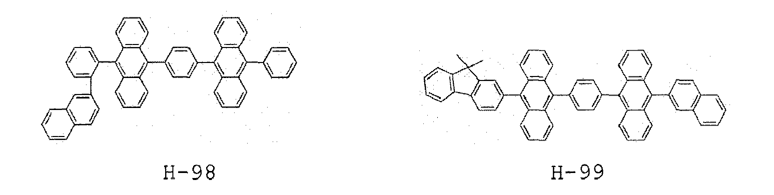

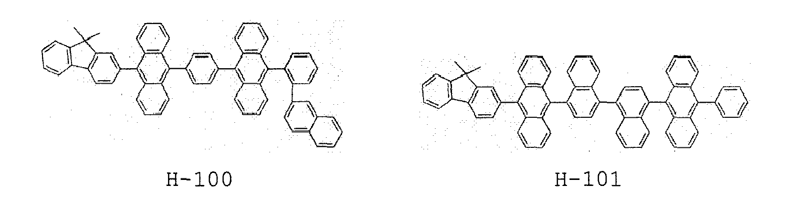

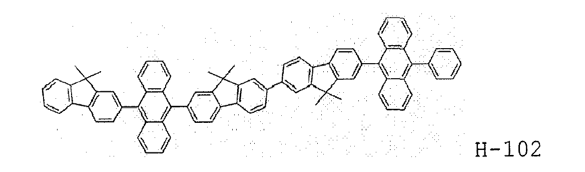

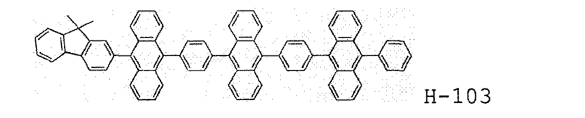

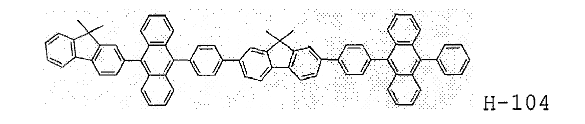

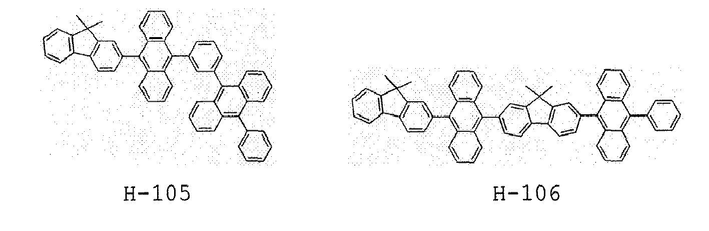

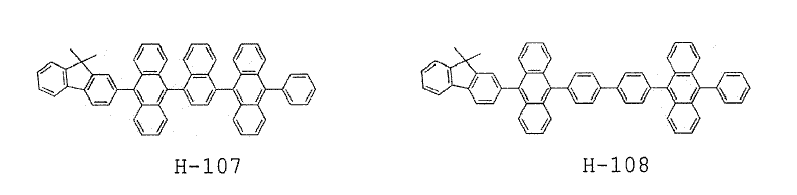

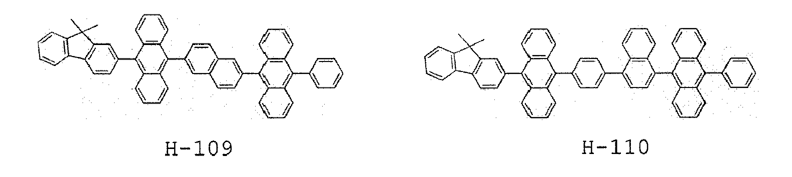

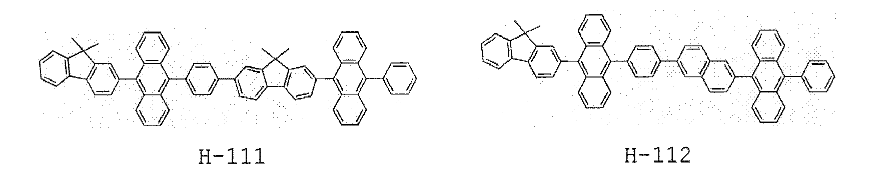

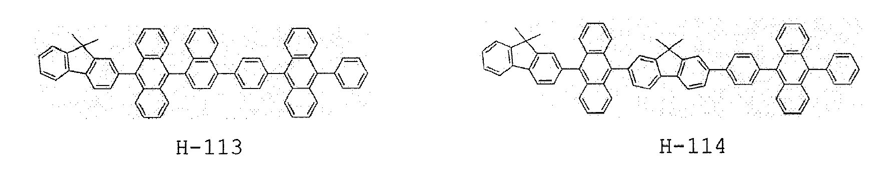

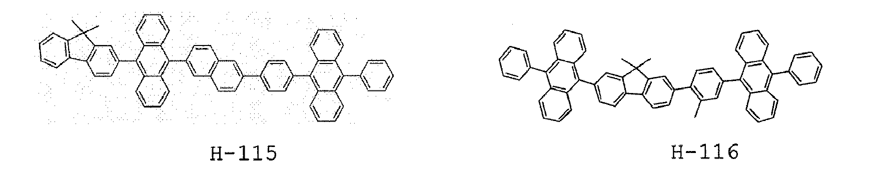

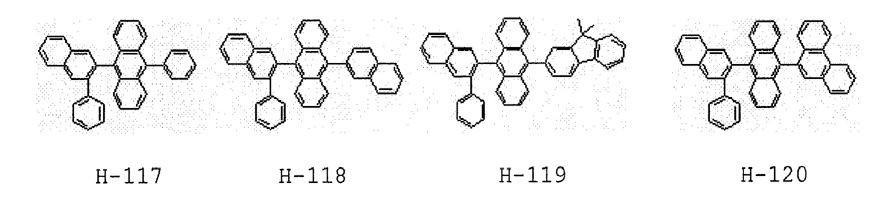

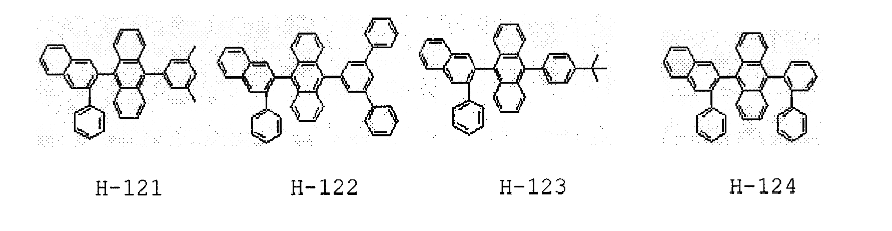

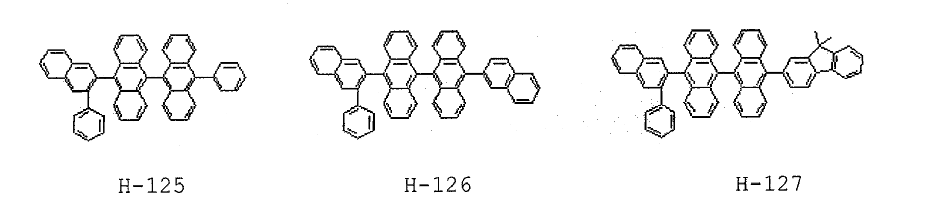

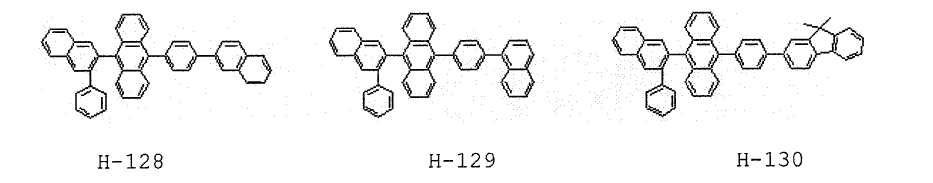

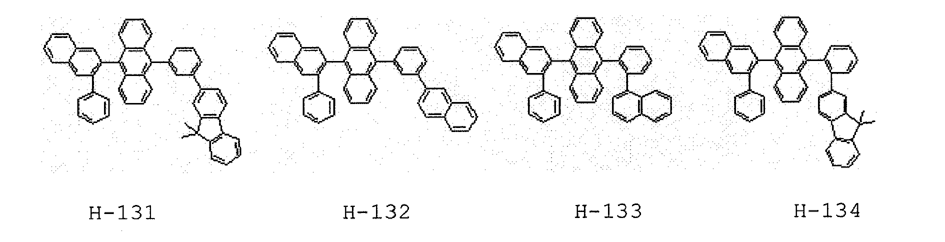

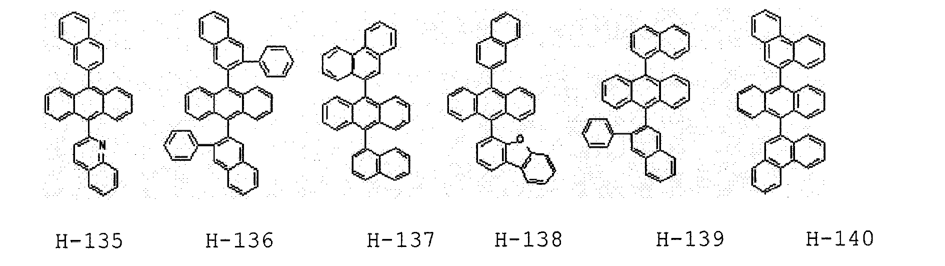

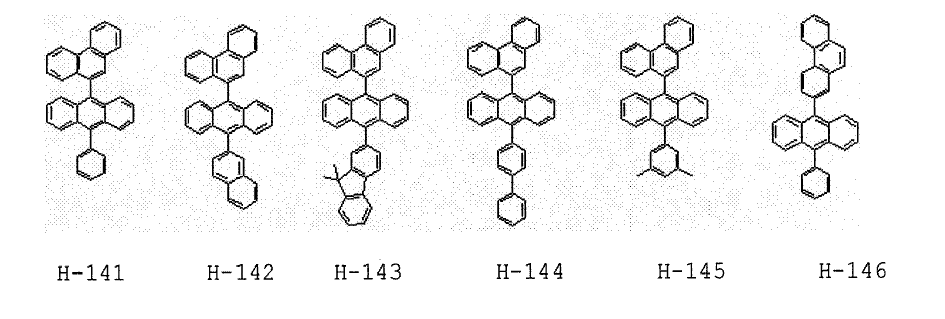

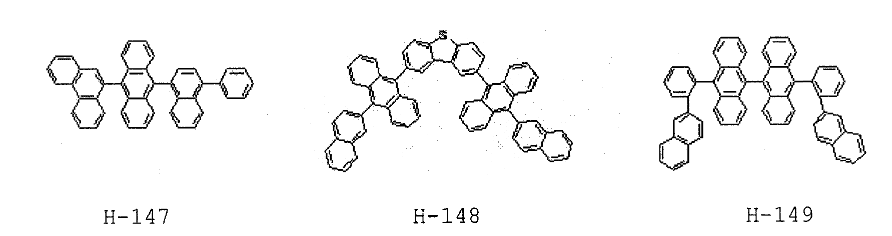

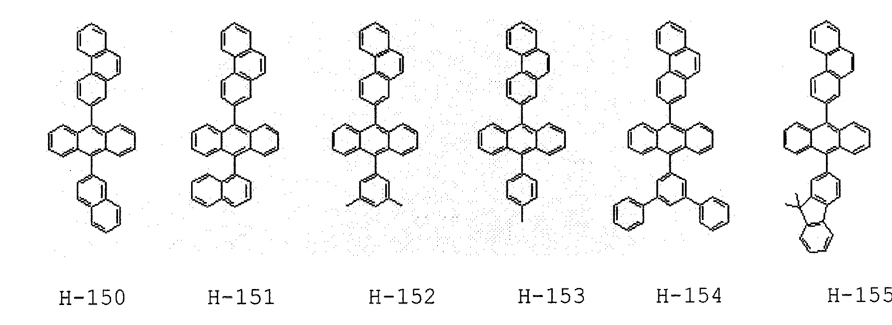

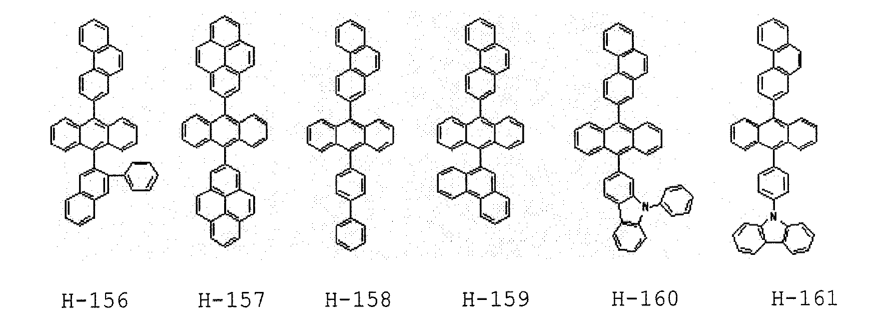

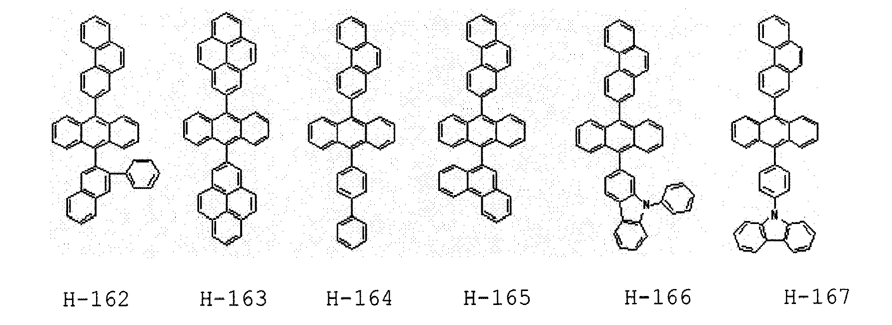

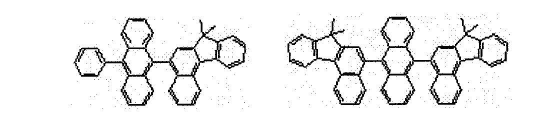

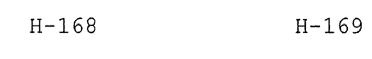

The electroluminescent layer means a layer where electroluminescence occurs. It may be either a single layer or may comprise two or more layers. In case a dopant and a host are used together in accordance with the present invention, remarkable improvement in luminous efficiency may be attained.

The electroluminescent device according to the present invention comprises the electroluminescent compound represented by Chemical Formula 1 and may further comprise one or more compound(s) selected from a group consisting of arylamine compounds and styrylarylamine compounds at the same time. For example, the arylamine compound or the styrylarylamine compound may be a compound represented by Chemical Formula 6, but not limited thereto: 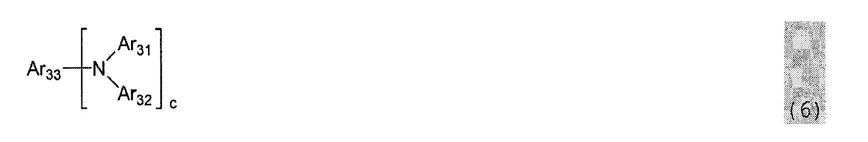 wherein
Ar₃₁ and Ar₃₂ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S or (C3-C60) cycloalkyl, and Ar₃₁ and Ar₃₂ may be linked via (C3-C60) alkylene or (C3-C60) alkenylene with or without a fused ring to form an alicyclic ring or a mono- or polycyclic ring;
Ar₃₃ represents (C6-C60) aryl, (C4-C60)heteroaryl or a substituent selected from the following structures, when c is 1: 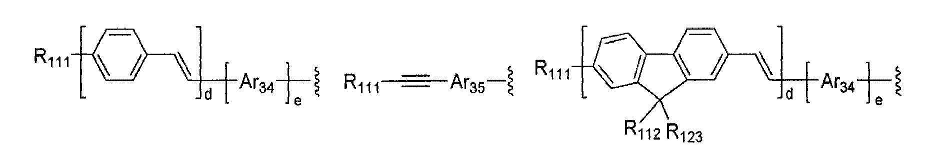 Ar₃₃ represents (C6-C60)arylene, (C4-C60)heteroarylene or a substituent selected from the following structures, when c is 2: 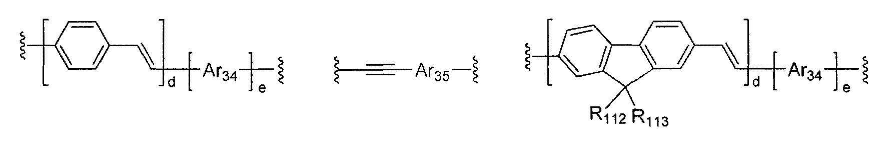 Ar₃₄ and Ar₃₅ independently represent (C6-C60)arylene or (C4-C60)heteroarylene;
R₁₁₁ through R₁₁₃ independently represent hydrogen, deuterium, (C1-C60)alkyl or (C6-C60)aryl;
d represents an integer from 1 to 4;
e represents an integer 0 or 1; and
the alkyl, aryl, heteroaryl, arylamino, alkylamino, cycloalkyl or heterocycloalkyl of Ar₃₁ and Ar₃₂, the aryl, heteroaryl, arylene or heteroarylene of Ar₃₃, the arylene or heteroarylene of Ar₃₄ and Ar₃₅, or the alkyl or aryl of R₁₁₁ through R₁₁₃ may be further substituted by one or more substituent(s) selected from a group consisting of deuterium, halogen, (C1-C60)alkyl, (C6-C60)aryl, (C4-C60)heteroaryl, 5- or 6-membered heterocycloalkyl containing one ormore heteroatom(s) selected from N, O and S, (C3-C60) cycloalkyl, tri (C1-C60) alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

More specifically, the arylamine compound or the styrylarylamine compound may be exemplified by the following compounds, but not limited thereto:

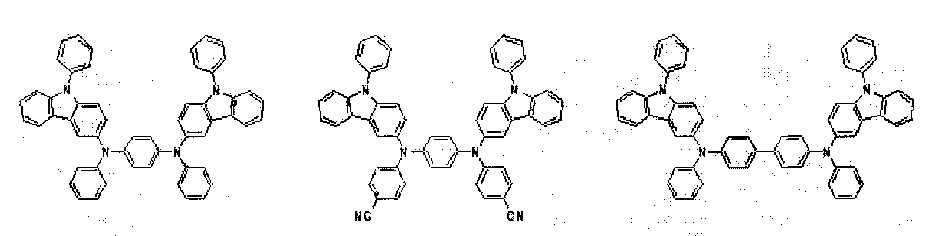

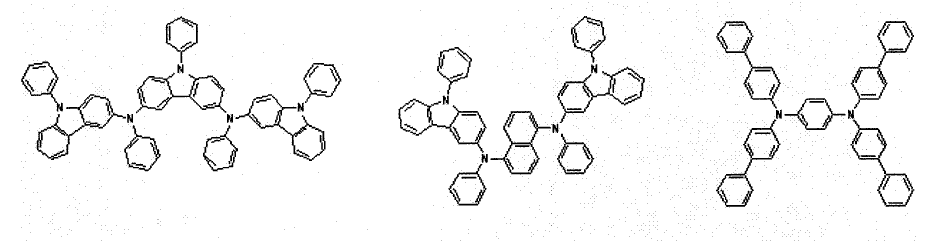

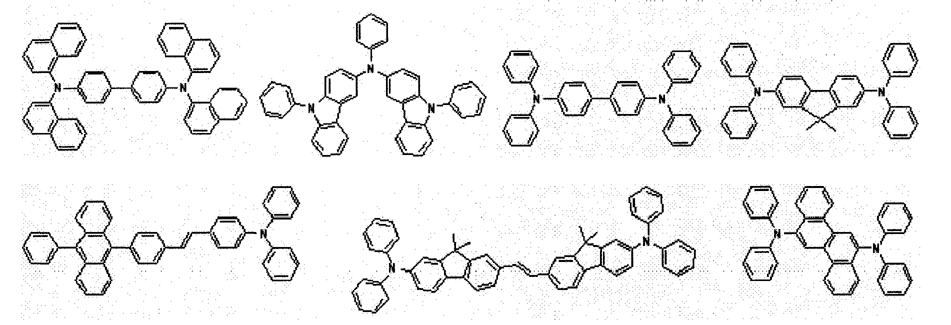

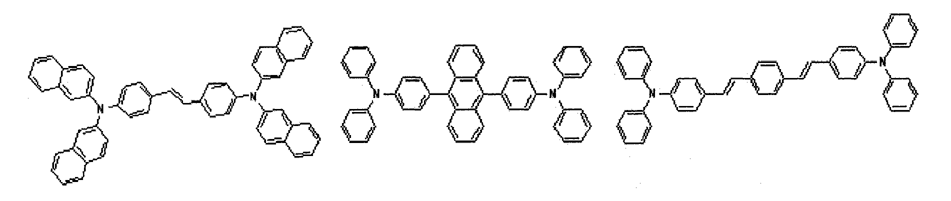

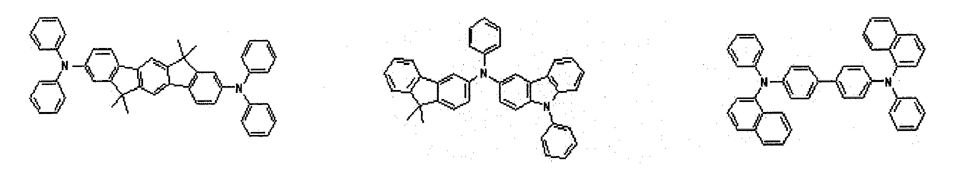

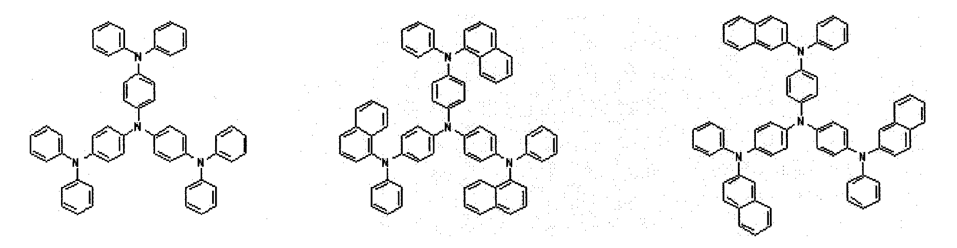

In the electroluminescent device according to the present invention, the organic layer may further comprise, in addition to the electroluminescent compound represented by Chemical Formula 1, one or more metal (s) selected from a group consisting of organic metals of Group 1, Group 2, 4th period and 5th period transition metals, lanthanide metals and d-transition elements. Also, the organic layer may comprise an electroluminescent layer and a charge generating layer at the same time.

The organic layer may further comprise, in addition to the electroluminescent compound represented by Chemical Formula 1 or 2, one or more compound(s) selected from a compound having an electroluminescence peak with red, green or blue wavelength to form a white-emitting electroluminescent device. The compound having an electroluminescence peak with red, green or blue wavelength may be exemplified by the compounds represented by Chemical Formulas 7 to 11, but not limited thereto:

**M¹L¹L²L³** (7)

wherein
M¹ represents a metal selected from a group consisting of Group 7, Group 8, Group 9, Group 10, Group 11, Group 13, Group 14, Group 15 and Group 16 metals;
ligands L¹⁰¹, L¹⁰² and L¹⁰³ are independently selected from the following structures: 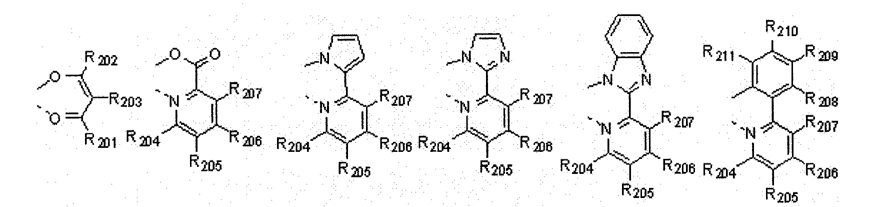 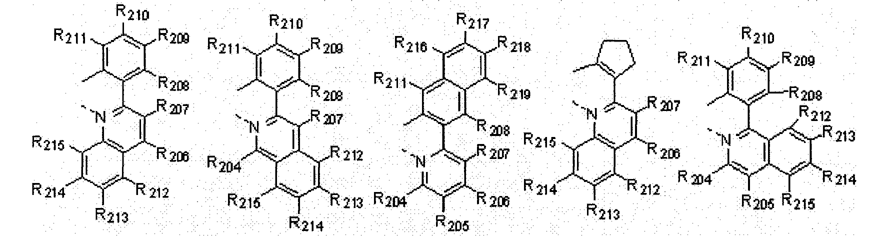 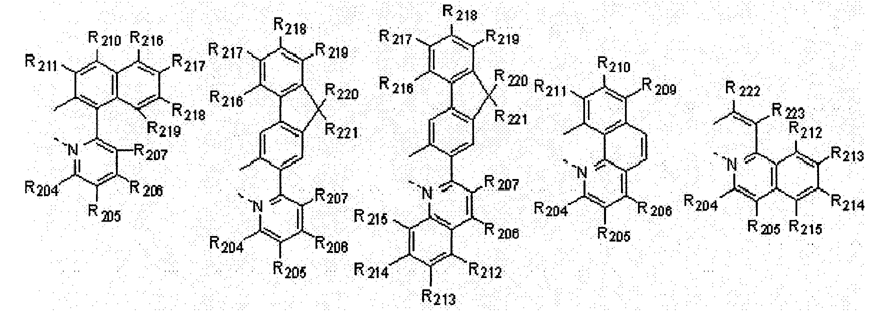 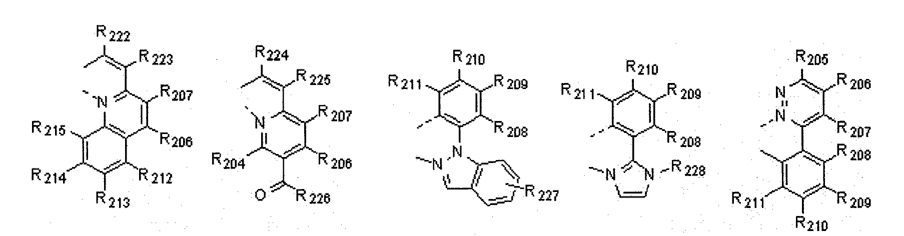 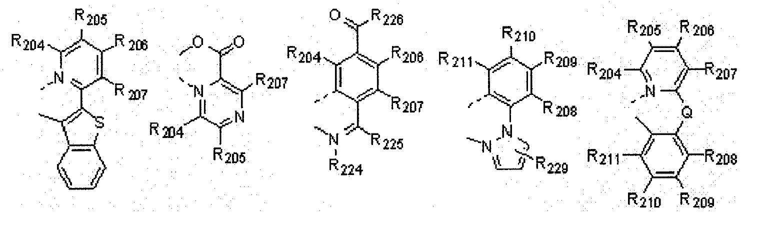 wherein
R₂₀₁ through R₂₀₃ independently represent hydrogen, deuterium, (C1-C60)alkyl with or without halogen substituent, (C6-C60) aryl with or without (C1-C60)alkyl substituent or halogen;
R₂₀₄ through R₂₁₉ independently represent hydrogen, deuterium, (C1-C60)alkyl, (C1-C30)alkoxy, (C3-C60)cycloalkyl, (C2-C30)alkenyl, (C6-C60)aryl, mono- or di(C1-C30)alkylamino, mono- or di(C6-C30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₂₀₄ through R₂₁₉ may be further substituted by one or more substituent (s) selected from deuterium, (C1-C60)alkyl, (C6-C60)aryl and halogen;
R₂₂₀ through R₂₂₃ independently represent hydrogen, deuterium, (C1-C60)alkyl with or without halogen substituent or (C6-C60)aryl with or without (C1-C60)alkyl substituent;
R₂₂₄ and R₂₂₅ independently represent hydrogen, deuterium, (C1-C60)alkyl, (C6-C60) aryl or halogen, or R₂₂₄ and R₂₂₅ may be linked via (C3-C12)alkylene or (C3-C12)alkenylene with or without a fused ring to form an alicyclic ring or a mono- or polycyclic ring, and the alkyl or aryl of R₂₂₄ and R₂₂₅, or the alicyclic ring or the mono- or polycyclic ring formed as they are linked via (C3-C12)alkylene or (C3-C12)alkenylene with or without a fused ring may be further substituted by one or more substituent(s) selected from deuterium, (C1-C60)alkyl with or without halogen substituent, (C1-C30)alkoxy, halogen, tri(C1-C30)alkylsilyl, tri(C6-C30)arylsilyl and (C6-C60)aryl;
R₂₂₆ represents (C1-C60)alkyl, (C6-C60)aryl, (C5-C60) heteroaryl containing one or more heteroatom (s) selected from N, O and S or halogen;
R₂₂₇ through R₂₂₉ independently represent hydrogen, deuterium, (C1-C60)alkyl, (C6-C60)aryl or halogen, and the alkyl or aryl of R₂₂₆ through R₂₂₉ may be further substituted by halogen or (C1-C60)alkyl;
Q represents 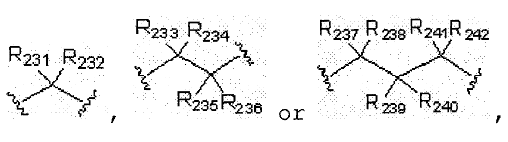 wherein R₂₃₁ through R₂₄₂ independently represent hydrogen, deuterium, (C1-C60)alkyl with or without halogen substituent, (C1-C30)alkoxy, halogen, (C6-C60)aryl, cyano or (C5-C60)cycloalkyl, or each of R₂₃₁ through R₂₄₂ may be linked to an adjacent substituent via alkylene or alkenylene to form a (C5-C7)spiro ring or a (C5-C9)fused ring, or may be linked to R₂₀₇ or R₂₀₈ via alkylene or alkenylene to form a (C5-C7)fused ring; 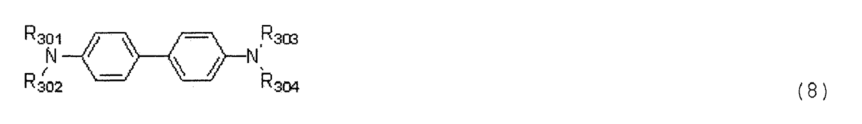 wherein
R₃₀₁ through R₃₀₄ independently represent (C1-C60)alkyl or (C6-C60) aryl, or each of them may be linked to an adjacent substituent via (C3-C60)alkylene or (C3-C60)alkenylene with or without a fused ring to form an alicyclic ring or a mono- or polycyclic ring;
the alkyl or aryl of R₃₀₁ through R₃₀₄, or the alicyclic ring or the mono- or polycyclic ring formed as they are linked via (C3-C60)alkylene or (C3-C60)alkenylene with or without a fused ring may be further substituted by one or more substituent(s) selected from (C1-C60)alkyl with or without halogen substituent, (C1-C60)alkoxy, halogen, tri(C1-C60)alkylsilyl, tri(C6-C60)arylsilyl and (C6-C60)aryl;

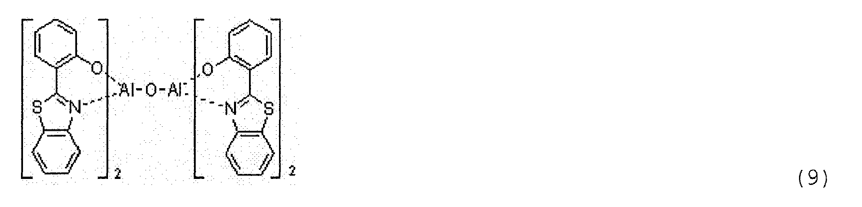

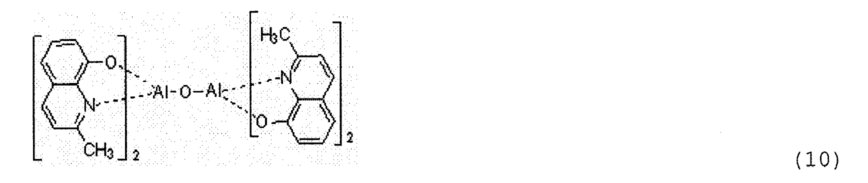

**L⁴L⁵M²(T)_{f}** (11)

wherein
ligands L⁴ and L⁵ are independently selected from the following structures: 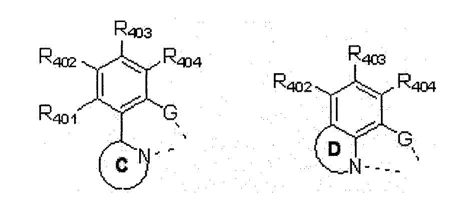 M² represents a divalent or trivalent metal;
f is 0 when M² is a divalent metal, and f is 1 when M² is a trivalent metal;
T represents (C6-C60)aryloxy or tri(C6-C60)arylsilyl, and the aryloxyor triarylsilyl of T may be further substituted by (C1-C60) alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and the ring D may be further substituted by (C1-C60)alkyl, phenyl with or without (C1-C60)alkyl substituent or naphthyl;
R₄₀₁ through R₄₀₄ independently represent hydrogen, deuterium, (C1-C60)alkyl, halogen, tri(C1-C60)alkylsilyl, tri(C6-C60)arylsilyl or (C6-C60)aryl, or each of themmay be linked to an adjacent substituent via (C3-C60)alkylene or (C3-C60)alkenylene to form a fused ring, and the pyridine or quinoline may be linked to R₅₀₁ via a chemical bond to form a fused ring; and
the aryl of ring C and R₄₀₁ through R₄₀₄ may be further substituted by (C1-C60)alkyl, halogen, (C1-C60)alkyl with halogen substituent, phenyl, naphthyl, tri(C1-C60)alkylsilyl, tri(C6-C60)arylsilyl or amino.

The compound having an electroluminescence peak with red, green or blue wavelength may be exemplified by the compounds represented by the following compounds, but not limited thereto:

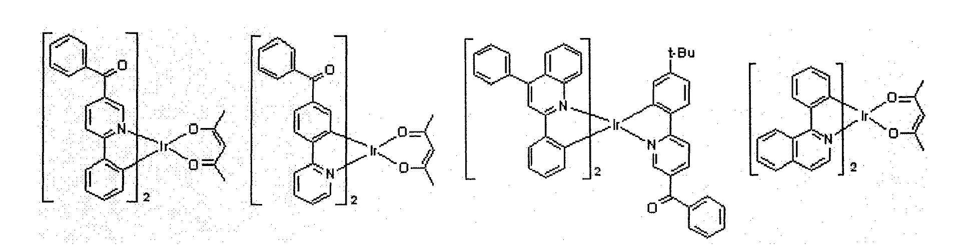

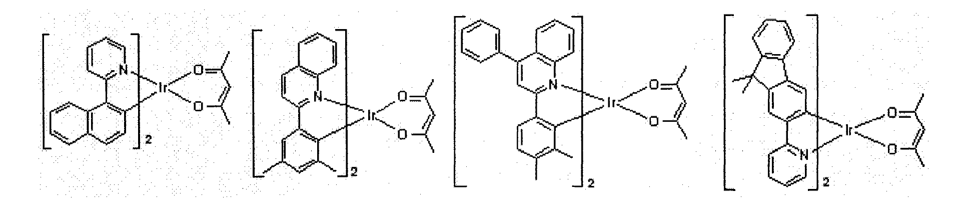

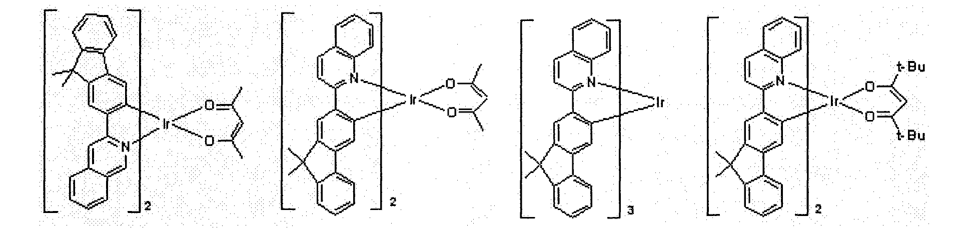

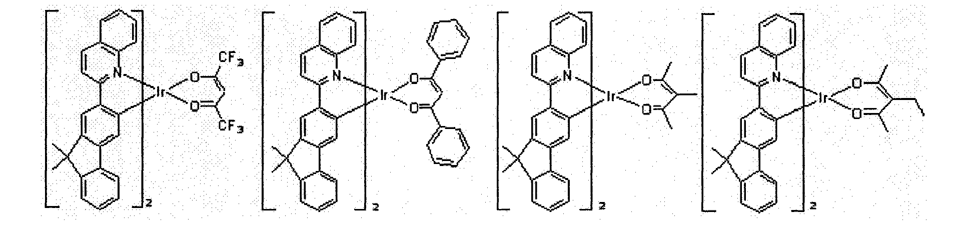

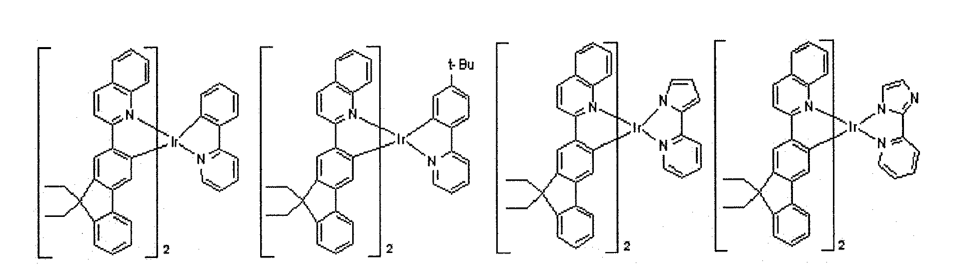

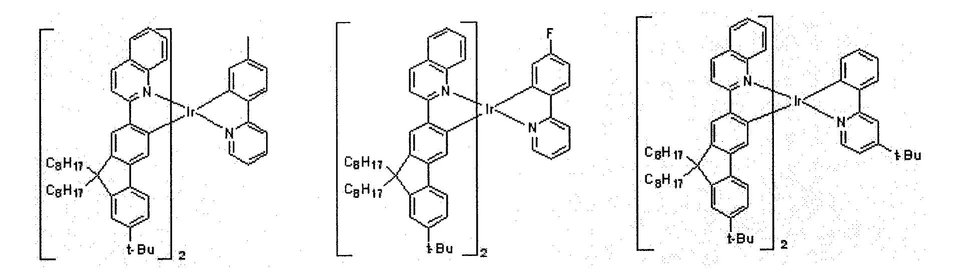

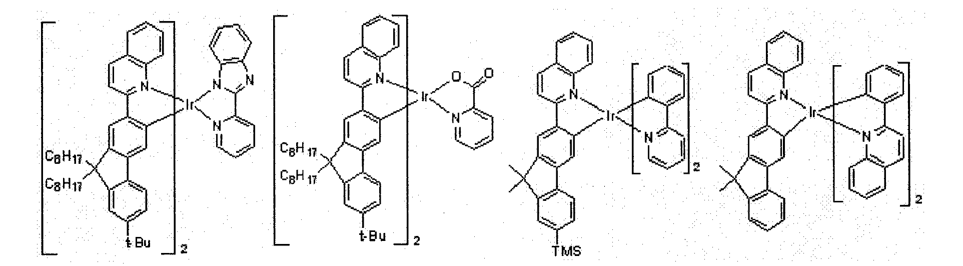

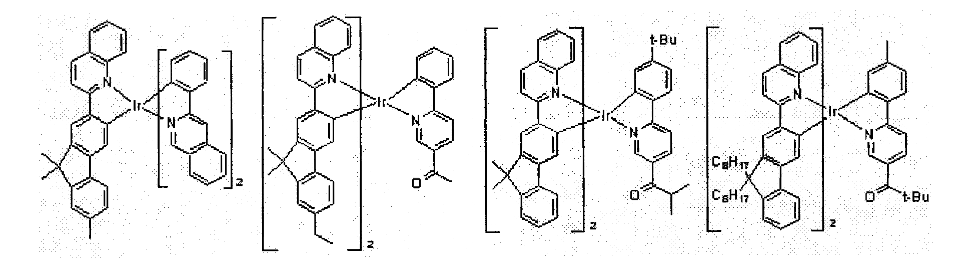

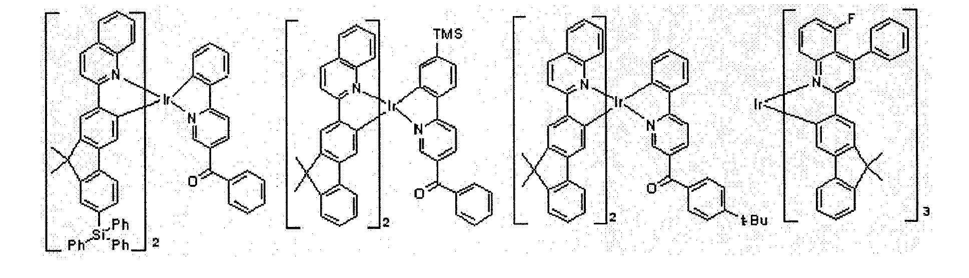

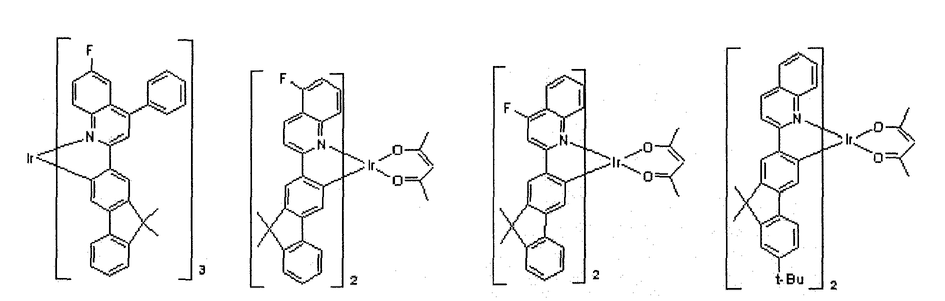

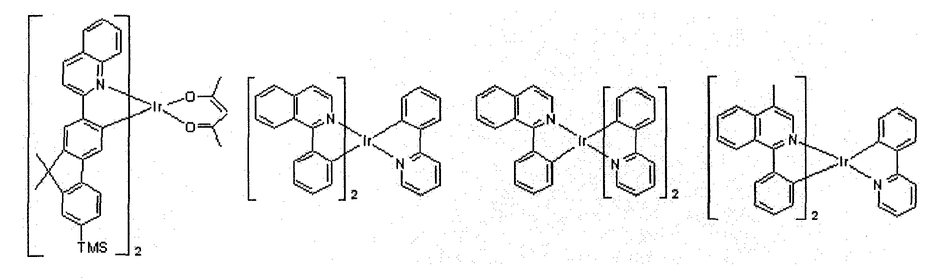

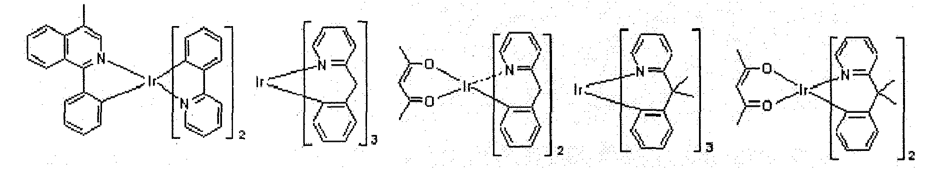

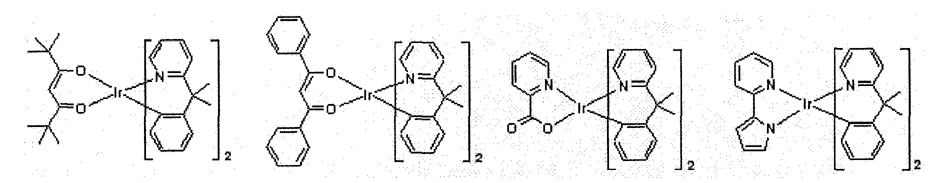

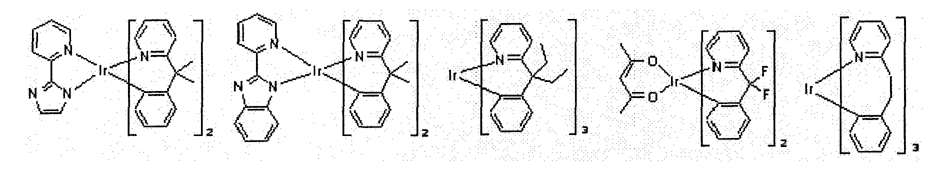

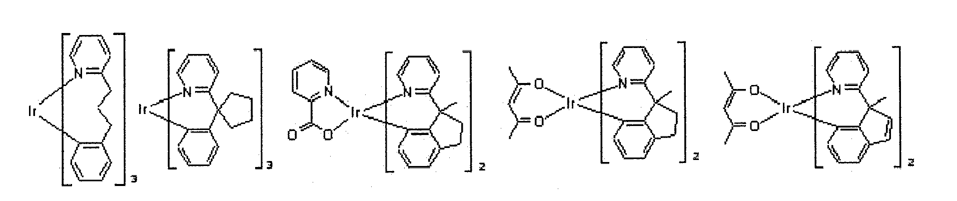

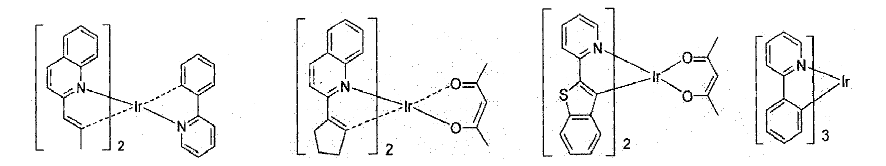

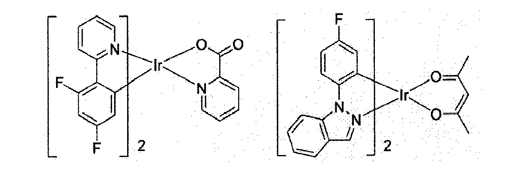

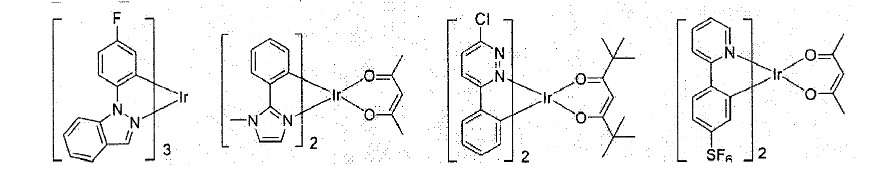

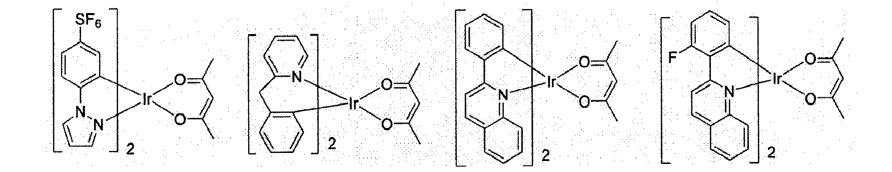

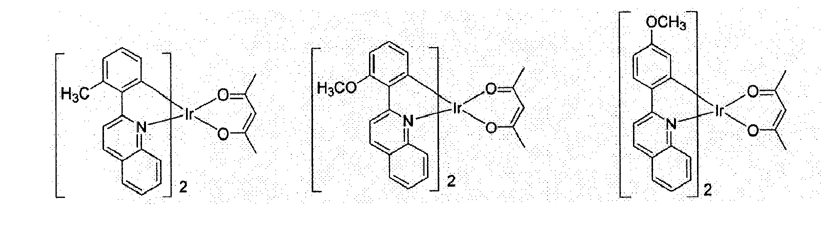

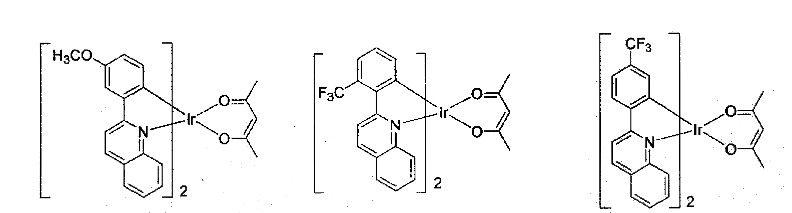

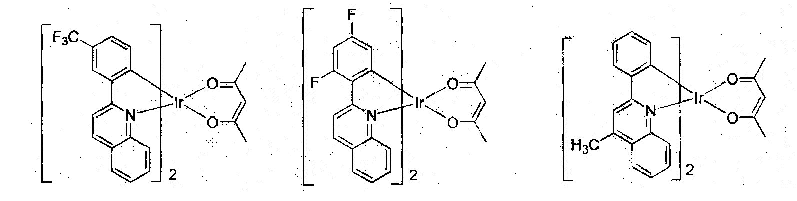

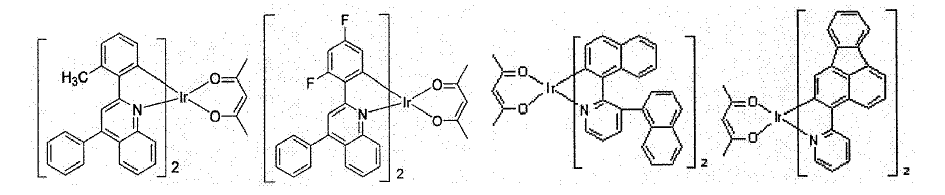

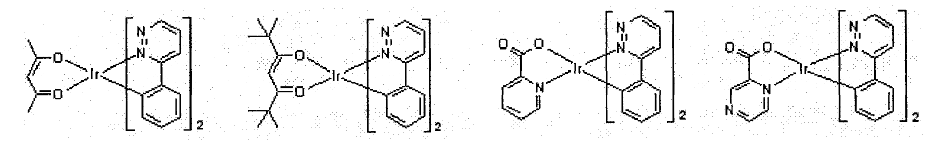

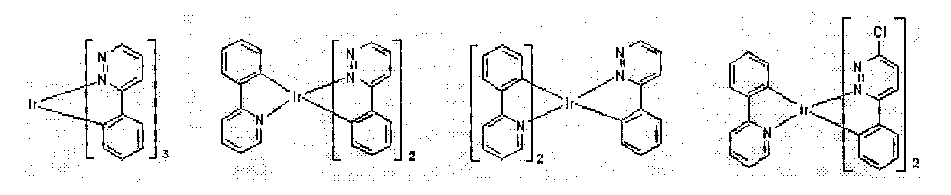

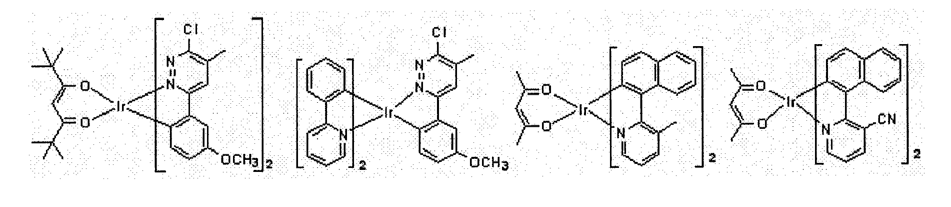

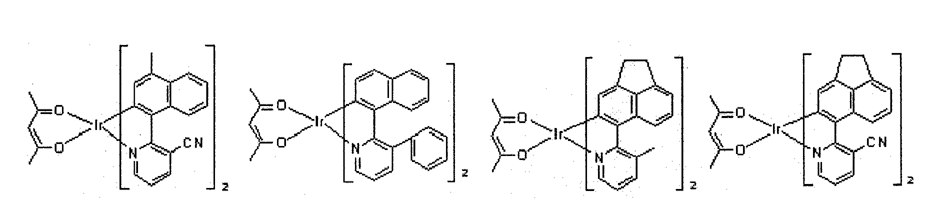

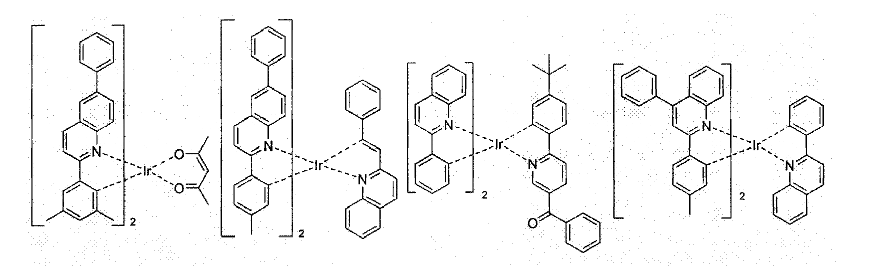

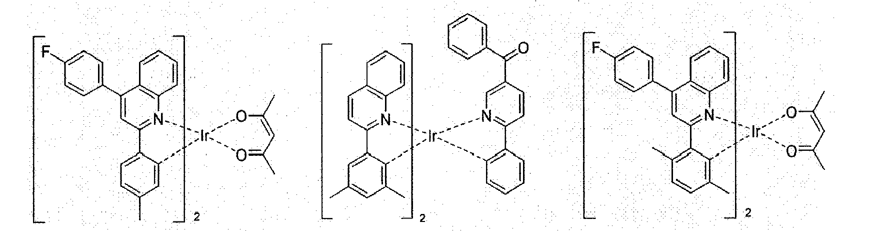

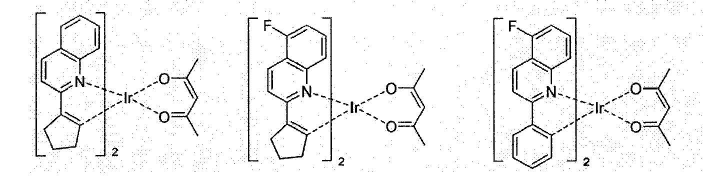

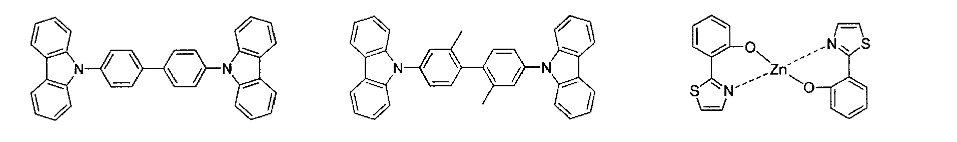

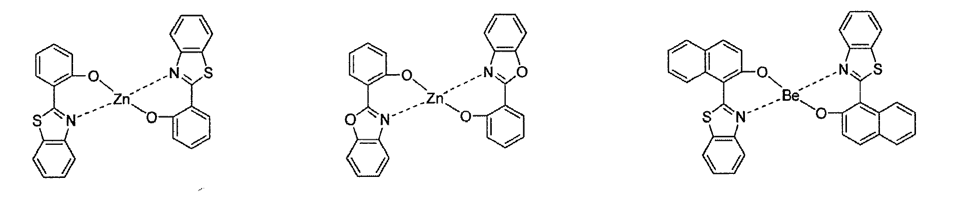

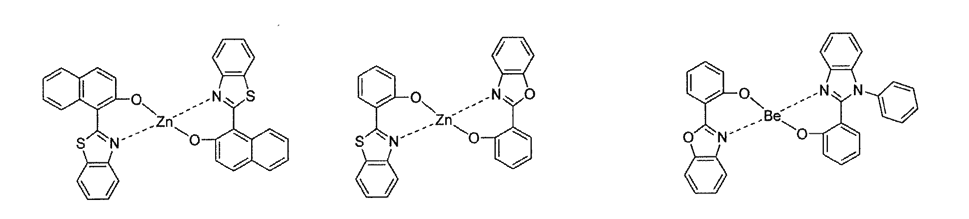

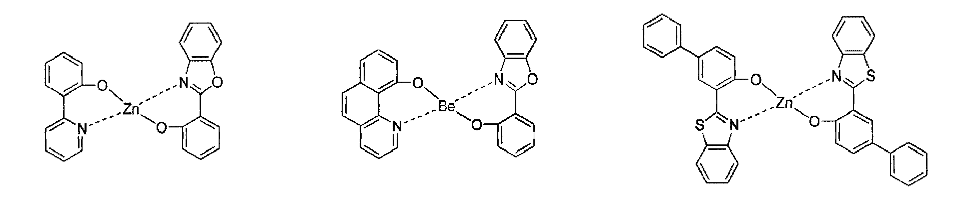

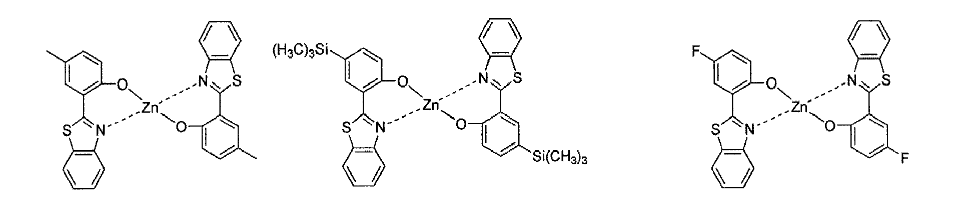

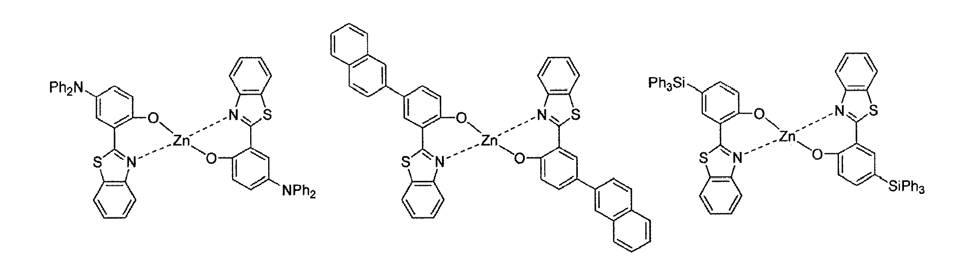

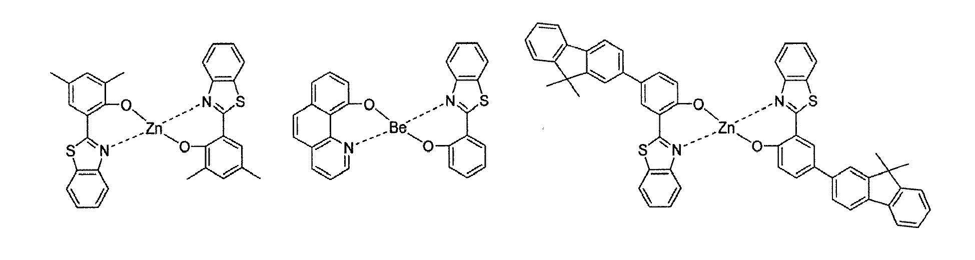

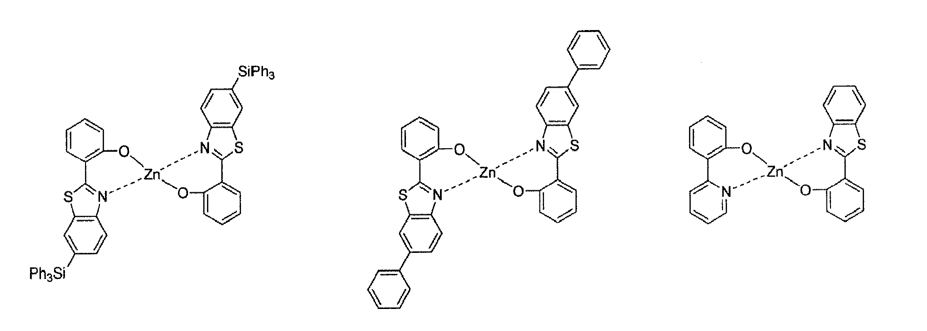

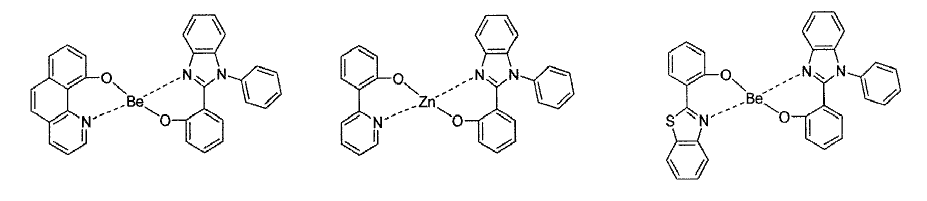

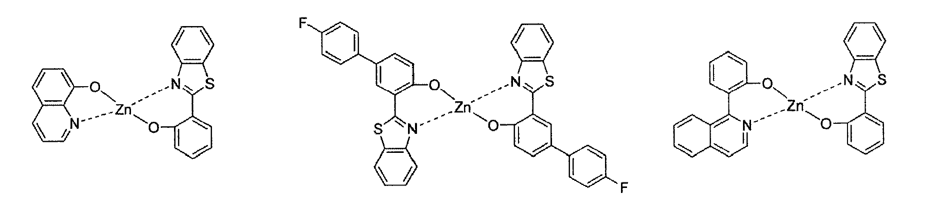

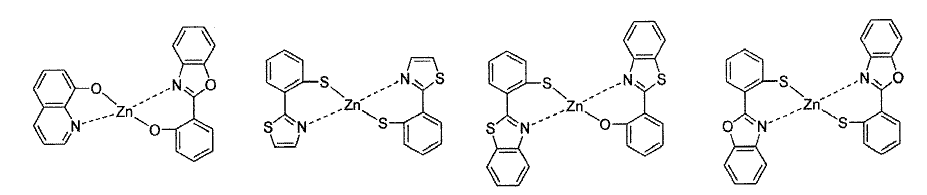

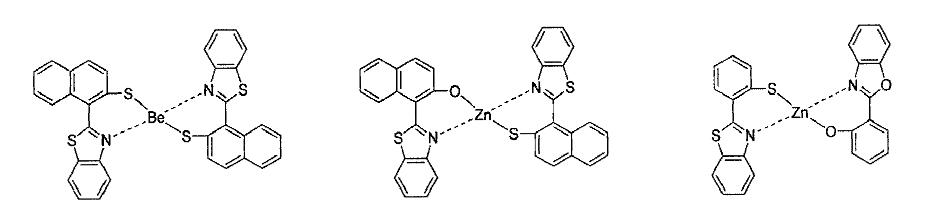

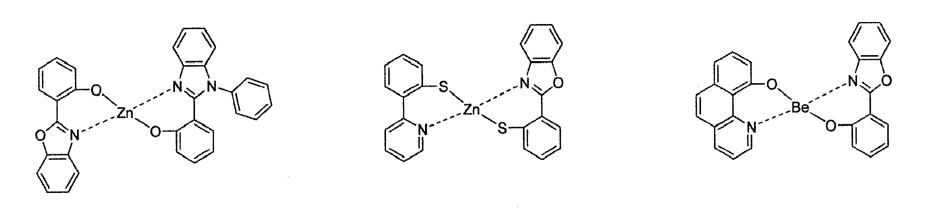

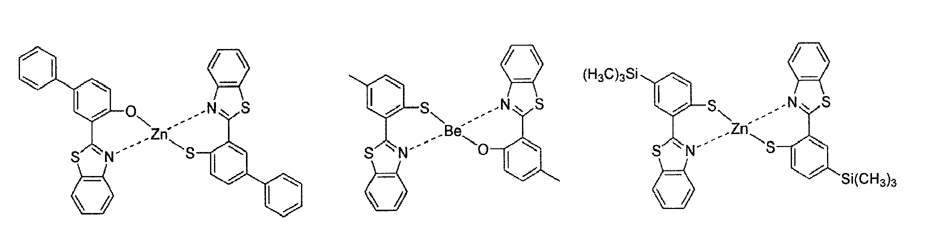

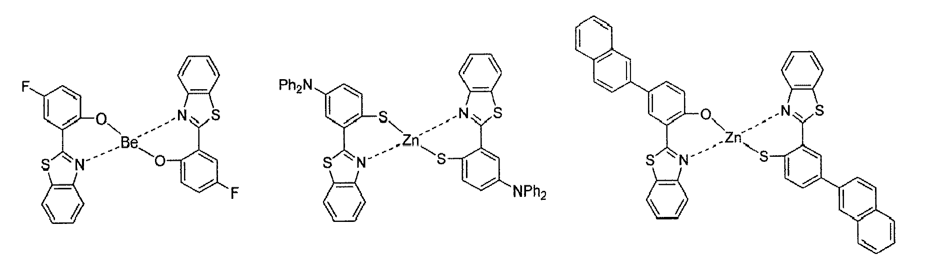

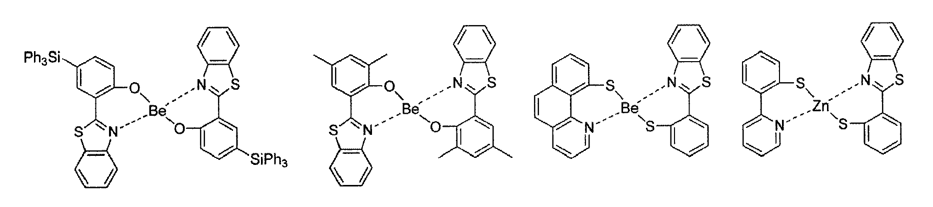

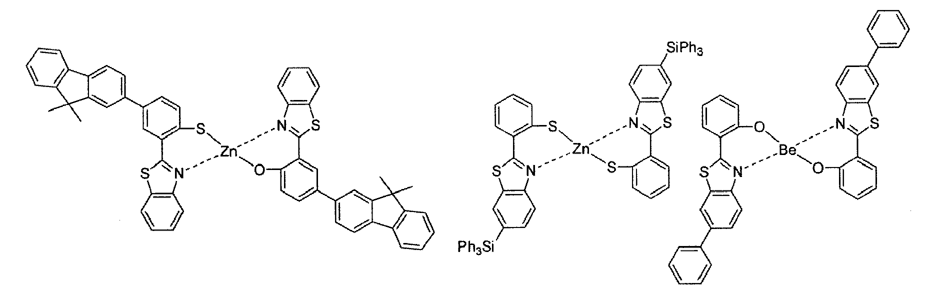

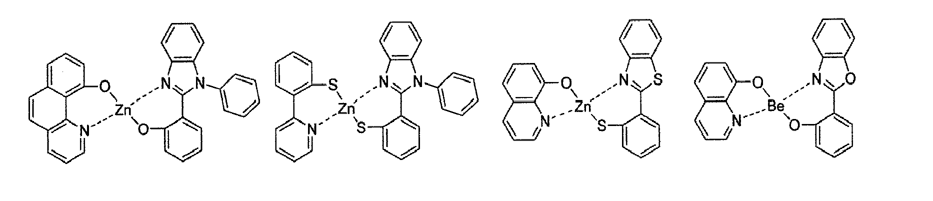

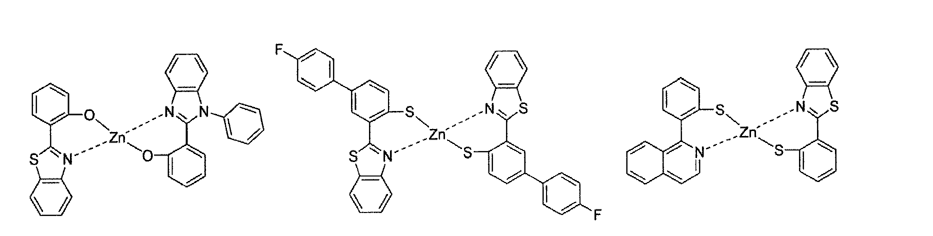

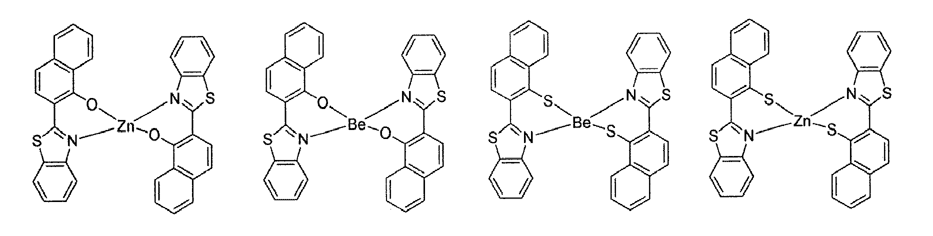

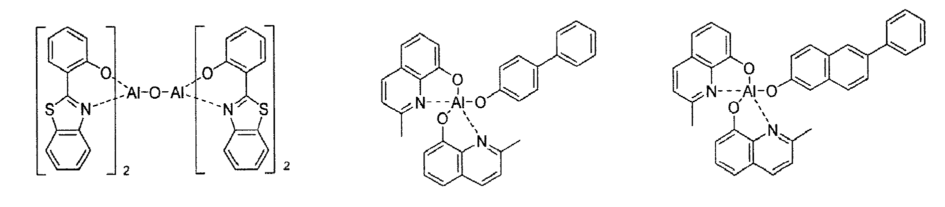

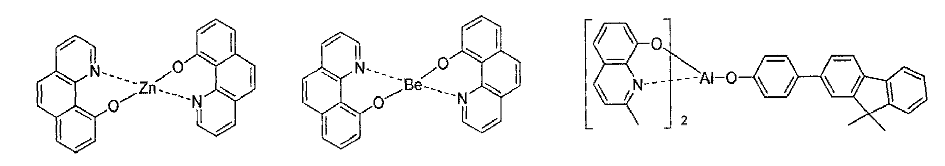

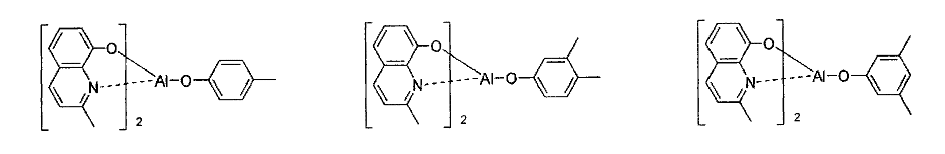

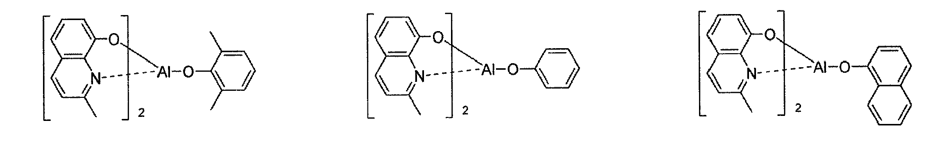

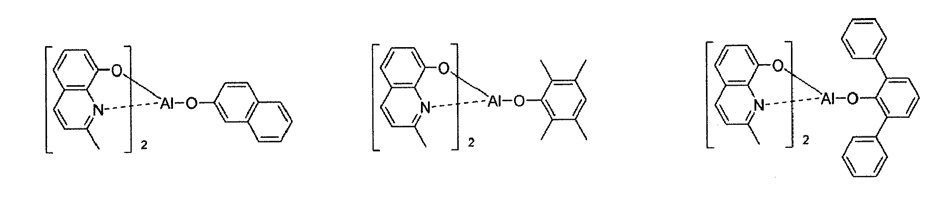

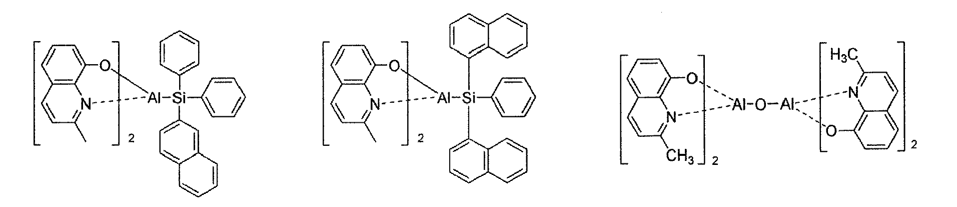

In the electroluminescent device according to the present invention, a layer (hereinafter referred to as "surface layer") selected from a chalcogenide layer, a metal halide layer and a metal oxide layer may be placed on the inner surface of one or both electrode (s) among the pair of electrodes. More specifically, a chalcogenide (including oxide) layer of silicon or aluminum may be placed on the anode surface of the electroluminescent layer, and a metal halide layer or metal oxide layer may be placed on the cathode surface of the electroluminescent layer. A driving stability may be attained therefrom.

The chalcogenide may be, for example, SiOₓ (1 ≤ x ≤ 2), AlOₓ (1 ≤ x ≤ 1.5), SiON, SiAlON, etc. The metal halide may be, for example, LiF, MgF₂, CaF₂, a rare earth metal fluoride, etc. The metal oxide may be, for example, Cs₂O, Li₂O, MgO, SrO, BaO, CaO, etc.

Further, in the electroluminescent device according to the present invention, a mixed region of an electron transport compound and a reductive dopant or a mixed region of a hole transport compound and an oxidative dopant may be placed on the inner surface of one or both electrode(s) among the pair of electrodes. In that case, transport of electrons from the mixed region to the electroluminescent layer becomes easier, because the electron transport compound is reduced to an anion. Further, transport of holes from the mixed region to the electroluminescent layer becomes easier, because the hole transport compound is oxidized to a cation. Preferred examples of the oxidative dopant include various Lewis acids and acceptor compounds. Preferred examples of the reductive dopant include alkali metals, alkali metal compounds, alkaline earth metals, rare earth metals and mixtures thereof.

The present invention further provides an organic solar cell. The organic solar cell according to the present invention comprises one or more electroluminescent compound(s) represented by Chemical Formula 1 or 2.

The electroluminescent device according to the present invention, which comprises an electroluminescent compound with at least one arylamine group (s) substituted by 1- or 2-naphthyl, exhibits superior luminous efficiency, excellent color purity and very good operation life.

### EXAMPLES

Hereinafter, electroluminescence characteristics of the devices according to the present invention will be described for the understanding of the present invention. However, the following examples are for illustrative purposes only and not intended to limit the scope of the present invention.

### [Preparation Examples]

### [Preparation Example 1] Preparation of Compound 90

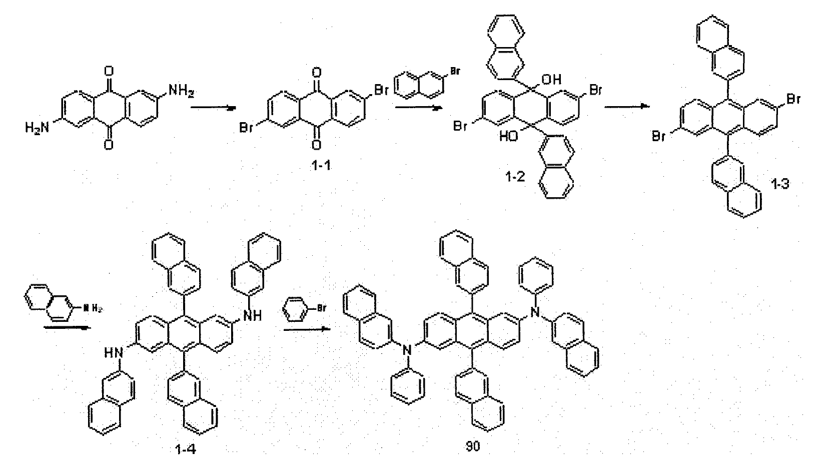

### Preparation of Compound 1-1

Copper bromide (101.0 g, 0.45 mmol), tert-butyl nitrite (58.34 mL, 0.49 mmol) and acetonitrile (800 mL) were mixed and stirred at 70°C. One hour later, 2,6-diaminoanthraquinone (45.0 g, 0.19 mmol) was added and the mixture was stirred at 85 °C. 48 hours later, after adding 20% hydrochloric acid (1 L), followed by stirring for 1 hour, the produced precipitate was filtered and washed several times with water and methanol. After washing 2 times with acetone and dichloromethane, respectively, Compound **1-1** (50.0 g, 72 %) was obtained.

### Preparation of Compound 1-2

2-Bromonaphthalene (80.10 g, 386.85 mmol) was dissolved in dried tetrahydrofuran (500 mL) under nitrogen atmosphere, and 2.5 M n-butyllithium (2.5 M solution in n-hexane, 163.90 mL, 409.83 mmol) was slowly added dropwise at -78°C. After stirring for 1 hour, Compound **1-1** (50.00 g, 136.61 mmol) was added, and the mixture was stirred while slowly heating to room temperature. 17 hours later, after adding water, followed by stirring for 30 minutes, extraction with ethyl acetate (500 mL) and washing with water (500 mL), a desiccant was added to the obtained organic layer to remove water. The solid obtained by distillation under reduced pressure was recrystallized using dichloromethane (300 mL) and n-hexane (300 mL). Compound **1-2** (51.0 g, 59.9 %) was obtained as white powder.

### Preparation of Compound 1-3

Compound **1-2** (51.0 g, 81.9 mmol), potassium iodide (54.38 g, 327.6 mmol), sodium hydrophosphite (52.08 g, 491.4 mmol) and acetic acid (800 mL) were mixed and stirred under reflux. 15 hours later, water (500 mL) was added and the mixture was stirred for 1 hour. The precipitate obtained by filtration under reduced pressure was washed 3 times with water (300 mL) and once with acetone (300 mL). Compound **1-3** (42.6 g, 87.16 %) was obtained with pale yellow color.

### Preparation of Compound 1-4

Compound **1-3** (10 g, 16.99 mmol), 2-naphthylamine (5.59g, 39.07 mmol), tris(dibenzylideneacetone)dipalladium(0) (0.31g, 0.34mmol), 2-di-tert-butylphosphinobiphenyl (0.25 g, 0.85 mmol), sodium tert-butoxide (4.89 g, 50.97 mmol) and toluene (100 mL) were mixed and stirred under reflux under nitrogen atmosphere. 8 hours later, after cooling to room temperature, extraction was carried out using dichloromethane (300 mL). Magnesium sulfate was added to the obtained organic layer to remove water and distillation was carried out under reduced pressure. The resulting product was separated by column chromatography (dichloromethane/n-hexane = 5/1). Compound **1-4** (7.64 g, 63 %) was obtained.

### Preparation of Compound 90

Compound **1-4** (7.51 g, 10.54 mmol), bromobenzene (4.14 g, 26.35 mmol), palladium acetate (0.047 g, 0.21 mmol), sodium tert-butoxide (3.04 g, 31.62 mmol) and dried toluene (100 mL) were heated under nitrogen atmosphere to 60 °C. 1 hour later, tri-tert-butylphosphine (0.11g, 0.53 mmol) was added and the mixture was stirred under reflux. 14 hours later, the produced precipitate was filtered at room temperature under reduced pressure and recrystallized using methanol (500 mL) and tetrahydrofuran (300 mL). Compound **90** (5.2 g, 57 %) was obtained.

### [Preparation Example 2] Preparation of Compound 291

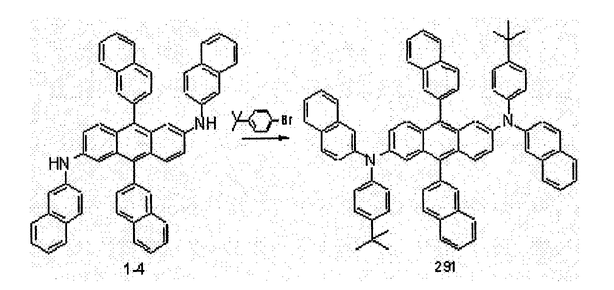

Compound **1-4** (8.5 g, 8.42 mmol), 1-tert-butyl-4-bromophenyl (4.48 g, 21.05 mmol), palladium acetate (0.038 g, 0.17 mmol), sodium tert-butoxide (2.43 g, 25.26 mmol) and dried toluene 100 mL were heated under nitrogen atmosphere to 60 °C. 1 hour later, tri-tert-butylphosphine (0.085 g, 0.42 mmol) was added and the mixture was stirred under reflux. 14 hours later, the produced precipitate was filtered at room temperature under reduced pressure and recrystallized using methanol (500 mL) and tetrahydrofuran (300 mL) . Compound **291** (5.33 g, 49.7%) was obtained.

Electroluminescent compounds (Compounds **1** to **383**) were prepared according to the method of Preparation Examples 1 and 2. Table 1 shows ¹H NMR and MS/FAB data of the prepared electroluminescent compounds.

**[Table 1]**

| compound | ¹H NMR(CDCl₃, 200 MHz) | MS/FAB | |
|---|---|---|---|
| | | found | calculated |
| **1** | δ = 6.63 (6H, m), 6.81∼6.83 (5H, m), 7.03 (2H, m), 7.2 (6H, m), 7.36∼7.41 (3H, m), 7.49∼7.52 (10H, m), 7.74∼7.77 (4H, m), 7.84∼7.88(2H, m) | 714.89 | 714.30 |
| **10** | δ = 1.35 (18H, s), 6.63 (6H, m), 6.81∼6.83 (5H, m), 7.03 (2H, m), 7.2 (6H, m), 7.36∼7.38 (9H, m), 7.49∼7.5 (2H, m), 7.74∼7.77 (4H, m), 7.84∼7.88 (2H, m) | 827.11 | 826.43 |
| **13** | δ = 6.63 (6H, m), 6.81∼6.83 (5H, m), 7.03 (2H, m), 7.2(6H, m), 7.36(1H, m), 7.49∼7.52(6H, m), 7.74∼7.77(4H, m), 7.84∼7.88(4H, m), 7.98∼8(6H, m), 8.45(2H, m) | 927.18 | 916.28 |
| **16** | δ = 6.63 (6H, m), 6.8∼6.81 (5H, m), 7(2H, m), 7.2∼7.36 (13H, m), 7.49∼7.5 (4H, m), 7.63 (2H, m), 7.74∼7.88 (6H,m), 7.94 (2H,m), 8.12 (2H,m), 8.55 (2H, m) | 893.08 | 892.36 |
| **17** | δ = 3.05 (4H, m), 4.14 (4H,m), 6.55 (2H, m), 6.63 (6H, m), 6.72 (2H, m), 6.8∼6.83 (5H, m), 6.96 (1H, m), 7.03∼7.07 (5H, m), 7.2 (6H, m), 7.36 (1H, m), 7.49∼7.5 (2H, m), 7.68 (1H, m), 7.74∼7.77 (3H, m), 7.84∼7.88 (2H, m) | 797.00 | 796.36 |
| **32** | δ = .72 (6H, s), 6.63 (6H, m), 6.81∼6.83 (5H, m), 7.03 (2H, m), 7.2 (6H, m), 7.28 (1H, m), 7.36∼7.41 (3H, m), 7.49∼7.55 (7H,m), 7.63 (1H,m), 7.74∼7.77 (5H,m), 7.84∼7.93 (4H, m) | 831.05 | 830.37 |
| **45** | δ = 6.63 (6H, m), 6.81∼6.83 (5H, m), 7.03 (2H, m), 7.2 (6H, m), 7.36 (1H, m), 7.49∼7.61 (8H, m), 7.73∼7.77 (5H, m), 7.84∼7.92 (3H, m), 8∼8.08 (4H, m), 8.42 (1H, m), 8.55 (1H, m) | 815.01 | 814.33 |
| **48** | δ = 1.96 (2H,m), 2.76 (2H,m), 3.06 (2H,m), 6.55 (1H, m), 6.63 (6H, m), 6.72 (1H, m), 6.81∼6.83 (5H, m), 6.97 (1H, m), 7.03∼7.07 (3H, m), 7.2 (6H, m), 7.36∼7.41 (2H, m), 7.49∼7.52 (6H, m), 7.74∼7.77 (4H, m), 7.84∼7.88 (2H, m) | 769.97 | 769.35 |
| **65** | δ = 1.72 (6H, s), 6.63 (6H, m), 6.81∼6.83 (5H, m), 7.03 (2H, m), 7.2 (6H, m), 7.28 (1H, m), 7.36∼7.38 (2H, m), 7.49∼7.63 (7H, m), 7.73∼7.77 (6H, m), 7.84∼7.93 (5H, m), 8 (2H, m) | 881.11 | 880.38 |
| **71** | δ = 1.72 (6H, s), 6.63 (6H, m), 6.81∼6.83 (5H, m), 7.03 (2H, m), 7.2∼7.28 (11H, m), 7.36∼7.41 (3H, m), 7.49∼7.55 (7H, m), 7.63 (1H, m), 7.74∼7.77 (5H, m), 7.84∼7.93 (4H, m) | 907.15 | 906.40 |
| **87** | δ = 6.63 (6H, m), 6.81∼6.83 (5H, m), 7.03 (2H, m), 7.2 (6H, m), 7.36 (1H, m), 7.49∼7.5 (2H, m), 7.74∼7.77 (4H, m), 7.84∼7.88 (2H, m) | 724.95 | 724.37 |
| **90** | δ = 6.63 (4H, m), 6.81∼6.83 (4H, m), 7.03 (2H, m), 7.2 (4H, m), 7.36 (2H, m), 7.49∼7.5 (4H, m), 7.58∼7.59 (6H, m), 7.73∼7.77 (8H, m), 7.84∼7.92 (6H, m), 8 (4H, m) | 865.07 | 864.35 |
| **97** | δ = 1.96 (4H, m), 2.76 (4H,m), 3.06 (4H, m), 6.55 (2H, m), 6.63 (4H, m), 6.72 (2H, m), 6.81 (4H, m), 6.99∼7.07 (6H, m), 7.2 (4H, m), 7.36 (2H, m), 7.49∼7.5 (4H, m), 7.71∼7.77 (6H, m), 7.84∼7.88 (4H, m) | 875.11 | 874.40 |
| **114** | δ = 6.63 (4H, m), 6.81∼6.83 (4H, m), 7.03 (2H, m), 7.2 (4H, m), 7.36∼7.41 (3H, m), 7.48∼7.51 (14H, m), 7.7∼7.77 (8H, m), 7.84∼7.92 (5H, m), 8 (2H, m) | 891.11 | 890.37 |
| **127** | δ = 2.34 (3H, m), 6.63 (4H, m), 6.81∼6.83 (4H, m), 7.03(2H, m), 7.2(4H, m), 7.29∼7.36(6H, m), 7.4g∼7.55(6H, m), 7.61(1H, m), 7.74∼7.77(6H, m), 7.84∼7.88(4H, m), 8.04∼8.08(2H, m), 8.42(1H, m), 8.55(1H, m) | 829.04 | 828.35 |
| **141** | δ = 6.63(4H, m), 6.81∼6.83(4H, m), 7.03(2H, m), 7.2∼7.25(8H, m), 7.36(2H, m), 7.49∼7.61(10H, m), 7.73∼7.77(7H, m), 7.84∼7.92(5H, m), 8∼8.08(4H, m), 8.42 (1H, m), 8.55 (1H, m) | 941.16 | 940.38 |
| **150** | δ = 1.72 (6H, s), 2.34 (6H, s), 6.63 (4H, m), 6.81∼6.83 (4H, m), 7.03 (2H, m), 7.2 (4H, m), 7.28∼7.38 (5H, m), 7.49∼7.63 (8H, m), 7.74∼7.77 (7H, m), 7.84∼7.93 (6H, m) | 909.16 | 908.41 |
| **158** | δ = 6.83 (2H, m), 7.03 (2H, m), 7.36∼7.41 (6H, m), 7.49∼7.52 (16H, m), 7.74∼7.77 (10H, m), 7.84∼7.88 (8H, m) | 865.07 | 864.35 |
| **194** | δ = 6.83 (2H, m), 7.03 (2H, m), 7.25 (4H, m), 7.36∼7.41 (5H, m), 7.49∼7.52 (12H, m), 7.58∼7.59 (3H, m), 7.73∼7.77 (11H, m), 7.84∼7.92 (9H, m), 8 (2H, m) | 991.22 | 990.40 |
| **203** | δ = 1.72 (6H, s), 6.83 (2H, m), 7.03 (2H, m), 7.25∼7.28 (5H, m), 7.36∼7.38 (5H, m), 7.49∼7.55 (11H, m), 7.61∼7.63 (2H, m), 7.74∼7.77 (11H, m), 7.84∼7.93 (10H, m), 8.04∼8.08 (2H, m), 8.42 (1H, m), 8.55 (1H, m) | 1107.38 | 1106.46 |
| **213** | δ = 6.63 (6H, m), 6.81∼6.83 (5H, m), 6.98∼7.03 (3H, m), 7.2 (6H, m), 7.38 (1H, m), 7.53∼7.57 (3H, m), 7.75 (2H, m), 7.82∼7.93 (10H, m), 8.02∼8.12 (6H, m), 8.93 (4H, m) | 915.13 | 914.37 |
| **222** | δ = 6.63 (6H, m), 6.81∼6.83 (5H, m), 6.98∼7.03 (3H, m), 7.2 (6H, m), 7.38 (1H, m), 7.53∼7.61 (6H, m), 7.75 (2H, m), 7.82∼7.93 (5H, m), 8.02∼8.12 (6H, m), 8.42 (1H, m), 8.55 (1H, m), 8.93 (2H, m) | 865.07 | 864.35 |
| **243** | δ = 6.63 (4H, m), 6.81∼6.83 (4H, m), 6.98∼7.03 (4H, m), 7.2 (4H, m), 7.38 (2H, m), 7.53∼7.59 (12H, m), 7.73∼7.75 (4H, m), 7.92 (2H, m), 8∼8.07 (8H, m) | 865.07 | 864.35 |
| **245** | δ = 6.63 (4H, m), 6.81∼6.83 (4H, m), 6.98∼7.03 (4H, m), 7.2 (4H, m), 7.38 (2H, m), 7.53∼7.57 (6H, m), 7.75 (2H, m), 7.82∼7.93 (10H, m), 8.02∼8.12 (8H, m), 8.93 (4H, m) | 965.19 | 964.38 |
| **262** | δ = 1.72 (6H, s), 6.63 (4H, m), 6.81∼6.83 (4H, m), 6.98∼7.03 (4H, m), 7.2∼7.28 (9H, m), 7.38 (3H, m), 7.53∼7.63 (11H, m), 7.75∼7.77 (3H, m), 7.87∼7.93 (2H, m), 8.02∼8.08 (6H, m), 8.42 (1H, m), 8.55 (1H, m) | 1007.27 | 1006.43 |
| **273** | δ = 6.83 (2H, m), 6.98∼7.03 (6H, m), 7.38 (4H, m), 7.53∼7.59 (18H, m), 7.73∼7.75 (4H, m), 7.92 (2H, m), 8∼8.07 (12H, m) | 965.19 | 964.38 |
| **287** | δ =2.34 (12H, s), 6.36 (4H,m), 6.71 (2H,m), 6.83 (2H, m), 7.03 (2H, m), 7.36 (2H, m), 7.49∼7.5 (4H, m), 7.74∼7.93 (20H, m), 8.12 (4H, m), 8.93 (4H, m) | 1021.29 | 1020.44 |
| **291** | δ = 1.35 (18H, s), 6.55 (4H, m), 6.83 (2H, m), 7.01∼7.03 (6H, m), 7.36 (2H, m), 7.49∼7.5 (4H, m), 7.58∼7.59 (6H, m), 7.73∼7.77 (8H, m), 7.84∼7.92 (6H, m), 8 (4H, m) | 977.28 | 976.48 |
| **293** | δ = 1.35(9H, s), 6.55(2H, m), 6.63(2H, m), 6.81∼6.83 (3H, m), 7.01∼7.03 (4H, m), 7.2∼7.25 (10H, m), 7.36∼7.41 (4H, m), 7.49∼7.52 (12H, m) 7.74∼7.77 (6H, m), 7.84∼7.88 (4H, m) | 973.25 | 972.44 |
| **300** | δ = 6.83 (2H, m), 7.03 (2H, m), 7.36 (2H, m), 7.49∼7.5 (4H, m), 7.58∼7.59 (6H, m), 7.73∼7.77 (8H, m), 7.84∼7.92 (5H, m), 8 (4H, m) | 875.13 | 874.41 |
| **303** | δ = 6.72 (2H, m), 6.83 (2H,m), 7.03 (2H,m), 7.22 (2H, m), 7.36∼7.41 (4H, m), 7.49∼7.52 (12H, m), 7.7∼7.77 (8H, m), 7.84∼7.88 (4H, m) | 950.97 | 950.28 |
| **309** | δ = 1.35 (18H, s), 6.55 (4H, m), 6.83 (2H, m), 6.98∼7.03 (8H, m), 7.38 (2H, m), 7.53∼7.59 (12H, m), 7.73∼7.75 (4H, m), 7.92 (2H, m), 8∼8.07 (8H, m) | 877.28 | 976.48 |
| **311** | δ = 6.69 (4H, m), 6.83 (2H, m), 6.98∼7.03 (4H, m), 7.38∼7.41 (6H, m), 7.51∼7.57 (26H, m), 7.75 (2H, m), 8.02∼8.07 (4H, m) | 917.14 | 916.38 |
| **313** | δ = 2.34 (12H, s), 6.36 (4H, m), 6.71 (2H, m), 6.83 (2H, m), 6.98∼7.03 (4H, m), 7.38 (2H, m), 7.53∼7.59 (12H, m), 7.73∼7.75 (4H, m), 7.92 (2H, m), 8∼8.07 (8H, m) | 921.18 | 920.41 |
| **323** | δ = 6.63 (4H, m), 6.81 (2H, m), 7.02 (2H, m), 7.2 (4H, m), 7.36 (2H, m), 7.49∼7.5 (4H, m), 7.71∼7.77 (10H, m), 7.84∼7.88 (4H, m) | 636.78 | 636.26 |
| **325** | δ = 1.35 (18H, s), 6.55 (4H, m), 7.01∼7.02 (6H, m), 7.36 (2H, m), 7.49∼7.5 (4H, m), 7.71∼7.77 (10H, m), 7.84∼7.88 (4H, m) | 748.99 | 748.38 |
| **328** | δ = 6.81 (4H, m), 7.02 (2H, m), 7.36∼7.39 (6H, m), 7.49∼7.5 (4H, m), 7.71∼7.77 (10H, m), 7.84∼7.88 (4H, m) | 686.80 | 686.25 |
| **335** | δ = 1.72 (12H, s), 6.58 (2H, m), 6.75 (2H, m), 7.24 (2H, s), 7.28 (2H, m), 7.36∼7.41 (6H, m), 7.49∼7.55 (10H, m), 7.62 (2H, m), 7.71∼7.88 (18H, m) | 1021.29 | 1020.44 |
| **344** | δ = 6.63 (4H, m), 6.81 (2H, m), 7.2 (4H, m), 7.36 (2H, m) 7.49∼7.5 (4H, m), 7.52 (2H, s), 7.74∼7.88 (12H, m), 8.12 (2H, m), 8.93 (2H, m) | 662.82 | 662.27 |
| **355** | δ = 1.72 (12H, s), 6.58 (2H, m), 6.75 (2H, m) 6.98 (2H, m), 7.28 (2H, m), 7.38 (4H, m), 7.52 (2H, s), 7.53∼7.62 (10H, m), 7.82∼7.88 (6H, m), 8.02∼8.12 (6H, m), 8.93 (2H, m) | 895.14 | 894.40 |
| **356** | δ = 1.78 (6H, s), 6.63∼6.64 (7H, m), 6.81∼6.83 (5H, m), 7.03 (1H, m), 7.2 (6H, m), 7.36∼7.41 (3H, m), 7.49∼7.52 (10H, m), 7.74∼7.75 (2H, m), 7.76 (1H, s), 7.77 (1H, m), 7.84∼7.88 (3H, m), 8.07 (1H, s), (H, ) | 831.05 | 830.37 |
| **357** | δ = 1.78 (6H, s), 6.63∼6.64 (5H, m), 6.81∼6.83 (4H, m), 7.03 (1H, m), 7.2 (4H, m), 7.36∼7.41 (4H, m), 7.49∼7.52 (12H, m), 7.74∼7.75 (3H, m), 7.76 (1H, s), 7.77 (2H, m), 7.84∼7.88 (5H, m), 8.07 (1H, s), (H, ) | 881.11 | 880.38 |
| **362** | δ = 1.72 (12H, s), 1.78 (6H, s), 6.63∼6.64 (5H, m), 6.81∼6.83 (4H, m), 7.03 (1H, m), 7.2 (4H, m), 7.28 (2H, m), 7.36∼7.38 (4H, m), 7.49∼7.55 (6H, m), 7.63 (2H, m), 7.74∼7.75 (3H, m), 7.76 (1H, s), 7.77 (4H, m), 7.84∼7.93 (9H, m), 8.07 (1H, s), (H, ) | 1113.43 | 1113.51 |
| **365** | δ = 6.63 (4H, m), 6.95∼6.98 (6H, m), 7.38 (4H, m), 7.53∼7.57 (12H, m), 7.77 (4H, m), 8.02∼8.07 (8H, m) | 714.89 | 714.30 |
| **374** | δ = 1.72 (6H, s), 6.58∼6.63 (7H, m), 6.75∼6.81 (3H, m), 6.95 (2H, m), 7.2 (4H, m), 7.36 (2H, m), 7.49∼7.54 (5H, m), 7.62 (1H, m), 7.71∼7.77 (7H, m), 7.84∼7.88 (5H, m) | 730.94 | 740.33 |
| **376** | δ = 1.72 (6H, s), 6.58∼6.63 (9H, m), 6.75∼6.81 (4H, m), 6.95∼6.98 (3H, m), 7.2 (6H, m), 7.38 (1H, m), 7.53∼7.62 (5H, m), 7.71∼7.77 (3H, m), 7.87 (1H, m), 8.02∼8.07 (2H, m) | 680.88 | 680.32 |
| **377** | δ = 1.72 (6H, s), 6.58∼6.63 (9H, m), 6.75∼6.81 (4H, m), 6.95 (2H, m), 7.2 (6H, m), 7.36 (1H, m), 7.49∼7.54 (3H, m), 7.62 (1H, m), 7.71∼7.77 (5H, m), 7.84∼7.88 (3H, m) | 680.88 | 680.32 |
| **383** | δ = 1.72 (12H, s), 6.58∼6.63 (6H, m), 6.75∼6.81 (4H, m), 6.95 (2H, m), 7.2 (4H, m), 7.36 (2H, m), 7.49∼7.54 (6H, m), 7.62 (2H, m), 7.71∼7.77 (6H, m), 7.84∼7.88 (6H, m) | 847.10 | 846.40 |

### [Example 1] Manufacture of electroluminescent device

First, a transparent electrode ITO film (15 Ω/□) 2 prepared from a glass substrate for an OLED (Samsung Corning) 1 was subjected to ultrasonic washing sequentially using trichloroethylene, acetone, ethanol and distilled water, and stored in isopropanol for later use.

Next, the ITO substrate was mounted on a substrate holder of a vacuum deposition apparatus. After filling 4,4',4"-tris(N,N-(2-naphthyl)-phenylamino)triphenylamine (2-TNATA) in a cell of the vacuum deposition apparatus, the pressure inside the chamber was reduced to 10⁻⁶ torr. Then, 2-TNATA was evaporated by applying electrical current to the cell. A hole injection layer 3 having a thickness of 60 nm was formed on the ITO substrate.

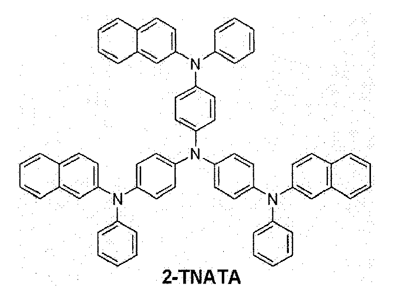

Subsequently, after filling *N,N'*-bis(α-naphthyl)-*N,N'*-diphenyl-4,4'-diamine (NPB) in another cell of the vacuum deposition apparatus, NPB was evaporated by applying electrical current. A hole transport layer 4 having a thickness of 20 nm was formed on the hole injection layer.

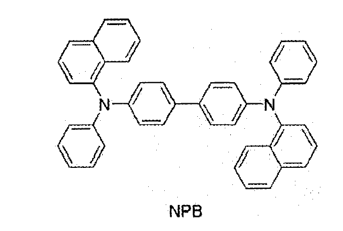

An electroluminescent layer 5 was formed on the hole transport layer as follows. Compound **H-28** was filled in a cell of a vacuum deposition apparatus as a host, and Compound **90** was filled in another cell as a dopant. The two materials were evaporated at different speed, so that an electroluminescent layer 5 having a thickness of 30 nm was formed on the hole transport layer at 2 to 5 mol% based on the host.

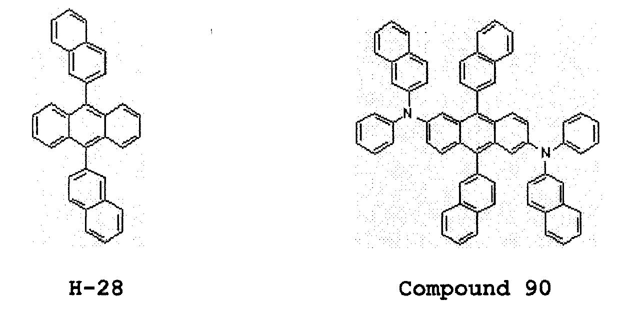

Thereafter, tris(8-hydroxyquinoline)-aluminum(III) (Alq) was deposited with a thickness of 20 nm as an electron transport layer 6. Then, lithium quinolate (Liq) was deposited with a thickness of 1 to 2 nm as an electron injection layer 7. Then, an Al cathode 8 having a thickness of 150 nm was formed using another vacuum deposition apparatus to manufacture an OLED.

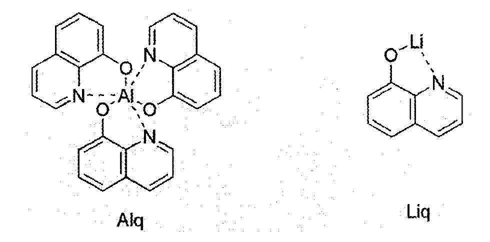

Each OLED electroluminescent material was purified by vacuum sublimation at 10⁻⁶ torr.

### [Comparative Example 1] Manufacture of electroluminescent device

After forming a hole injection layer 3 and a hole transport layer 4 as in Example 1, Compound **H-28** was filled in a cell of a vacuum deposition apparatus as a host, and Compound **A** was filled in another cell. The two materials were evaporated at different speed, so that an electroluminescent layer 5 having a thickness of 30 nm was formed on the hole transport layer at 2 to 5 mol% based on the host.

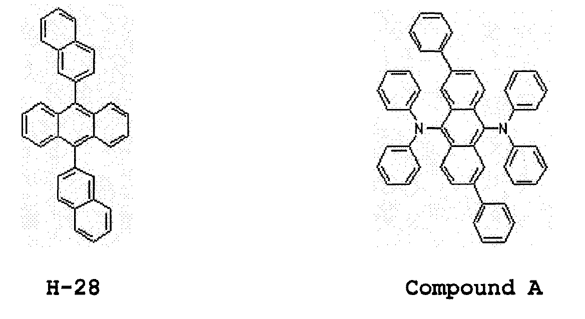

Thereafter, after forming an electron transport layer 6 and an electron injection layer 7 as in Example 1, an Al cathode 8 having a thickness of 150 nm was formed using another vacuum deposition apparatus to manufacture an OLED.

Luminous efficiency of the OLED devices manufactured in Example 1 and Comparative Example 1 was measured at 5,000 cd/m². The result is given in Table.

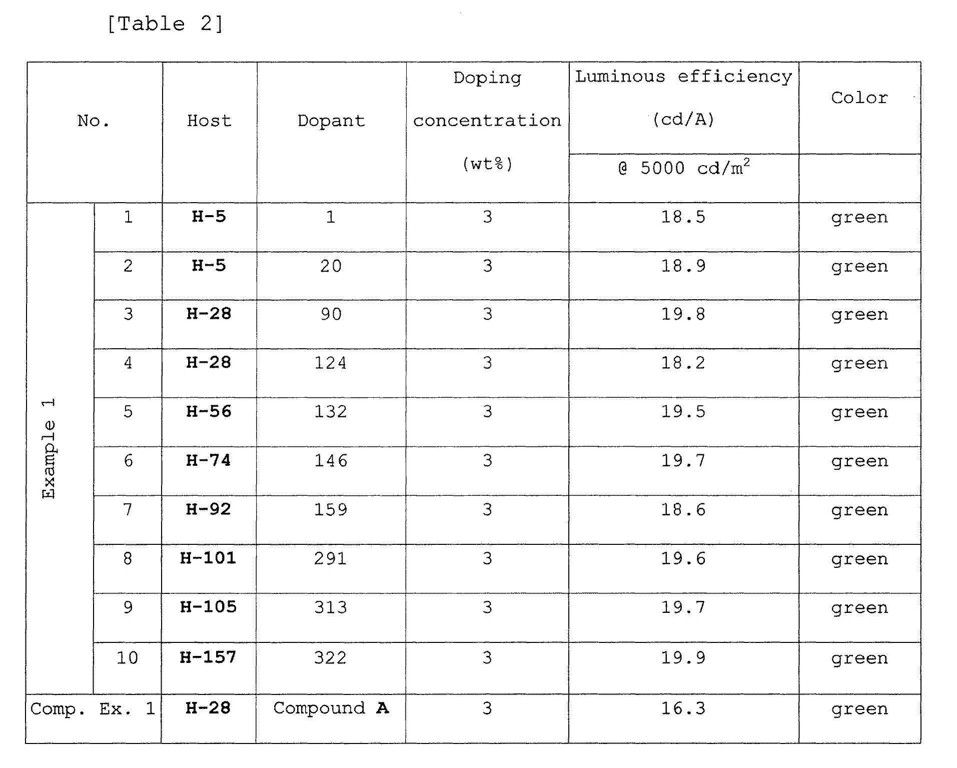

As seen in Table 2, when the electroluminescent materials according to the present invention were used to manufacture green-emitting electroluminescent devices, the electroluminescent devices exhibited improved luminous efficiency while maintaining color purity comparable to or better than that of Comparative Example 1.

While the exemplary embodiments have been shown and described, it will be understood by those skilled in the art that various changes in form and details may be made thereto without departing from the spirit and scope of this disclosure as defined by the appended claims.

In addition, many modifications can be made to adapt a particular situation or material to the teachings of this disclosure without departing from the essential scope thereof. Therefore, it is intended that this disclosure not be limited to the particular exemplary embodiments disclosed as the best mode contemplated for carrying out this disclosure, but that this disclosure will include all embodiments falling within the scope of the appended claims.

## Claims

1. An electroluminescent device comprising an organic layer interposed between an anode and a cathode on a substrate, wherein the organic layer comprises an electroluminescent layer comprising one or more dopant compound(s) represented by Chemical Formula 1 and one or more host compound(s) represented by Chemical Formula 2: 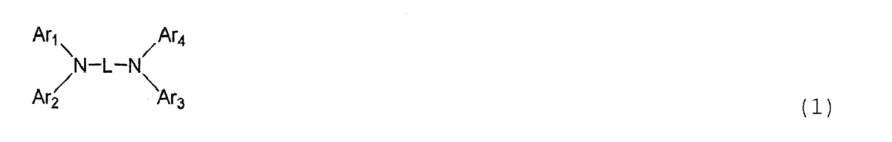 wherein
Ar₁ through Ar₄ independently represent (C6-C60)aryl with or without substituent or (C5-C50) heteroaryl with or without substituent, with the proviso that at least one of Ar₁ through Ar₄ is 1- or 2-naphthyl with or without substituent;
the substituent substituted at the aryl or heteroaryl of Ar₁ through Ar₄ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsily tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
L represents (C6-C60)arylene with or without substituent, (C3-C60)heteroarylene with or without substituent, stilbenylene with
or without substituent, 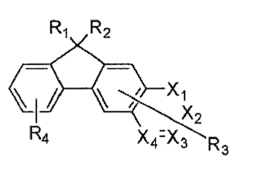 or 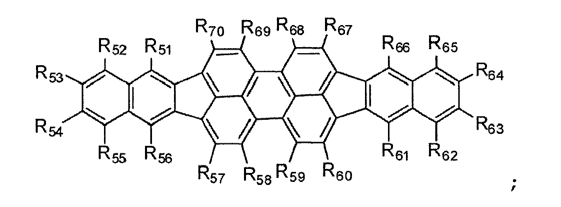 the substituent substituted at the arylene, heteroarylene or stilbenylene of L is one or more selected from deuterium, halogen, (C1-C60)alkyl, halo(C1-C60)alkyl, (C6-C60)aryl, (C3-C60) heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
X₁ through X₄ independently represent carbon or nitrogen;
R₁ through R₄ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, amino, mono- or di-(C1-C60)alkylamino, mono- or di-(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₁ through R₄ may be linked to an adjacent substituent to form a saturated or unsaturated mono- or polycyclic ring or heteroaromatic ring;
R₅₁ through R₇₀ independently represent hydrogen, deuterium, halogen, (C1-C60)alky (C6-C60)aryl, (C4-C60)heteroaryl, (C1-C60)alkylamino or (C6-C60)arylamino, or each of R₅₁ through R₇₀ may be linked to an adjacent substituent via (C3-C60)alkylene or (C3-C60) alkenylene with or without a fused ring to form an alicyclic ring or a mono- or polycyclic ring;
the aryl, heteroaryl or arylamino, or the mono- or polycyclic aromatic ring may be further substituted by one or more substituent (s) selected from deuterium, halogen, (C1-C60)alkyl, (C6-C60)aryl, (C4-C60) heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(Cl-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, carboxyl, nitro and hydroxyl;
Ar₁₀-(An)ₐ-Ar₂₀ (2)
wherein
An represents anthracenylene with or without one or more substituent(s) or -X-L₁₀₀-Y-;
X and Y independently represent anthracenylene;
L₁₀₀ represents (C6-C60)arylene or (C5-C60)heteroarylene with or without one or more substituent(s);
Ar₁₀ and Ar₂₀ independently represent (C6-C60) aryl with or without substituent or (C5-C60)heteroaryl with or without substituent;
the substituent substituted at An, Ar₁₀, Ar₂₀ or L₁₀₀ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a represents an integer from 1 to 4.

2. The electroluminescent device according to claim 1, wherein Ar₁ through Ar₄ are independently selected from the following structures: 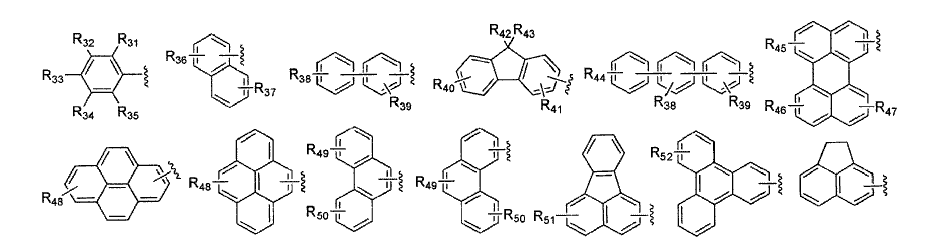 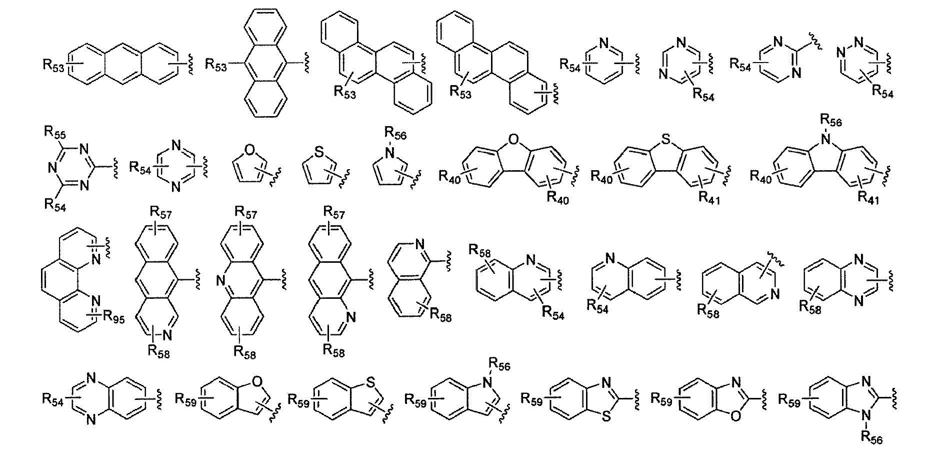 wherein
R₃₁ through R₅₉ independently represent hydrogen, deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, and R₄₂ and R₄₃ may be linked via (C3-C60)alkylene or (C3-C60)alkenylene with or without a fused ring to form an alicyclic ring or a mono- or polycyclic ring.

3. The electroluminescent device according to claim2, wherein at least one of 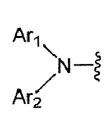 and 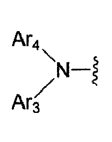 is selected from the following structures:

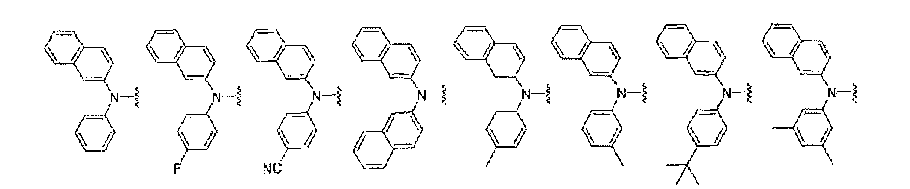

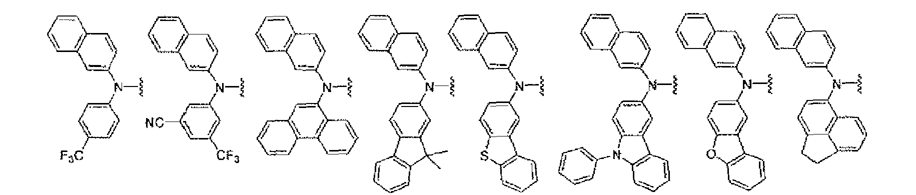

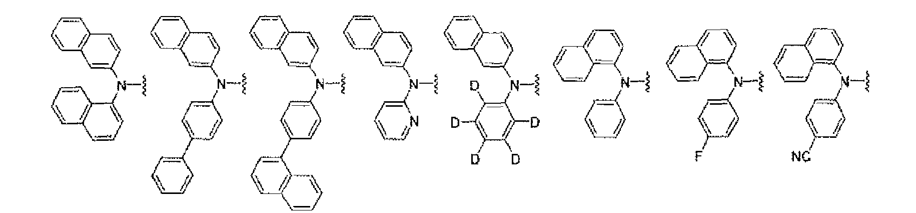

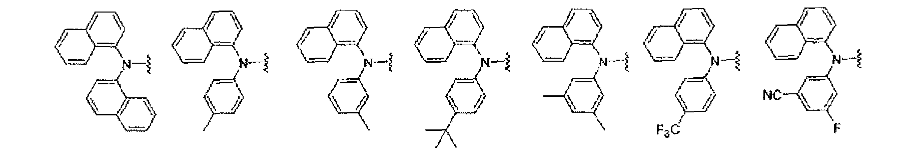

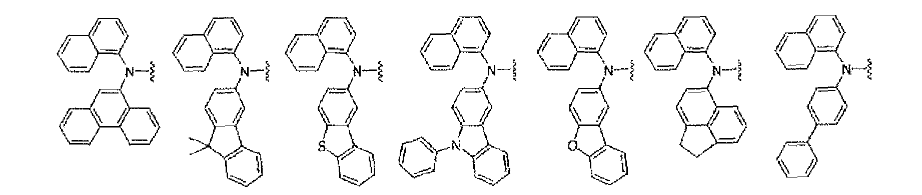

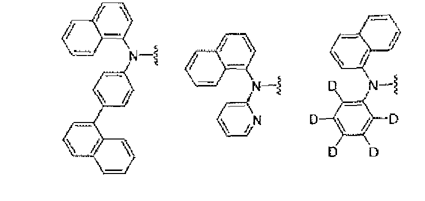

4. The electroluminescent device according to claim 1, wherein L is selected from the following structures: 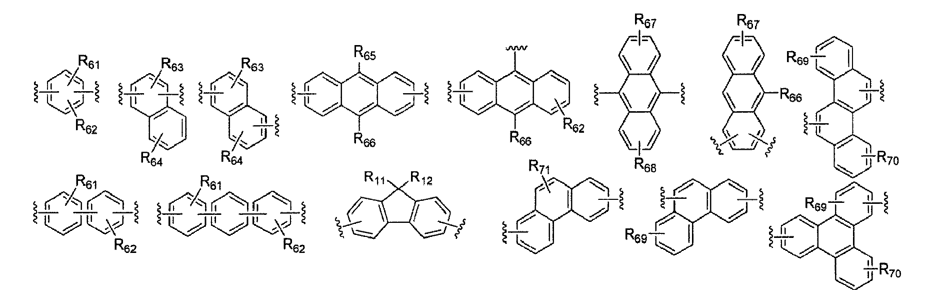 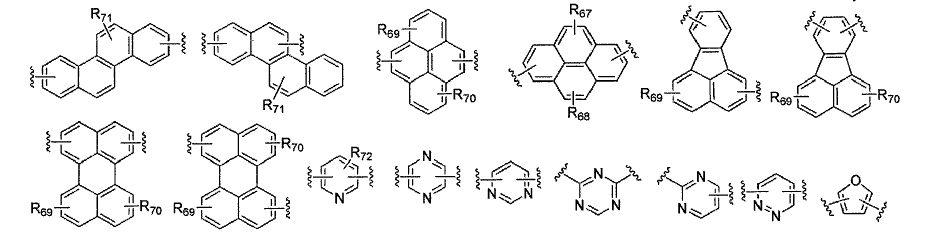 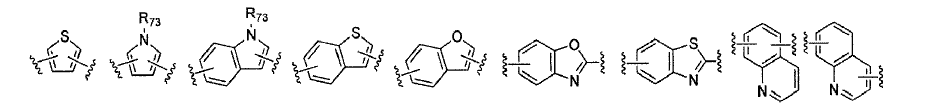 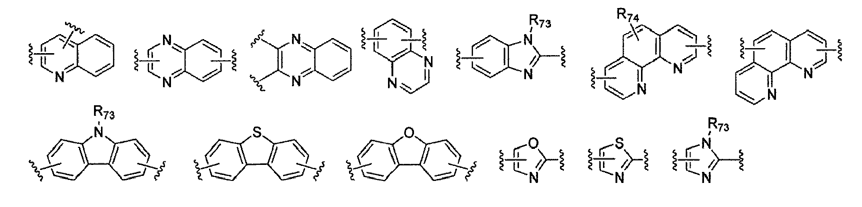 wherein
R₆₁ through R₇₄ independently represent hydrogen, deuterium, halogen, halo(C1-C60)alkyl, (C1-C60)alkyl, (C6-C60)aryl, (C3-C60)heteroaryl with or without (C6-C60)aryl substituent, 5- or 6-membered heterocycloalkyl containing one or more heteroatom(s) selected from N, O and S, 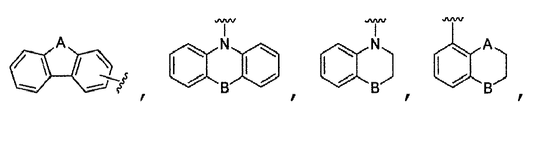 (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, carbazolyl, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, and the alkyl, cycloalkyl, heterocycloalkyl, aryl, heteroaryl, arylsilyl, alkylsilyl, alkylamino or arylamino may be further substituted by one or more substituent(s) selected from deuterium, halogen, (C1-C60)alkyl, halo(C1-C60)alkyl, (C6-C60)aryl, (C3-C60) heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
A represents -C(R₁₂)(R₁₃)-, -N(R₁₄)-, -S-, -O- or -Si(R₁₅)(R₁₆)-;
B represent a chemical bond, -(CR₁₂R₁₃)ₘ-, -N(R₁₄)-, -S-, -O-, -Si(R₁₅)(R₁₆)- or -(R₂₅)C=C(R₂₆)-;
R₁₂ through R₁₆, R₂₅ and R₂₆ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, amino, mono- or di-(C1-C60)alkylamino, mono- or di-(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₂ and R₁₃, and R₁₅ and R₁₆ may be linked via (C3-C60) alkylene or (C3-C60) alkenylene with or without a fused ring to form an alicyclic ring or a mono- or polycyclic ring; and
m represents an integer from 1 to 4.

5. The electroluminescent device according to claim 1, wherein the host is selected from the compounds represented by Chemical Formulas 3 to 5: 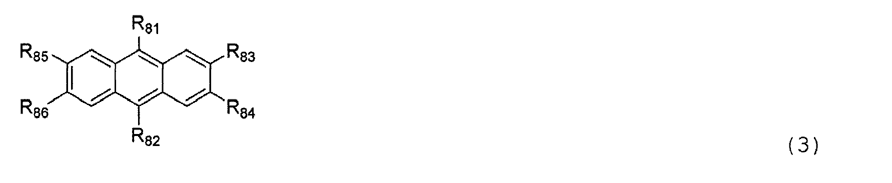 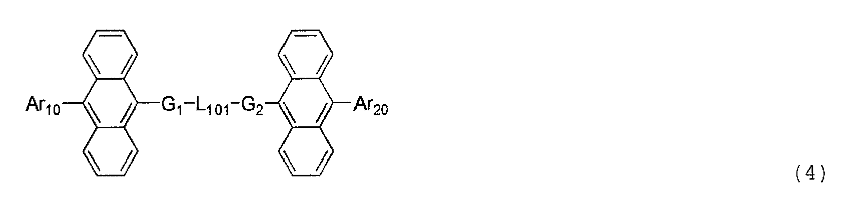 wherein
R₈₁ and R₈₂ independently represent (C6-C60)aryl, (C4-C60)heteroaryl, 5- or 6-membered heterocycloalkyl containing one or more heteroatom(s) selected from N, O and S or (C3-C60) cycloalkyl, and the aryl or heteroaryl of R₈₁ and R₈₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₈₃ through R₈₆ independently represent hydrogen, (C1-C60) alkyl, (Cl-C60)alkoxy, halogen, (C4-C60)heteroaryl, (C5-C60)cycloalkyl or (C6-C60)aryl, and the heteroaryl, cycloalkyl or aryl of R₈₃ through R₈₆ may be further substituted by one or more substituent (s) selected from a group consisting of (C1-C60)alkyl with or without halogen substituent, (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond or (C6-C60)arylene with or without one or more substituent(s) selected from (C1-C60)alkyl, (C1-C60)alkoxy, (C6-C60)aryl, (C4-C60)heteroaryl and halogen;
Ar₁₀ and Ar₂₀ independently represent (C4-C60)heteroaryl or aryl selected from the following structures: 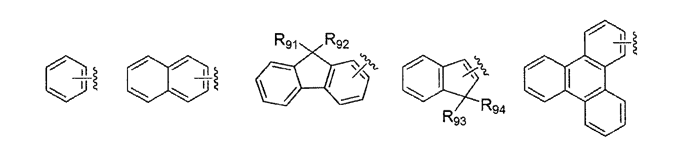 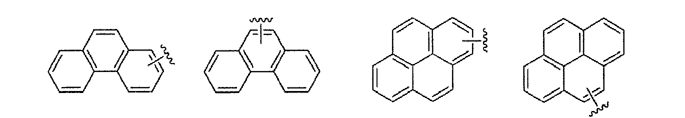 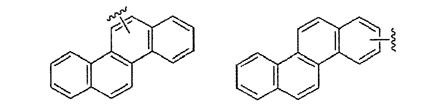 the aryl or heteroaryl of Ar₁₀ and Ar₂₀ may be substituted by one ormore substituent (s) selected from (C1-C60)alkyl, (C1-C60)alkoxy, (C6-C60)aryl and (C4-C60)heteroaryl;
L₁₀₁ represents (C6-C60)arylene, (C4-C60)heteroarylene or a compound of the following formula: 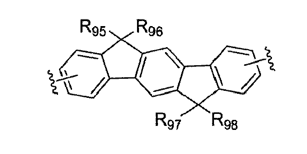 the arylene or heteroarylene of L₁₀₁ may be substituted by one or more substituent(s) selected from (C1-C60)alkyl, (C1-C60)alkoxy, (C6-C60)aryl, (C4-C60)heteroaryl and halogen;
R₉₁ through R₉₄ independently represent hydrogen, (C1-C60)alkyl or (C6-C60)aryl, or each of themmay be linked to an adjacent substituent via (C3-C60)alkylene or (C3-C60)alkenylene with or without a fused ring to form an alicyclic ring or a mono- or polycyclic ring;
R₉₅ through R₉₈ independently represent hydrogen, (C1-C60) alkyl, (C1-C60)alkoxy, (C6-C60)aryl, (C4-C60)heteroaryl or halogen, or each of them may be linked to an adjacent substituent via (C3-C60) alkylene or (C3-C60)alkenylene with or without a fused ring to form an alicyclic ring or a mono- or polycyclic ring; 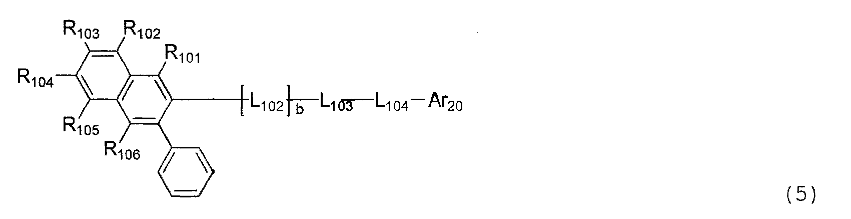 wherein
L₁₀₂ represents anthracenylene;
L₁₀₃ and L₁₀₄ independently represent a chemical bond, (C1-C60)alkyleneoxy, (Cl-C60)alkylenethio, (C6-C60)aryleneoxy, (C6-C60)arylenethio, (C6-C60)arylene or (C3-C60)heteroarylene containing one or more heteroatom(s) selected from N, O and S;
Ar₂₀ represents (C6-C60)aryl or (C5-C60)heteroaryl with or without one or more substituent (s) selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
R₁₀₁ through R₁₀₆ independently represent hydrogen, deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl; and
b represents an integer from 1 to 4.

6. The electroluminescent device according to claim 1, wherein the organic layer comprises one or more compound(s) selected from arylamine compounds and styrylarylamine compounds.

7. The electroluminescent device according to claim 1, wherein the organic layer further comprises one or more metal(s) selected from a group consisting of organic metals of Group 1, Group 2, 4th period and 5th period transition metals, lanthanide metals and d-transition elements.

8. The electroluminescent device according to claim 1, wherein the organic layer further comprises a compound having an electroluminescence peak with red, green or blue wavelength.

9. The electroluminescent device according to claim 1, wherein the organic layer comprises an electroluminescent layer and a charge generating layer.

10. An organic solar cell comprising an electroluminescent compound represented by Chemical Formula 1 or 2: 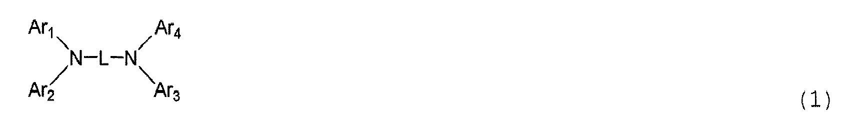 wherein
Ar₁ through Ar₄ independently represent (C6-C60)aryl with or without substituent or (C5-C50)heteroaryl with or without substituent, with the proviso that at least one of Ar₁ through Ar₄ is 1- or 2-naphthyl with or without substituent;
the substituent substituted at the aryl or heteroaryl of Ar₁ through Ar₄ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
L represents (C6-C60)arylene with or without substituent, (C3-C60) heteroarylene with or without substituent, stilbenylene with or without substituent, 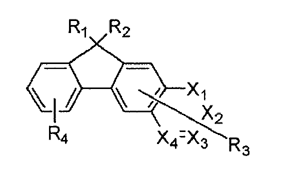 or 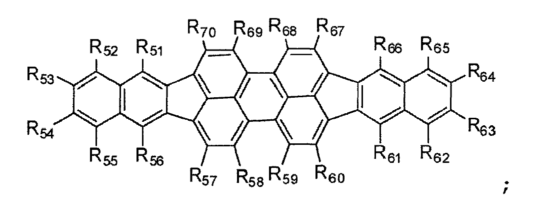 the substituent substituted at the arylene, heteroarylene or stilbenylene of L is one or more selected from deuterium, halogen, (C1-C60)alkyl, halo(C1-C60)alkyl, (C6-C60)aryl, (C3-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl;
X₁ through X₄ independently represent carbon or nitrogen;
R₁ through R₄ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, amino, mono- or di-(C1-C60)alkylamino, mono- or di-(C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₁ through R₄ may be linked to an adjacent substituent to form a saturated or unsaturated mono- or polycyclic ring or heteroaromatic ring;
R₅₁ through R₇₀ independently represent hydrogen, deuterium, halogen, (C1-C60)alkyl, (C6-C60)aryl, (C4-C60)heteroaryl, (C1-C60)alkylamino or (C6-C60)arylamino, or each of R₅₁ through R₇₀ may be linked to an adjacent substituent via (C3-C60)alkylene or (C3-C60)alkenylene with or without a fused ring to form an alicyclic ring or a mono- or polycyclic ring;
the aryl, heteroaryl or arylamino, or the mono- or polycyclic aromatic ring may be further substituted by one or more substituent (s) selected from deuterium, halogen, (C1-C60)alkyl, (C6-C60)aryl, (C4-C60) heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, carboxyl, nitro and hydroxyl;
Ar₁₀-(An)ₐ-Ar₂₀ (2)
wherein
An represents anthracenylene with or without one or more substituent(s) or -X-L₁₀₀-Y-;
X and Y independently represent anthracenylene;
L₁₀₀ represents (C6-C60)arylene or (C5-C60)heteroarylene with or without one or more substituent(s);
Ar₁₀ and Ar₂₀ independently represent (C6-C60) aryl with or without substituent or (C5-C60)heteroaryl with or without substituent;
the substituent substituted at An, Ar₁₀, Ar₂₀ or L₁₀₀ is one or more selected from deuterium, halogen, (C1-C60)alkyl, halo(C1-C60)alkyl, (C6-C60)aryl, (C3-C60)heteroaryl, morpholino, thiomorpholino, 5- or 6-membered heterocycloalkyl containing one or more heteroatom(s) selected from N, O and S, (C3-C60)cycloalkyl, tri(C1-C60)alkylsily, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono- or di(C1-C60)alkylamino, mono- or di(C6-C60)arylamino, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro and hydroxyl; and
a represents an integer from 1 to 4.
